# EUROPEAN PATENT APPLICATION

(11) **EP 4 790 685 A1**
(43) Date of publication of application: **12.08.2026**
(21) Application number: 24873939.3
(22) Date of filing: 04.10.2024
(51) Int. Cl.: G09B 23/28, G09B 23/00, G16H 50/20, G06V 40/16, A61B 5/107, A61B 5/11, A61B 90/00

(54) **COMPUTING METHOD AND SYSTEM FOR CONSULTATION, DIAGNOSTIC EVALUATION AND FINANCIAL PLANNING**

(30) Priority: 05.10.2023 BR 102023020687; 28.03.2024 BR 102024006331
(71) Applicant: De Maio Domingos, Mauricio, 04029-200 San Paulo (BR)
(72) Inventor: De Maio Domingos, Mauricio, 04029-200 San Paulo (BR)
(74) Representative: Uexküll & Stolberg
(86) International application number: PCT/BR2024/050456
(87) International publication number: WO 2025/073034

(57) **Abstract**

The present invention relates to a method and computing system for obtaining a treatment plan for a user. The method comprises the steps of receiving, on a computing platform, a plurality of general input data from a given user, generating a treatment identifier code from the plurality of general input data, collecting a plurality of consultation data related to the user, scanning, on the computing platform, at least a portion of the user, and generating the treatment plan from the scanning of at least a portion of the user. The present invention also relates to a system and computing platform in harmony with the described method.

## Description

The present invention relates to a method and computing system for supporting consultation, diagnostic evaluation, and *budgeting* (financial planning). More specifically, it refers to a method and system in which, based on the user's interaction with a computing platform, a specific treatment plan will be provided to the user. A device capable of performing the proposed method is also addressed.

### Description of the state of the art

Treatment plans are commonly generated, and with increasing frequency, related to a particular user. By way of example, a treatment plan may be related to an aesthetic treatment plan and/or a health treatment plan that must be carried out by a specific user. By user, it should be understood as, for example, a specific patient, healthcare professional, or any person qualified to interact with a computing platform.

There are several existing challenges today, challenges that result from the increased number of non-physician professionals authorized to perform procedures that were previously only permitted to be performed by physicians, such as carrying out an aesthetic and health treatment and obtaining an appropriate and personalized treatment plan related to a user. The uncontrolled growth of unqualified professionals from various healthcare fields, and of companies offering aesthetic products including minimally invasive procedures, is putting into question the ability of regulatory agencies to provide adequate guidance and supervision of processes-processes that range from considering the inclusion of a patient for a procedure to the completion of treatment and long-term follow-up.

For example, one of the challenges found in the state of the art lies in the difficulty of interaction between the medical professional or qualified healthcare professional and the user, whether due to issues of distance, scheduling, language, costs, among other factors.

In one example, a user must travel to the physician's or healthcare professional's clinic, an action that, in some situations, may not be easily and simply carried out by the user.

Moreover, in many situations, there are difficulties for the physician or healthcare professional to adequately identify what is the best treatment plan to be provided to a given user, considering that the diagnosis received by the user often differs according to the experience or even the lack of experience of the professional who will indicate a treatment, which can lead to complications and unnatural results, that is, unsatisfactory for the user.

Such difficulties, for example, lie in identifying the exact location where a given substance should be applied, as well as the manner (method) of application of the substance in question. An additional difficulty lies in the lack of information available in medical records; thus, neither the physician or qualified healthcare professional, nor the user, is aware of the history of treatment plans generated for that user, especially in aesthetic procedures. Thus, the lack of organized and controlled information negatively influences the treatment to be performed as well as future treatments.

Furthermore, it is up to the physician or qualified healthcare professional to conduct a comprehensive and specialized consultation, perform an appropriate evaluation, and provide the costs associated with the treatment plan to be generated; in other words, the healthcare professional assumes a commercial role, thus distancing themselves from their primary role, which lies in working within the healthcare sector. This fact ends up creating distortions in service delivery and provision, potentially placing financial interests above the user's well-being and health.

The challenges encountered in the state of the art are not limited to the scope of the user and the medical professional (or healthcare professional).

These challenges also extend to the manufacturers of various products, including aesthetic ones, such as injectable substances, suspension threads, various laser machines, ultrasound, radiofrequency (among others), topical products, among others.

In the case of unsatisfactory results, mild to severe complications, the lack of organized and uniform control of the user's medical record can cause conflicts between the involved parties, such as, for example, neither the healthcare professional, nor the user, nor the companies, often do not know which procedure or specific substance was applied to a user, as well as which healthcare professional was responsible for the application. The present invention overcomes such problems, allowing, for example, greater organization and control of information linked to the user.

Moreover, despite numerous training sessions conducted for healthcare professionals, the performance of aesthetic procedures, including the administration of injectable substances, may be carried out improperly, which ultimately proves detrimental to all parties involved, whether it be the user, who will receive ineffective treatment, as well as the medical or healthcare professional and the manufacturing company, whose reputations will be affected. The present invention will also assist in the educational support and training of the professionals involved in the process.

Thus, manufacturing companies simply do not know how the substances they have developed are being injected or even how their products or equipment are being used, whether due to a lack of structured training or even due to inadequate documentation.

The present invention overcomes these and several other problems found in the state of the art related to various aesthetic treatments through the proposal of a method and computing system for consultation, diagnostic evaluation, and *budgeting*.

Furthermore, based on the teachings of the present invention, it is possible to provide legal support and assistance to regulatory agencies that will require a structured and organized platform to promote balance and sustainable growth in the healthcare sector, including aesthetics, a field that has shown uncontrolled exponential growth and may cause harm to users, often difficult to repair.

As will be better understood from the following description, said method and system are based on the use of a computing platform by the user, so that the use of said platform by the user may occur with or without the presence of a physician or qualified healthcare professional. Thus, either through the user's direct interaction with the computing platform, or with the assistance of any person able to interact with the computing platform, a suggested treatment plan will be provided to the platform user.

Thus, with the present invention, there is no specific need for user interaction with the medical or healthcare professional in the initial stages of care, such as, for example, for conducting the consultation, diagnostic evaluation, *budgeting*, and generation of the treatment plan, so that the medical or healthcare professional, if desired by the user, may then be selected or suggested by the database of the present system.

There are several advantages obtained from the actions and teachings of the present invention, advantages that can be absorbed by all links in the chain, whether by the user (patient), by the physician or healthcare professional, by companies manufacturing injectables or any product or machinery used in aesthetics, and even by the various existing regulatory agencies that aim to ensure good medical and health practices.

### Objectives of the Invention

The present invention aims to propose a computer method for consultation, diagnostic evaluation, and financial planning to obtain a treatment plan, organized data management, and provision of future treatment possibilities (including artificial intelligence) linked to a specific user.

One of the objectives of the present invention is that the proposed method be carried out through a computing platform, such as a cell phone, tablet, television, kiosk, or equivalent electronic device that may be created and that enables interaction with the user.

One of the objectives of the present invention is that the consultation, diagnostic evaluation, treatment plan, and *budgeting* (financial planning) can be generated from the interaction between the user and the computing platform, not necessarily requiring the participation of a medical professional or healthcare professional in these stages.

The present invention also aims to propose a computing system for consultation, diagnostic evaluation, and financial planning for generating a treatment plan linked to a specific user.

One of the objectives of the present invention also lies in providing a computing platform capable of carrying out the methodology described in the present invention.

### Brief Description of the Invention

A computer method is described for consultation, diagnostic evaluation, and financial planning to obtain a treatment plan related to a user, the method comprising the steps of: receiving, on a computing platform, a plurality of general input data from a given user, generating a treatment identifier code from the plurality of general input data, collecting a plurality of consultation data related to the user, scanning, on the computing platform, at least a portion of the user, and generating the treatment plan from the scanning of at least a portion of the user.

The present invention also describes a system and computing platform in harmony with the proposed method.

### Brief Description of the Drawings

The present invention will hereinafter be described in greater detail based on an exemplary embodiment illustrated in the drawings. The figures show:
FIG. 1 - is a representation of one of the steps that make up the methodology described in the present invention, illustrating the computing platform as well as the receipt of general input data, such as identification data, detailed inquiry about anamnesis, habits, personal history, medications, surgeries and previous treatments, including aesthetic ones, among other relevant information about the user's health status;
FIG. 2 - is a representation of one of the steps that make up the methodology described in the present invention, illustrating the computing platform as well as the provision of aesthetic data including the areas of treatment interest by the user;
FIG. 3 - is a representation of one of the steps that is part of the methodology described in the present invention, illustrating the computing platform as well as the provision of the expected emotional and/or social messages desired by the user;
FIG. 4 - is a block diagram of the methodology described in the present invention illustrating the steps for generating the final set of aesthetic data as well as the final set of emotional data;
FIG. 5 - is a block diagram of the methodology described in the present invention illustrating the steps for generating the final set of social data;
FIG. 6 - is a block diagram of the methodology described in the present invention illustrating the steps for generating the difficulty index for achieving the result desired by the user. The difficulty index can be obtained, for example, from the user's age, the presence of signs of aging, the degree of severity, among other parameters to be determined, for example, after facial scanning;
FIG. 7 - is a representation of one of the steps that is part of the methodology described in the present invention, illustrating the computing platform as well as the execution of the horizontal scanning of the user's portion;
FIG. 8 - is an additional representation of one of the steps that make up the methodology described in the present invention, illustrating the computing platform as well as the execution of the horizontal scanning of the user's portion;
FIG. 9 - is an additional representation of one of the steps that make up the methodology described in the present invention, illustrating the computing platform as well as the execution of the horizontal scanning of the user's portion;
FIG. 10 - is an additional representation of one of the steps that make up the methodology described in the present invention, illustrating the computing platform as well as the execution of the horizontal scanning of the user's face, in an oblique mode;
FIG. 11 - is an additional representation of one of the steps that make up the methodology described in the present invention, illustrating the computing platform as well as the execution of the horizontal scanning of the user's face, in a lateral mode;
FIG. 12 - is a representation of one of the steps that is part of the methodology described in the present invention, illustrating the computing platform as well as the execution of the vertical scanning of the user's portion, in a frontal mode;
FIG. 13 - is a representation of one of the steps that is part of the methodology described in the present invention, illustrating the computing platform as well as the execution of the vertical scanning of the user's portion, in an oblique mode;
FIG. 14 - is a representation of one of the steps that is part of the methodology described in the present invention, illustrating the computing platform as well as the execution of the vertical scanning of the user's portion, in a lateral mode;
FIG. 15 - is a representation of one of the steps that is part of the methodology described in the present invention, illustrating the computing platform as well as the execution of the step of generating a plurality of segments from the horizontal scanning of the user's face, in which Fig. 15 (a) illustrates the user's face in a frontal view, Fig. 15 (b) illustrates the user's face in an oblique view, and Fig. 15 (c) illustrates the user's face in a lateral view;
FIG. 16 - is a representation of one of the steps that is part of the methodology described in the present invention, illustrating the computing platform as well as the execution of the step of generating a plurality of segments from the vertical scanning of the user's face, in which Fig. 16 (a) illustrates the user's face in a frontal view, Fig. 16 (b) illustrates the user's face in an oblique view, and Fig. 16 (c) illustrates the user's face in a lateral view;
FIG. 17 - is a representation of the step of correlating the scanning of the user's portion with the set of virtual emotional and social data;
FIG. 18 - is a representation of one of the steps that make up the methodology described in the present invention, illustrating the step of comparing the user's portion in a static state and in a dynamic state, including various facial expressions such as smiling, surprise, disappointment, etc.;
FIG. 19 - is an additional representation of one of the steps that make up the methodology described in the present invention, illustrating the step of comparing the user's portion in a static state and in a dynamic state;
FIG. 20 - is an additional representation of one of the steps that make up the methodology described in the present invention, illustrating the step of segmenting the user's portion into two halves;
FIG. 21 - is an additional representation of one of the steps that make up the methodology described in the present invention, illustrating the step of forming the first representation and the second representation of the user;
FIG. 22 - illustrates an additional representation of one of the steps that is part of the methodology described in the present invention, illustrating the step of scanning the first representation and the second representation of the user;
FIG. 23 - is a representation of one of the steps that is part of the methodology described in the present invention, illustrating the step of vertical scanning of a second representation of the user;
FIG. 24 - is a representation of one of the steps that is part of the methodology described in the present invention, illustrating the step of representing an unfavorable indication to the user;
FIG. 25 - is a representation of one of the steps that is part of the methodology described in the present invention, illustrating the step of vertical scanning of a usual portion of the user;
FIG. 26 - is a representation of one of the steps that is part of the methodology described in the present invention, illustrating the step of displaying a negative indication to the user;
FIG. 27 - is a representation of one of the steps that is part of the methodology described in the present invention, illustrating the step of scanning a portion of the user in a dynamic state;
FIG. 28 - is a representation of one of the steps that is part of the methodology described in the present invention;
FIG. 29 - is a representation of one of the steps that make up the methodology described in the present invention, illustrating the step of scanning a portion of the user in a frontal manner (Fig. 29 (a)), oblique (Fig. 29 (b)), and lateral (Fig. 29 (c));
FIG. 30 - is a representation of one of the steps that make up the methodology described in the present invention, illustrating the step of scanning a portion of the user in a frontal manner (Fig. 30 (a)), oblique (Fig. 30 (b)), and lateral (Fig. 30 (c));
FIG. 31 - is a representation of one of the steps that make up the methodology described in the present invention, illustrating the step of scanning a portion of the user in a frontal manner (Fig. 31 (a)), oblique (Fig. 31 (b)), and lateral (Fig. 31 (c));
FIG. 32 - is a representation of one of the steps that is part of the methodology described in the present invention, illustrating the step of scanning a portion of the user in a frontal manner (Fig. 32 (a)), oblique (Fig. 32 (b)), and lateral (Fig. 32 (c));
FIG. 33 - is a representation of one of the steps that is part of the methodology described in the present invention, illustrating the step of scanning a portion of the user in a static state and in a dynamic state, in which Fig. 33 (a) represents the static and dynamic state, Fig. 33 (b) represents the segmentation of the face into a plurality of quadrants, and Fig. 33 (c) represents the indication of an aesthetic marker;
FIG. 34 - is an additional representation of one of the steps that is part of the methodology described in the present invention, illustrating the step of scanning a portion of the user in a static state and in a dynamic state, in which Fig. 34 (a) represents the static and dynamic state, Fig. 34 (b) represents the segmentation of the face into a plurality of quadrants, and Fig. 34 (c) represents the indication of an aesthetic marker;
FIG. 35 - is an additional representation of one of the steps that make up the methodology described in the present invention, illustrating the step of scanning a portion of the user in a static state and in a dynamic state, in which Fig. 35 (a) represents a first expression and Fig. 35 (b) represents a second expression;
FIG. 36 - is an additional representation of one of the steps that make up the methodology described in the present invention, illustrating the step of segmenting the user's face into a plurality of micro segments MS;
FIG. 37 - is a representation of one of the steps that make up the methodology described in the present invention, illustrating an example of a model considered ideal, in which Fig. 37 (a) illustrates the segmentation of the model considered ideal into a plurality of quadrants and Fig. 37 (b) illustrates the assignment of an ideal index to each of these quadrants;
FIG. 38 - is a representation of one of the steps that make up the methodology described in the present invention, illustrating the step of comparing a portion considered ideal with a current portion of the user;
FIG. 39 - is an additional representation of one of the steps that make up the methodology described in the present invention, illustrating the step of comparing a portion considered ideal with a current portion of the user, in which said portions are segmented into a plurality of quadrants;
FIG. 40 - is a representation of one of the steps that is part of the methodology described in the present invention, illustrating the exemplary step of indicating the monitoring, consideration, and treatment portions based on the concept of ATP (*Aging Trigger Points*, or aging trigger points);
FIG. 41 - is a representation of one of the steps that is part of the methodology described in the present invention, illustrating the exemplary step of indicating at least one treatment code, such that, in this case, they refer to codes for the treatment of dynamic expressions;
FIG. 42 - is a representation of one of the steps that is part of the methodology described in the present invention, illustrating the exemplary step of indicating at least one application dose;
FIG. 43 - is a representation of an application seal that incorporates the teachings of the present invention, in which Fig. 43 (a) illustrates a generic application seal and Fig. 43 (b) illustrates the application seal comprising a plurality of information, including the application site (treatment code), the type of product, the layer to be injected, the method of product distribution, and the amount to be injected;
FIG. 44 - is a representation of one of the steps that is part of the methodology proposed in the present invention, showing the generation of a treatment plan in the form of a table for a given session;
FIG. 45 - illustrates the step of correlating the treatment plan with the application seals for a given application session;
FIG. 46 - illustrates the step of correlating the treatment plan with the application seals for a given application session;
FIG. 47 - illustrates the step of correlating the treatment plan with the application seals for a given application session;
FIG. 48 - illustrates the step of correlating the treatment plan with the application seals for a given application session;
FIG. 49 - illustrates one of the steps that is part of the methodology proposed in the present invention, representing the generation of an application chain for a given user;
FIG. 50 - is a representation of one of the steps that is part of the methodology described in the present invention, showing the arrangement of reference lines for evaluation of the treatment performed;
FIG. 51 - is an exemplary illustration of an electronic device capable of absorbing the teachings of the present invention;
FIG. 52 - illustrates an example of body treatment codes;
FIG. 53 - illustrates a second example of body treatment codes;
FIG. 54 - illustrates a third example of body treatment codes;
FIG. 55 - illustrates a fourth example of body treatment codes;
FIG. 56 - is an example of a graphic representation generated by artificial intelligence of a professional in the aesthetic field.
FIG. 57 - is an example of a graphic representation generated by artificial intelligence, illustrating a multidisciplinary graphic representation.
FIG. 58 - is an example of one of the steps that make up the methodology described in the present invention, representing the simulation of an aging process;

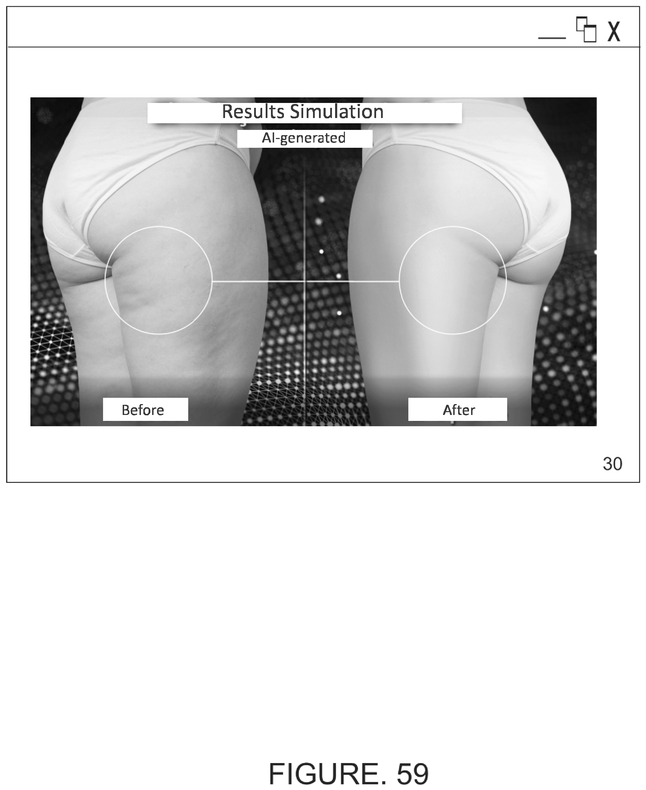

FIG. 59 - is an example of one of the steps that make up the methodology described in the present invention, representing the simulation of the illustration of possible results through the implementation of a treatment plan.

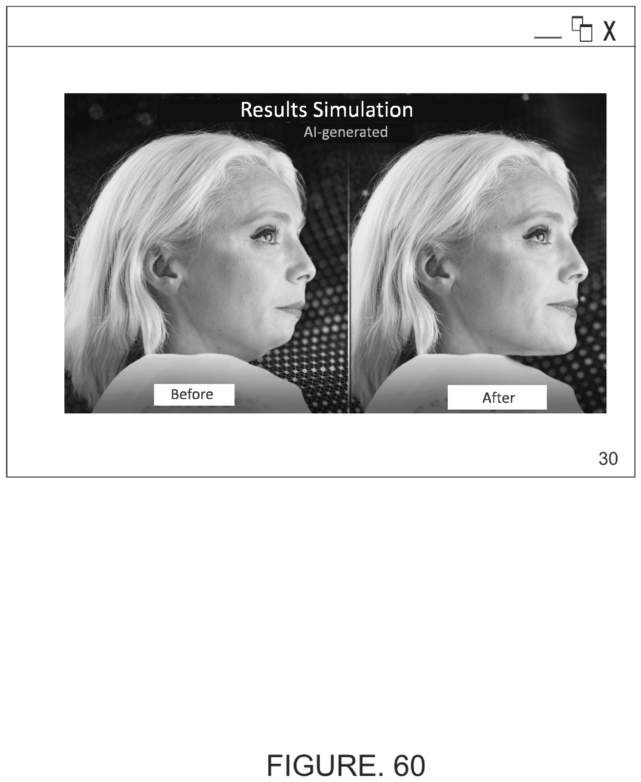

FIG. 60 - is an additional example of one of the steps that is part of the methodology described in the present invention, representing the simulation of the illustration of possible results through the implementation of a treatment plan;
FIG. 61 - is an additional example of one of the steps that make up the methodology described in the present invention, representing the simulation of the illustration of possible results through the implementation of a treatment plan;
FIG. 62 - is a representation of a user who may make use of the teachings of the present invention, in which said user makes use of an augmented reality device;
FIG. 63 - is an example of a first instruction perceivable through the use of an augmented reality device;
FIG. 64 - is an example of a marking made on the patient who will receive a specific application;
FIG. 65 - is an example of a second instruction perceivable through the use of an augmented reality device;
FIG. 66 - is an example of a marking made on the patient who will receive a specific application;
FIG. 67 - is a representation of a third instruction perceivable through the use of an augmented reality device;
FIG. 68 - is a representation of a fourth instruction perceptible through the use of an augmented reality device;
FIG. 69 - is a representation of a fifth instruction perceptible through the use of an augmented reality device;
FIG. 70 - is a representation of a sixth instruction perceivable through the use of an augmented reality device;

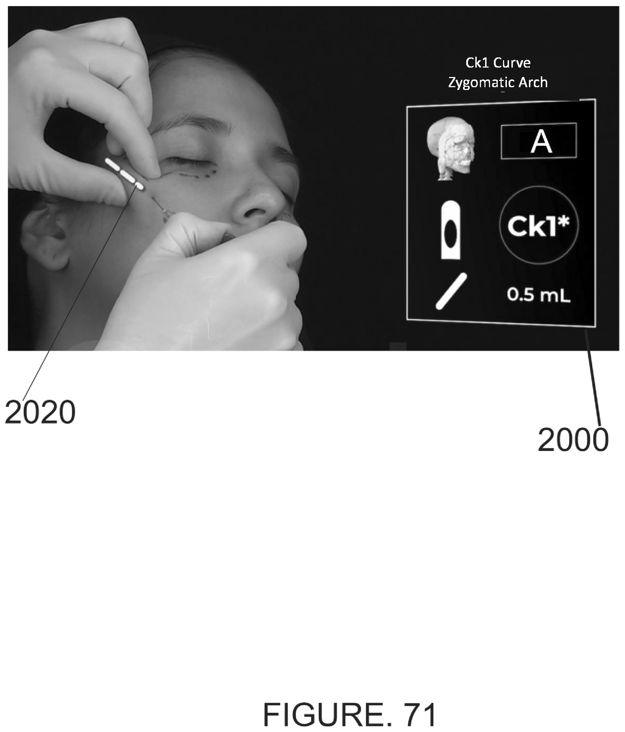

FIG. 71 - is an additional representation of a sixth instruction perceptible through the use of an augmented reality device;
FIG. 72 - is an example of a graphical indication perceptible through the use of an augmented reality device;
Figure 73 - illustrates a representation of a mode of interaction of a user with the computing platform;
Figure 74 - illustrates a representation of an additional embodiment of user interaction with the computing platform and also through the graphical representation generated by artificial intelligence, in which a case study is presented to the user;
Figure 75 - illustrates a representation of an additional mode of user interaction with the computing platform, illustrating the association of an application seal with a case study;
Figure 76 - illustrates a representation of an additional mode of user interaction with the computing platform, showing the arrangement of a set of instructions in an overlay manner on the case study;
Figure 77 - illustrates a representation of an additional mode of user interaction with the computing platform, showing the arrangement of a set of instructions in an overlay manner on the case study;
Figure 78 - illustrates a representation of an additional mode of user interaction with the computing platform, showing the generation of a test seal by the user.

### Detailed Description of the Drawings

The present invention initially describes a method and computing system for consultation, diagnostic evaluation, and financial planning (*budgeting*) related to the generation of a treatment plan for a given user. More specifically, the proposed method will provide a treatment plan to the user based on their interaction with a computing platform 30.

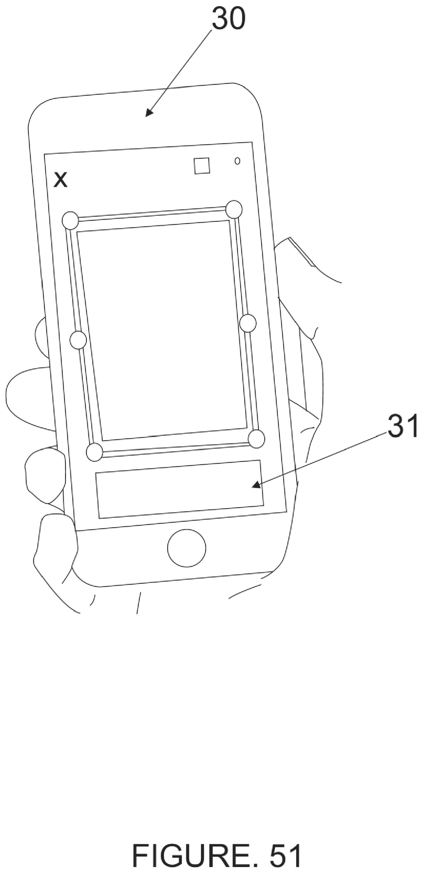

A computing platform 30 is understood to be any electronic device capable of establishing an internet connection, such as a computer, cell phone, tablet, *smart-watch*, television, kiosk, among others. FIG. 51 is an example of an electronic device that can be understood as a computing platform 30. It is also noteworthy that the teachings of the present invention do not necessarily require the computing platform 30 to be connected to the internet, such that interaction with the user may be performed *offline* and subsequently uploaded to the platform once the connection is reestablished or initiated.

It is proposed that said computing platform 30 comprises a module capable of receiving at least one specific user input data and inserting said input data into the methodology proposed in the present invention; thus, the computing platform must comprise an input module 31, wherein the input module 31 can be understood, for example, as a keyboard, a camera, a combination thereof, or any other means capable of receiving data (information) from the user and inserting such data (information) from the user into the methodology proposed in the present invention.

The methodology proposed in the present invention must then be accessed by the user through their interaction with the aforementioned computing platform. The way the user will access the computing platform does not represent the essential aspect of the invention, so any form of access would be fully valid, such as access through *login*, password, and/or facial biometrics.

For example, in one embodiment, the computing platform 30 could be accessed by the user through an electronic address entered in an internet browser, alternatively, or additionally, through the user's access to a specific application installed on an electronic device, such as on a television, cell phone, or tablet.

The user's access to the computing platform can be confirmed through prior registration and provision of a *login* and password; thus, when accessing the computing platform, an account linked to the user will be created. It is thus understood that the methodology proposed in the present invention comprises the step of creating an account linked to the user, so that a user code could be generated.

The method proposed in the present invention proposes the step of receiving, on the computing platform, a plurality of general input data from a given user.

Thus, upon accessing the computing platform, the user will be prompted to provide a plurality of general input data, which should be stored, for example, in a memory of the computing platform and/or of the methodology proposed in the present invention. It is thus understood that the computing platform comprises a memory that stores a set of instructions, in which said set of instructions can be executed by a processor. In one embodiment, said memory could be arranged in a cloud environment or could be configured as the memory of the computing platform itself.

For general input data, this should be understood as the provision of data that allows the identification of the user's name, as well as the identification of basic user information, such as nationality, gender, and date of birth. Usually, providing the information contained in the user's passport or in their national driver's license or in their national identity card constitutes data suitable to configure the general entry data.

The aforementioned general input data can be entered into the computing platform by any means, whether by typing, document scanning, document upload, by voice, as well as a combination of these. The way in which the general input data will be entered into the computing platform does not represent the essential aspect of the present invention, so that any form of entry would be fully valid.

Furthermore, it is also proposed that the computing platform motivate the user, through a generator block, to indicate consent with an adherence term, which may be linked to practices related to data protection, health practices, or any practice associated with a specific country or region.

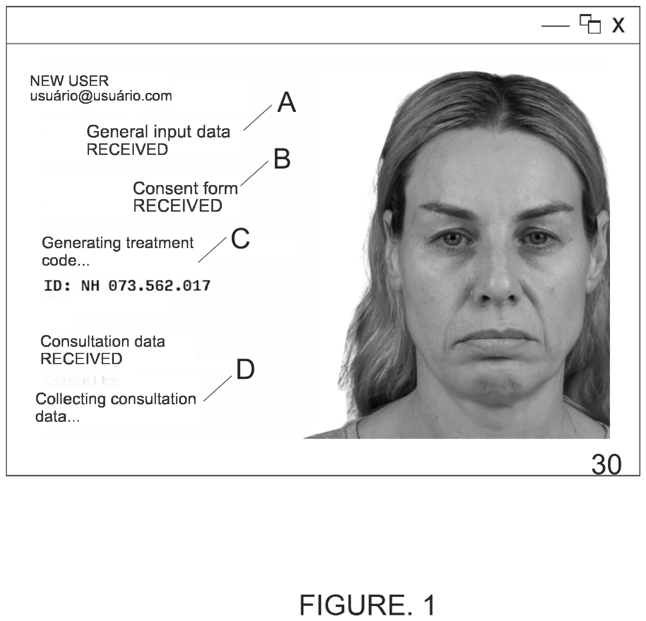

Thus, FIG. 1 illustrates a representation of the computing platform (of its screen) in which the execution of the steps of receiving the general input data A and consenting to the adherence term B is illustrated.

Thus, once the user has provided the general input data and established consent with the terms of adherence, the computing platform evaluates whether such data has been properly entered and, in this way, the sequence of the methodology proposed in the present invention proceeds.

Still taking the illustration of FIG. 1 as a reference, the methodology proposed in the present invention comprises the step of generating an identifier code for treatment C from the plurality of general input data. Thus, it is proposed that the computing platform will generate a specific treatment code linked to that user, in which the aforementioned code can be understood as an identification code for entry into the database system to then proceed with the subsequent proposed steps, such as consultation, diagnostic evaluation, and creation of a suggested treatment plan.

Said treatment code can be randomly generated by the computing platform and may be composed, for example, of numbers, letters, as well as a combination thereof. Any form of representation of the treatment code may be used. The way the treatment code is displayed in the figure in question should not be considered a limiting feature of the present invention.

It should be noted that the aforementioned treatment identification code should be understood as a code that associates a specific user with consultation data, diagnostic evaluation, and financial planning. Thus, a given user may understand a plurality of treatment identifier subcodes associated with the user, since the user may undergo a series of treatments throughout their life.

Thus, if the user obtains a treatment plan nowadays, a specific treatment code will be generated and associated with the user. If the same user obtains a new treatment plan at a future date, a new treatment code will be generated and associated with the user. Thus, it is understood that each treatment code is associated with a specific date.

It is thus understood that the same user may be associated with a series of treatment identifier subcodes.

Furthermore, and taking as reference the illustration of FIG. 1, it is observed that the treatment identification code generated for the user is the code NH 073.562.017.

Once the treatment identification code has been generated, the methodology described herein further proposes the step of collecting a plurality of consultation data related to the user, this step being identified with reference D, as can be seen from FIG. 1.

Consultation data should be understood as data (information) commonly found in medical records (or health records), for example, and without limitation, consultation data may be understood as the following data: (i) whether the user has any clinical condition (disease), (ii) whether the user has completed their vaccination schedule, (iii) whether the user has recently undergone any dental procedure, (iv) whether the user has any allergies, (v) whether the user is taking any medication, (vi) whether the user has already undergone any aesthetic procedure, such as a facial aesthetic procedure, (vii) whether the user has already received a botulinum toxin application, (viii) whether the user has already received an application of *permanent fillers*, (ix) whether the user has already received a hyaluronic acid application, (x) whether the user has already received biostimulators, (xi) whether the user has already received any type of treatment, (xii) whether the user has ever had any adverse reaction to any type of medication, treatment, and/or application and/or (xiii) anamnesis data.

The consultation data addressed in the previous paragraph should not be considered as limiting, such that other consultation data could be addressed and integrated into the methodology proposed in the present invention. Thus, the description provided is intended to allow an understanding of the consultation data.

In a valid embodiment of the present invention, the information provided by the user to the computing platform 30 could be collected through the user's interaction with a graphical representation of a medical professional, such as a graphical representation generated by artificial intelligence.

Thus, said graphical representation should be understood as a representation, for example, of the face of the medical professional, further including the voice of the medical professional himself. In this way, all interaction between the user and the computing platform will be conducted by this type of "avatar" (graphical representation of the physician generated by artificial intelligence). Furthermore, the aforementioned graphical representation should be understood as a representation of the user's face, capable of expression, that is, endowed with movement, and which also includes the voice of the physician or healthcare professional.

In this way, it is understood that the voice of the aforementioned graphical representation may be selected, for example, according to the user's preferences for a given language.

Furthermore, the proposal to use the graphical representation generated by artificial intelligence becomes beneficial as it provides greater confidence to the user in the process of interacting with the computing platform, since the user will literally see (observe, perceive) the face of a physician or healthcare professional, endowed with expressions, and hear their voice, thus acting as a mentor in the process of generating the treatment plan.

In an additional embodiment, the user's interaction with the computing platform could also be carried out through the user's interaction with a plurality of additional graphical representations 200, 300, 400, 500, 600, 700, 800, 900, and 1000.

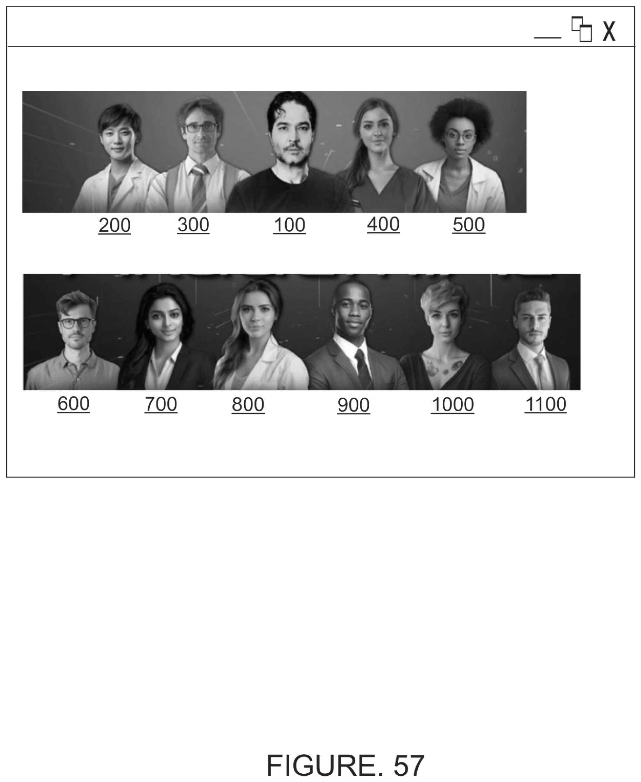

With reference to FIG. 57, said additional graphic representations 200, 300, 400, 500, 600, 700, 800, 900, 1000, and 1100, also referred to as multidisciplinary representations, are intended to assist both the professional and the user during their interaction with the computing platform.

Thus, said multidisciplinary representations 200, 300, 400, 500, 600, 700, 800, 900, 1000, and 1100, as well as the physician representation 100, must also be generated by artificial intelligence. Furthermore, the multidisciplinary representations 200, 300, 400, 500, 600, 700, 800, 900, 1000, 1100 are configured as representations of professionals from different fields of expertise, which assist both the professional and the user during interaction with the computing platform.

Thus, FIG. 57 is an exemplary representation of the multidisciplinary graphic representations 200, 300, 400, 500, 600, 700, 800, 900, 1000, and 1100, as well as the graphic representation of the medical professional 100.

In this way, a first multidisciplinary graphical representation 200 may refer, for example, to a graphical representation generated by artificial intelligence of a biomedical professional, who is responsible for, during the user's interaction with the computing platform, assisting with the evaluation and monitoring of patients.

A second multidisciplinary graphical representation 300, on the other hand, may refer, for example, to a graphical representation generated by artificial intelligence of a teacher, who is responsible, for example, during the user's interaction with the computing platform, for keeping both the user and the professional updated with the most recent scientific reports in the field.

A third multidisciplinary graphical representation 400 may refer, for example, to a graphical representation generated by artificial intelligence of a nurse, who is responsible for providing information about pre- and post-treatment care during the user/professional's interaction with the computing platform.

Additionally, a fourth multidisciplinary graphical representation 500 may refer, for example, to a pharmaceutical company, which is responsible, for example, for sharing important details about the care and storage of injectable products.

A fifth multidisciplinary graphical representation 600 may refer, for example, to the graphical representation of an accounting professional, who may provide guidance on fees, taxes, and related topics.

A sixth multidisciplinary graphical representation 700, for example, may refer to a graphical representation generated by artificial intelligence of a marketing professional, who is responsible for providing tips on promotional strategies to the manager of an aesthetic clinic, for example.

Additionally, a seventh multidisciplinary graphical representation 800 may refer, for example, to a product consultant, who is responsible for presenting both to the user and to the professional a portfolio of products that may be used for generating the treatment plan.

An eighth multidisciplinary graphical representation 900 may refer, for example, to a business manager, who may be responsible for providing guidance on clinic management.

A ninth multidisciplinary graphical representation 1000, for example, may refer to a digital influencer, who may assist, for example, in promoting the work performed by a professional on social media.

Furthermore, a tenth multidisciplinary graphical representation 1100 may refer, for example, to a graphical representation generated by artificial intelligence of a lawyer, who is responsible for assisting with legal matters, such as consent forms and patient documentation.

It should be noted that the multidisciplinary graphic representations mentioned above should not refer to limiting features of the present invention, such that the representation of other professions/positions would be fully acceptable.

Furthermore, the multidisciplinary graphic representations 200, 300, 400, 500, 600, 700, 800, 900, 1000, and 1100 can be configured according to the country of residence of the user and/or the professional. For example, if the user who absorbs the teachings of the present invention resides in Japan, the multidisciplinary graphical representation 1100 of the attorney will assist both the user and the professional with legal matters specific to this location (Japan).

Furthermore, it is proposed that each multidisciplinary graphic representation be given a name, with such name containing the letters AI or IA in its designation, as an allusion to artificial intelligence, and that said multidisciplinary graphic representation be presented to the user and/or the professional, for example, through a video.

It is noteworthy that all the features already discussed for the graphical representation of the medical professional 100 are also valid for the multidisciplinary graphical representations 200, 300, 400, 500, 600, 700, 800, 900, 1000, and 1100.

Thus, FIG. 1 is a non-limiting representation of the computing platform 30, illustrating some of the steps that make up the methodology proposed in the present invention. For example, the step of receiving the general input data A, acceptance of the consent form B, generation of the treatment identifier code C, and collection of the consultation data D is illustrated.

The collection of consultation data D may occur from any database associated with the computing platform, such as from a memory capable of storing consultation data from a plurality of users. It is thus understood that the consultation data are filled in by the user and stored in the aforementioned memory.

Once the consultation data has been collected, the methodology described in the present invention further proposes the step of evaluating whether there is, in the consultation data related to the user, any restrictive data. Restrictive data should be understood as data (information) that counterindicates the user's obtaining the treatment plan.

For example, restrictive data may be understood as data indicating whether the user has recently undergone a dental procedure, or data indicating that the user has an allergy, or data indicating that the user is receiving a certain medication that counterindicates the application of injectables, or data indicating that the user has had a certain reaction to a previously performed procedure.

Furthermore, the present invention also proposes carrying out the step of associating the restrictive data with the treatment identifier code of that user, as well as associating the restrictive data with the user's code.

Thus, suppose that in a treatment plan previously carried out by the user, they experienced a certain adverse reaction. Thus, it is proposed that the occurrence of this undesired reaction (restrictive data) be associated with the treatment identifier code of that user, as well as associated with the user's identifier code, so that if the user generates a new treatment subcode in the future, it will be possible to know that, in the past, a restrictive data related to that user occurred. Restrictive data do not con-traindicate the treatment, but will serve as a warning for the future clinical evaluation by the physician or qualified healthcare professional selected to perform the procedure.

Thus, the present invention comprises the step of evaluating whether, in the consultation data related to the user, there is a restrictive data as well as evaluating whether there is restrictive data related to the user.

If restrictive data is detected, it is proposed to carry out the step of issuing a perceptible representation to the user, for example, on the screen of the computing platform 30. Such representation can be configured as a text, an image, an audible representation, as well as a combination of any of these.

The purpose of issuing the perceptible representation to the user is to issue an alert to the user regarding the existence of the restrictive data, so that the existence of the restrictive data should not necessarily be understood as an impediment to the continuation of the proposed methodology.

In sequence with the description of the teachings of the present invention, there is the step of obtaining at least a first set of aesthetic data from the user based on an impulsive representation of the patient.

Thus, it is proposed that the computing platform should motivate the user so that the user provides a first set of aesthetic data. More specifically, the provision of a first set of aesthetic data must occur through an impulsive representation of the patient. By impulsive representation, it can also be understood as a subjective representation, as will be described below.

By first set of aesthetic data, it should be understood as a series of aesthetic data that a given user is interested in treating; more specifically, the first set of aesthetic data should be understood as the regions/parts of the face and/or body that the user is interested in correcting or improving through the treatment plan.

On the other hand, by impulsive (subjective) representation of the user, it should be understood that the user must, impulsively, provide the first set of aesthetic data to the computing platform 30. More specifically, the user may simply input the first set of aesthetic data into the computing platform. To this end, the computing platform may, for example, display a text box (input block 31) that prompts the user to enter the first set of aesthetic data. The system may also provide a list of areas, regions/parts of the face and/or body so that the user is aware of the potential treatment areas.

Furthermore, the user's impulsive representation can be understood as the act of activating an optical capture element (such as a camera) of the computing platform and, through capturing a video of the user, the user will be prompted to provide the first set of aesthetic data.

Thus, the computing platform 30 will prompt the user to provide the first set of aesthetic data by activating the optical capture element, recording a video of the user, and providing a field for the user to enter the first set of aesthetic data, for example. It is further proposed that the first set of aesthetic data be provided by the user while the user views themselves in the video captured by the camera.

In one analogy, the user's impulsive representation can be understood as the act of the user looking at themselves in a mirror, so that the mirror, in this case, may also refer to the capture of the user's video through the computing platform.

Thus, it is understood that the step of obtaining at least a first set of aesthetic data from the user from an impulsive representation of the user further comprises the step of activating an optical capture element of the computing platform and capturing a video of the user, thereby motivating the user to provide the first set of aesthetic data. More specifically, the user evaluates the video and provides the first set of aesthetic data.

Additionally, the methodology described in the present invention further comprises the step of obtaining at least one second set of aesthetic user data from a graphical representation of the user. Graphical representation should be understood as a photo of the user.

Thus, the methodology proposed in the present invention motivates the user, through the computing platform, to evaluate their own photo and enter the second set of aesthetic data based on the evaluation of the photo. The user's photo can be evaluated through the *upload* of a photo of the user on the computing platform, or through the capture of a photo of the user using the optical capture element of the computing platform 30 itself.

It is thus understood that the step of obtaining at least one second set of aesthetic data from the user from a graphical representation of the user further comprises the step of obtaining the second set of aesthetic data through the user's analysis of their own photo. More specifically, the user evaluates their photo and provides the second set of aesthetic data. Graphical representation can also be understood as an objective representation of the user, since the user evaluates their photo and, objectively, provides the second set of aesthetic data.

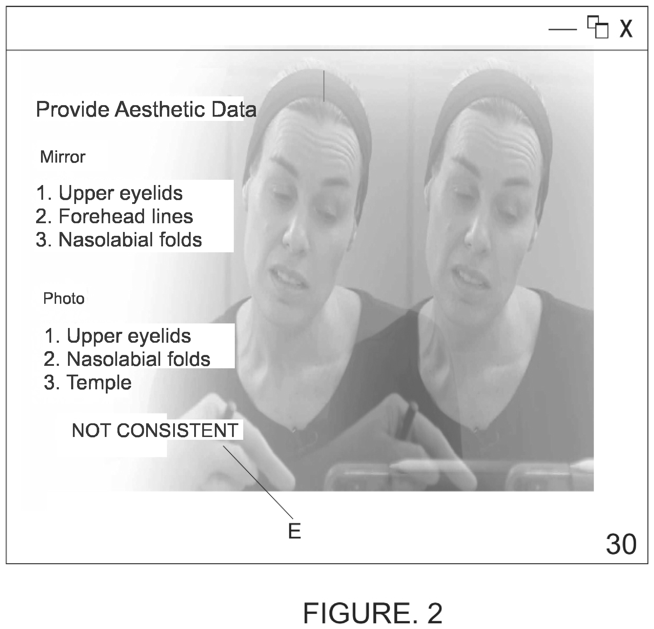

FIG. 2 is an illustration of the computing platform representing the previously discussed steps for obtaining the first and second sets of aesthetic data.

As previously described with reference to FIG. 2, said evaluation may be performed by mirror, photo, camera, or any other equivalent means. The system will identify whether there is consistency or not between the request intended by the user when looking in the mirror and the photo taken during the consultation. The system is also configured to assist the user in defining the priorities of the treatment areas, highlight inconsistencies, and will function as an educational method regarding the user's subjective self-evaluation.

Taking FIG. 2 as a reference, the first set of aesthetic data obtained from the impulsive representation can be considered as the following aesthetic data: 1. Upper eyelids; 2. Forehead lines; 3. Nasolabial folds. The second set of aesthetic data obtained from the user's graphical representation can be understood as 1. Upper eyelids; 2. Nasolabial folds and 3. Temples.

Furthermore, it is noteworthy that the aesthetic data initially provided by the user (or provided first in the list of aesthetic data sets) can be understood as the most relevant aesthetic data, that is, the aesthetic data that the user has the greatest interest/desire to treat. In the case of the representation of FIG. 2, the aesthetic data regarding the upper eyelid can be understood as the most relevant data, both for impulsive (subjective) feedback and for graphic (objective) feedback.

Once the first and second sets of aesthetic data have been obtained, the methodology described herein proposes the step of evaluating, in a comparator module, whether there is consistency between each of the aesthetic data from the first set of aesthetic data and the second set of aesthetic data.

Thus, in a comparator block, understood as a processor, microprocessor, or any element capable of performing a comparison between data, it will be evaluated whether there is consistency between the aesthetic data of the first set and the aesthetic data of the second set.

More specifically, it will be evaluated whether the aesthetic data of the first set of aesthetic data are the same aesthetic data as the second set of aesthetic data. Even more specifically, it will be evaluated whether the aesthetic data provided by the user in the impulsive feedback (impulsive representation) are the same aesthetic data provided by the user in the graphical feedback (graphical representation). Even more specifically, it will be evaluated whether the order provided for the aesthetic data is the same, considering the first and second sets of aesthetic data.

In the exemplary illustration of FIG. 2, the comparator block, when evaluating the first aesthetic data of the impulsive representation (mirror) and the first aesthetic data of the graphic representation (photo), will detect a consistency, since such aesthetic data are the same (upper eyelids).

In the comparison of the second aesthetic data of the impulsive representation with the second aesthetic data of the graphic representation, an inconsistency will be detected, since the data refers to forehead lines for the impulsive return and to nasolabial folds for the graphic representation. Similarly, an inconsistency will also be detected for the comparison between the third aesthetic data of the impulsive representation and the third aesthetic data of the graphic representation.

In the representation of FIG. 2, the comparison indicates the existence of inconsistencies; in this case, it is proposed to carry out the step of issuing a perceptible representation to the user. More specifically, the computing platform must inform/alert the user that an inconsistency has been detected, since the aesthetic data provided by the user in the impulsive return does not match the aesthetic data provided by the user from the graphical representation. The indication of an inconsistency is illustrated in FIG. 2 from step E.

Furthermore, in the event of an inconsistency, the user shall be prompted, through the computing platform, to enter a final set of aesthetic data. The final set of aesthetic data should be understood as the aesthetic data that the user actually wishes to treat. More specifically, the computing platform identified an inconsistency between the first set of aesthetic data and the second set of aesthetic data, such that said inconsistency was reported to the user.

Thus, the user, being aware of the detected inconsistency, must inform the computing platform which aesthetic data should actually be considered, that is, the user must provide the final set of aesthetic data. In the absence of achieving consistency, the system will establish the information obtained from the photo analysis as the final option because it is less subjective.

Said perceptible representation to the user can be configured as a text, video, audio, or color representation, as well as a combination thereof. It is noteworthy that the manner in which the representation will be perceptible to the user does not represent the essential aspect of the present invention.

If a consistency is detected by the comparator block, that is, if there is a consistency between the first set of aesthetic data and the second set of aesthetic data, it is proposed that the user also receive a perceptible representation indicating that said consistency has been detected and, additionally, it is also proposed to carry out the step of transforming the first set of aesthetic data and the second set of aesthetic data into a final set of aesthetic data.

Thus, based on the previously discussed steps, the methodology proposed in the present invention, through the computing platform, motivates (prompts) the user to indicate which aesthetic data should be addressed in the treatment plan. More specifically, by obtaining the aesthetic data through impulsive (subjective) and graphic (objective) representation, as previously described, the methodology intensifies the provocation to the user, thus aiming to assess whether the user is fully certain and convinced of which aesthetic data should be considered in the treatment plan.

In this sense, it often happens that the user appears to be fully convinced that a certain portion of their body and/or face (such as a given aesthetic feature) requires an aesthetic treatment. However, when performing the aforementioned aesthetic treatment on the given aesthetic data in question, the user's dissatisfaction with that particular aesthetic data persists. That is, the user appeared to be fully convinced that the treatment of that aesthetic data would be beneficial to them; however, after the treatment was performed, the user's dissatisfaction remains.

This is one of the reasons why it becomes important to motivate the user to provide aesthetic data from different representations, such as from an impulsive and graphical representation, as previously described. In this way, the treatment plan to be provided to the user becomes more assertive.

Aiming to further increase the accuracy of the treatment plan to be provided to the user, the methodology described in the present invention also proposes the step of obtaining a first set of emotional (and/or social) data (messages) from the user based on the first set of aesthetic data and also obtaining a second set of emotional (and/or social) data from the user based on the second set of aesthetic data.

More specifically, it is understood that the first and second sets of aesthetic data refer to specific portions of the face and/or body that the user wishes to treat. In the case of the representation of FIG. 2, such portions are indicated as eyelids, forehead, nasolabial region (nose/mouth), and temple. That is, such aesthetic data should be understood as anatomical data, which indicate specific regions of the user's face.

Emotional and/or social data/messages, on the other hand, seek a broader motivation from the user than aesthetic data, that is, if when providing aesthetic data the user indicated a specific portion of the face they wish to treat, when providing emotional data/messages the user will provide the reason why they wish to treat that particular aesthetic data.

More specifically, the user must indicate the reason that led them to choose that particular aesthetic data for treatment, that is, the user should state why they wish to treat that specific aesthetic data.

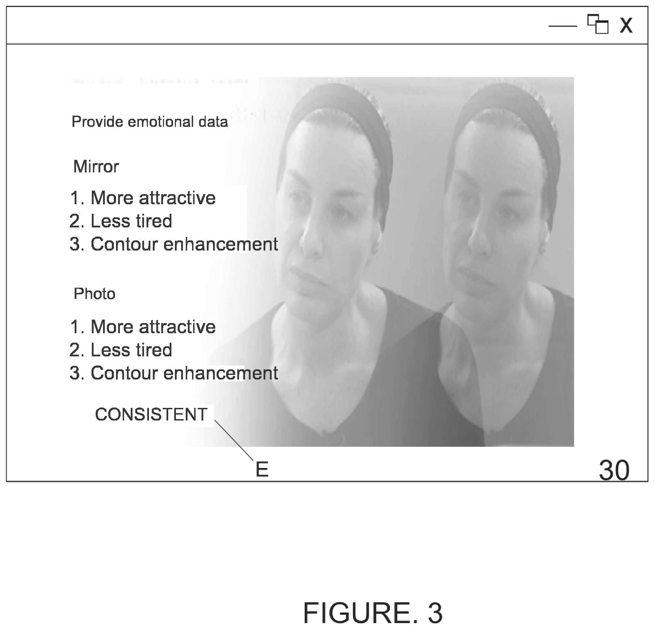

Thus, and now with reference to the exemplary representation of FIG. 3, it is noted that the first set of emotional data provided by the user refers to the following emotional data: (i) more attractive appearance, (ii) less tired appearance, (iii) more defined appearance (enhancement of contours).

Similarly, the second set of emotional data/messages provided by the user refers to the following emotional data: (i) more attractive, (ii) less tired, (iii) enhancement of contours.

Thus, this means that the user wishes to treat the upper eyelid, forehead lines, and nasolabial fold to appear more attractive, less tired, and more defined. That is, at this stage of the proposed methodology, a correlation occurs between the set of data/emotional messages between the impulsive and graphical analysis, which did not occur previously regarding the aesthetic data.

However, treating only the aesthetic data (areas/regions of the face and/or body) that the user indicates they wish to address, such as the upper eyelids to appear more attractive, the nasolabial folds to look less tired, and the temple to enhance facial contours, will not necessarily make it possible to achieve the objectives of the emotional data/messages, objectives which would require a broader facial and/or body treatment. Thus, the methodology and system described in the present invention could indicate other treatment areas/regions in order to achieve the user's desired outcomes.

Additionally, and as proposed when obtaining the set of aesthetic data, it is further proposed to include the step of evaluating, in the comparator module, whether there is consistency between each of the emotional data from the first set of emotional data and each of the emotional data from the second set of emotional data.

Based on the example of FIG. 3, it is observed that a consistency was detected, since emotional data 1 to 3 from the first set of emotional data are the same emotional data 1 to 3 from the second set of emotional data.

Thus, once consistency is detected, a perceptible representation is provided to the user, as indicated in step E illustrated in FIG. 3. Furthermore, once consistency has been detected, it is also proposed to transform the first set of emotional data and the second set of emotional data into a final set of emotional data.

Alternatively, if a consistency is not detected, it is further proposed to carry out the step of prompting the user, through the computing platform, to enter the final set of emotional data. Thus, the user will be alerted, through a perceptible representation, about the detected inconsistency and will subsequently be prompted to provide the final set of emotional data. The handling of unfavorable or negative messages will be prioritized.

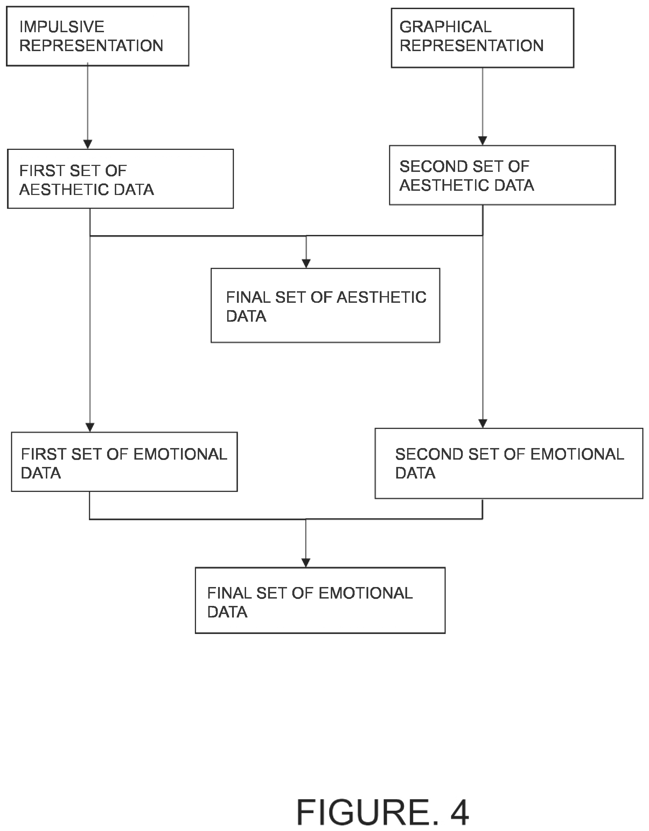

Thus, from the previously provided description and the illustration of FIGS. 2 and 3, and still based on the illustration of FIG. 4, a final set of aesthetic data and a final set of emotional data will be obtained. It is proposed that the final set of aesthetic data and the final set of emotional data be stored on the computing platform, so that these may be considered for the future generation of the treatment plan. The diagnostic evaluation to be performed by the system will assist in prioritizing the areas and messages to be addressed, so that the evaluation made by the system may differ from those indicated by the user, since the user's evaluation may present a distorted evaluation of their own image. For example, requesting excessive treatments that may worsen your appearance instead of improving it. The user will be warned of this possibility.

In addition to emotional data, the computer method will also enable the step of obtaining, from a socializing block, a plurality of positive social data (favorable) linked to the user and a plurality of negative social data (unfavorable) linked to the user's appearance.

Positive social data is understood as the appearance that the user would like to achieve, based on a social aspect. For example, positive social data can be understood as: confident, caring, and friendly. In this case, such positive social data could have been provided if the user has the profession of a nurse; thus, the user would like to have a more confident, caring, and friendly appearance.

It is proposed that the socializing block comprise eight positive social data and eight negative social data, so that the user must provide a certain amount of positive and negative social data to proceed with the methodology. By way of example, when analyzing the graphic data (photo), the user will provide negative social data about their appearance that they would like to have corrected or improved and will determine positive social data that they would like to convey with their appearance after the treatment.

Also by way of example, positive social data may be considered as: a more trustworthy, confident, determined, friendly, kind, active, cultured, and optimistic appearance. Negative social data, on the other hand, may be considered as: appearance: untrustworthy, insecure, fragile, antisocial, indifferent, discouraged, inexperienced, and pessimistic.

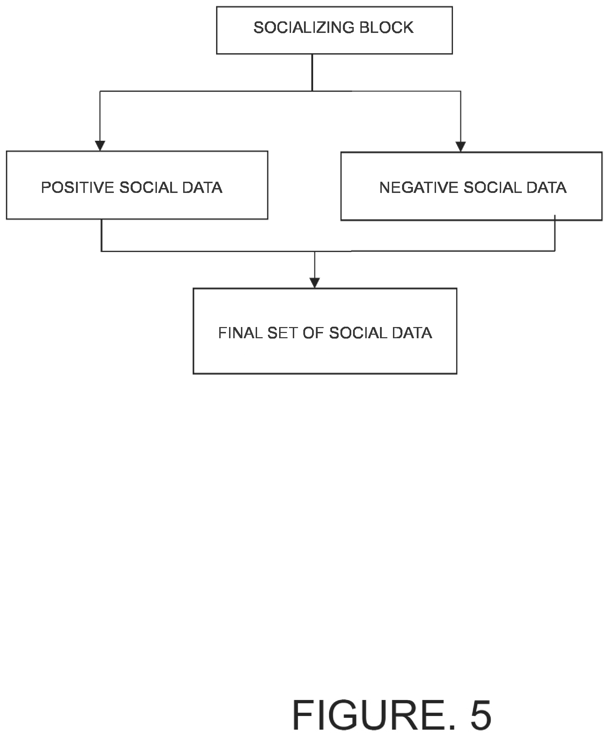

Once the positive and negative social data have been obtained, it is further proposed to carry out the step of consolidating the plurality of positive social data and the plurality of negative social data into a final set of social data, as can be seen from FIG. 5.

Thus, based on the illustration of FIGS. 3 to 6, the output of the described methodology will be the final set of aesthetic data, the final set of emotional data, and the final set of social data. Such data will later be used to analyze the feasibility of delivering the desired result. The treatment plan will prioritize addressing the user's negative emotional messages about their appearance.

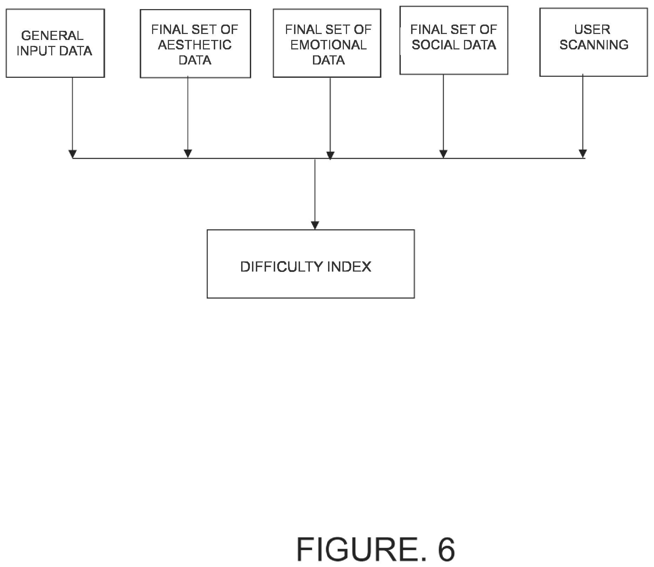

Additionally, and still with reference to FIG. 6, the methodology and system described in the present invention further comprise the step of generating a difficulty index based at least on the evaluation of the final set of aesthetic data, the final set of emotional data, and the final set of social data. More specifically, the difficulty index can be understood as a *score* that indicates, to the user and the applicator, the existing difficulty for applying the treatment plan that is being generated based on the final set of aesthetic, emotional, and social data.

It should be noted that, for generating the difficulty index, at least the content of the user's general input data, such as, for example, their age, must also be considered. Thus, in a hypothetical situation, a user of older age or with significant asymmetries tends to have a difficulty index generated with a higher *score*.

It should be noted that any data previously provided by the user may be considered for generating the difficulty index, such as, for example, consultation data. Furthermore, it should be noted that, preferably but not restrictively, the difficulty index should be generated after scanning the user's face.

It is proposed that the difficulty index be displayed to the user through the computing platform. Thus, if the computing platform is understood as a computer, tablet, cell phone, or television, the aforementioned difficulty index will be displayed on the screen of the said computing platform.

The difficulty index can be understood as a numbering, such as a numbering that starts at 0 and goes up to 100, such that a difficulty index with the maximum value of 100 indicates that the difficulty in implementing that treatment plan is at its maximum.

Alternatively, or additionally, the difficulty index may be configured as a text and color representation, so that, in this case, the difficulty index may be represented by the arrangement of the texts "LOW," "MEDIUM," and "HIGH." Additionally, such texts can be displayed on the screen of the computing platform in certain colors; for example, the difficulty index "LOW" could be displayed in green, the difficulty index "MEDIUM" can be displayed in yellow, and the difficulty index "HIGH" can be displayed in red.

Any form of representation and generation of the difficulty index would be acceptable, such as a representation in video, text, animation, colors, as well as a combination of these.

It is proposed that the difficulty index is also generated from a user scan, as further indicated in FIG. 6. It is thus understood that the methodology proposed in the present invention comprises the step of scanning (or any other form of graphical or digital analysis, such as the analysis of photos and/or videos), on the computing platform, at least one portion of the user. The user's portion can be understood, without limitation, to be the user's face.

Thus, through the computing platform, the scanning of the user's portion will be performed. To do so, the input block 31 of the computing platform must be activated, such as the camera of said platform.

The step of scanning at least a portion of the user through the computing platform further comprises the step of scanning said portion of the user in both horizontal and vertical directions, also considering a frontal portion, an oblique portion, and a profile portion of the user.

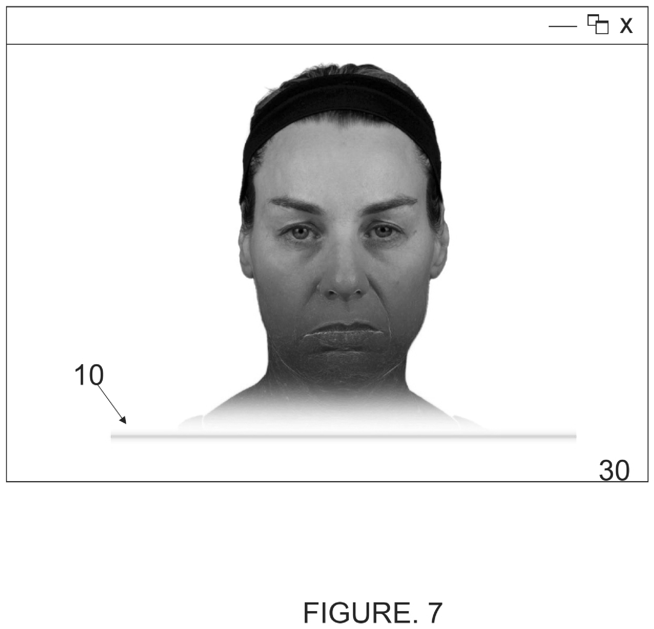

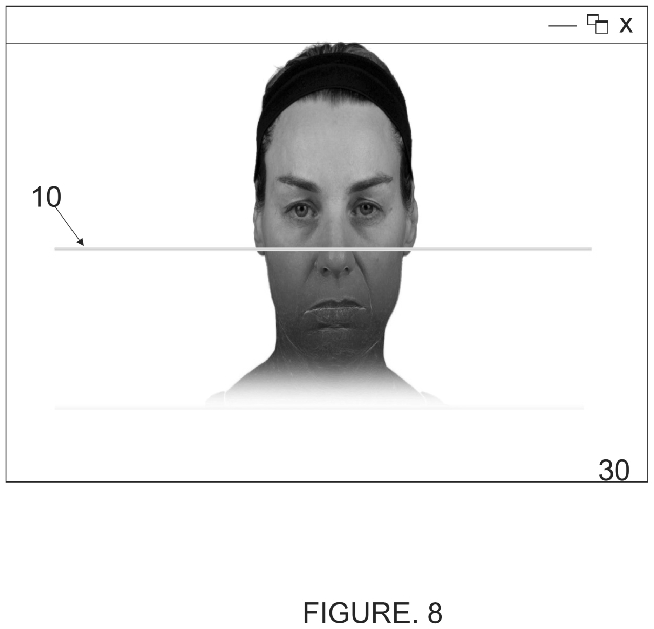

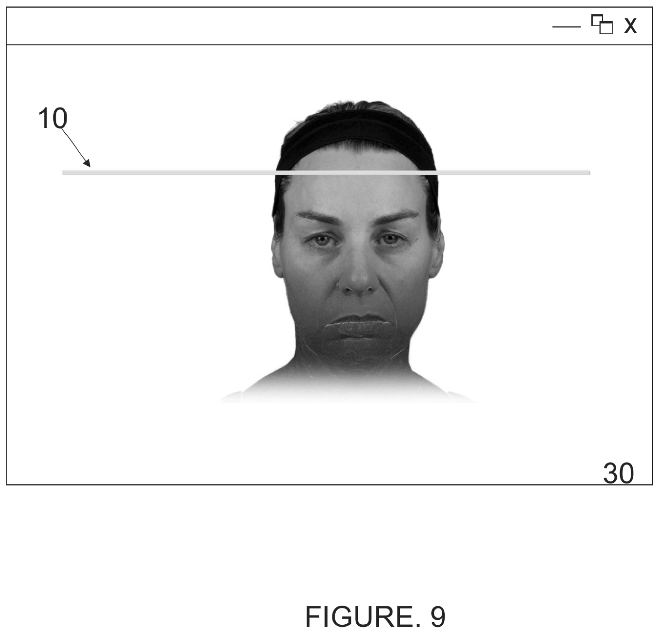

FIG. 7 is a representation of the computing platform indicating the execution of the horizontal scanning step of the user's portion, in which, in FIG. 7 the horizontal scanning line is indicated by the numerical reference 10. Thus, the horizontal scanning line 10 will cover the entire height of the user's portion at least once. FIGURES 8 and 9 illustrate the movement of the horizontal scanning line 10 across the user's face. In one embodiment, it is proposed that the user's face be segmented into a plurality of quadrants, and as the scanning occurs, a specific index (value) is assigned to each of the quadrants of the plurality of quadrants.

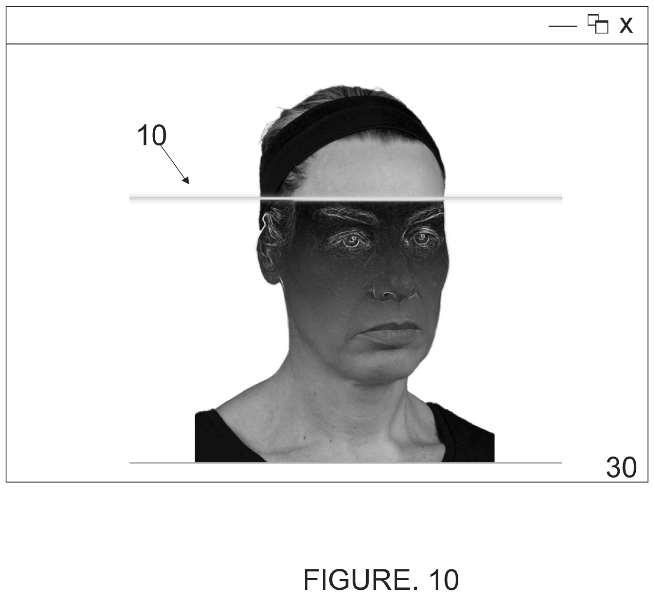

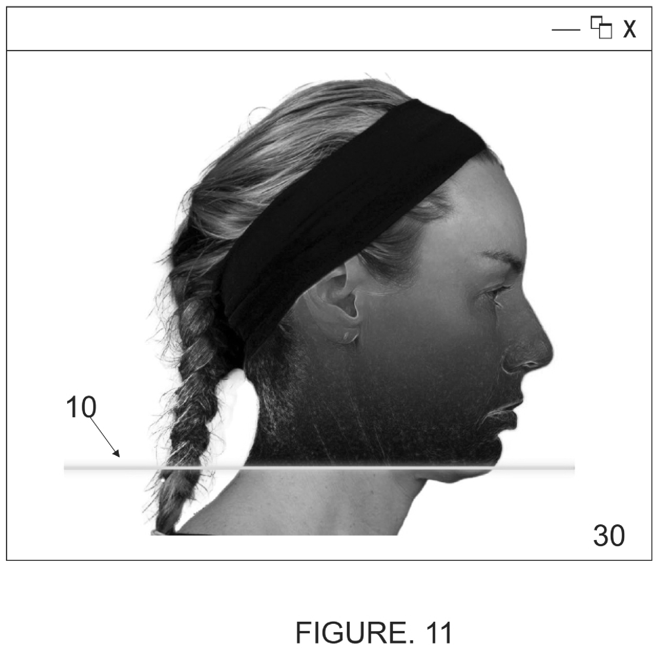

Thus, it is proposed that the user's horizontal scanning be performed considering a frontal portion of the user (FIG. 7), an oblique portion (FIG. 10) as well as a portion of the user profile (FIG. 11). Scanning can be performed using the graphic data (photo) at rest and in various expressions. Video capture may also be considered for the dynamic evaluation of the user.

Similarly, in addition to scanning through the horizontal scanning line 10, the described methodology proposes that vertical scanning of the user will also be performed, that is, scanning through a vertical scanning line 11.

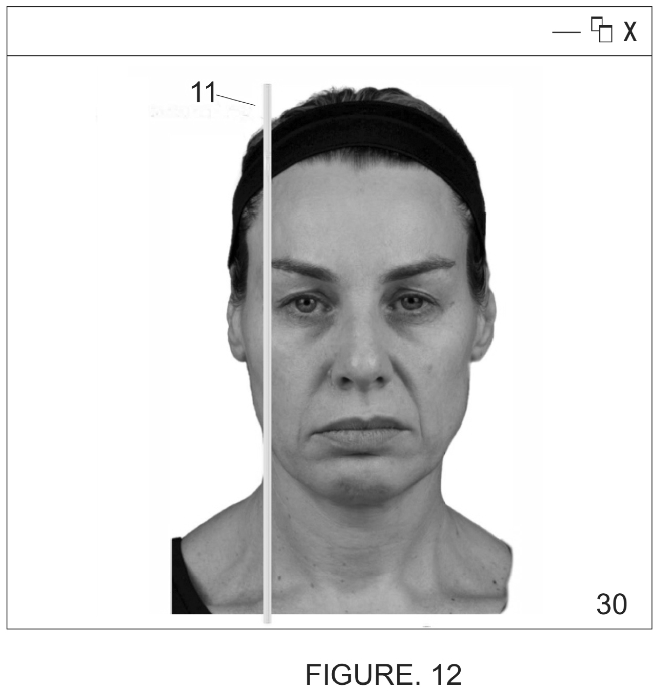

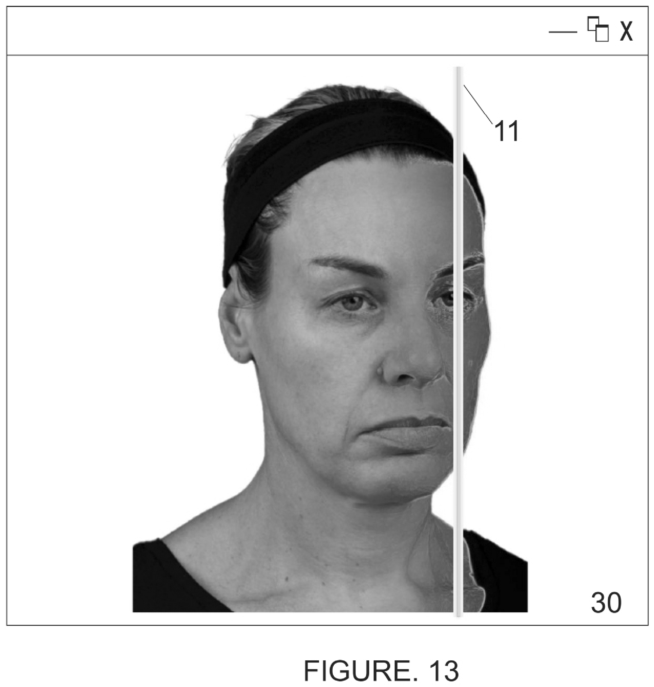

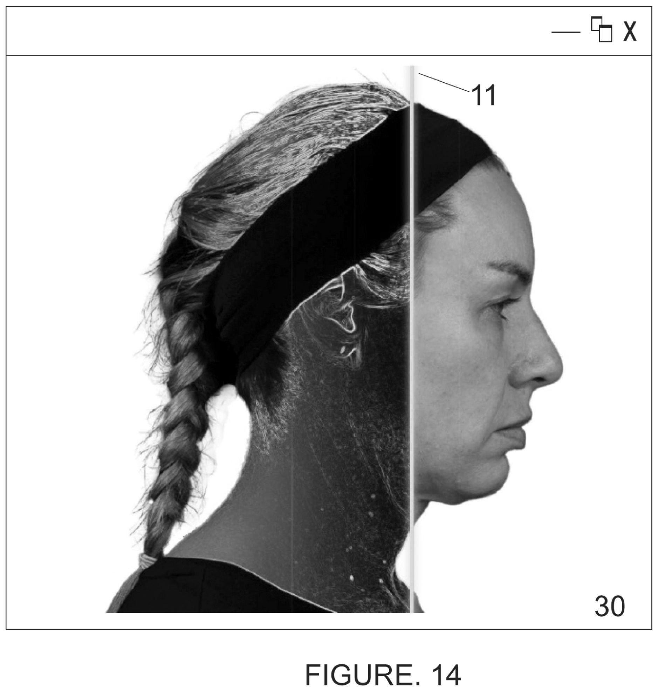

Thus, the user's vertical scan 11 must be performed from the front, oblique, and profile views, as illustrated in FIGS. 12, 13, and 14, respectively. Similarly, the user's face can be segmented into a plurality of quadrants and then a certain index (value) can be assigned to the quadrants as the scanning occurs.

Additionally, in addition to the scanning as previously described and illustrated in FIGS. 7 to 14, it is established that the step of scanning the user portion further comprises the step of segmenting the user portion into a plurality of segments. Thus, the computing platform will generate a plurality of slices in the user's portion and from the horizontal and vertical scanning.

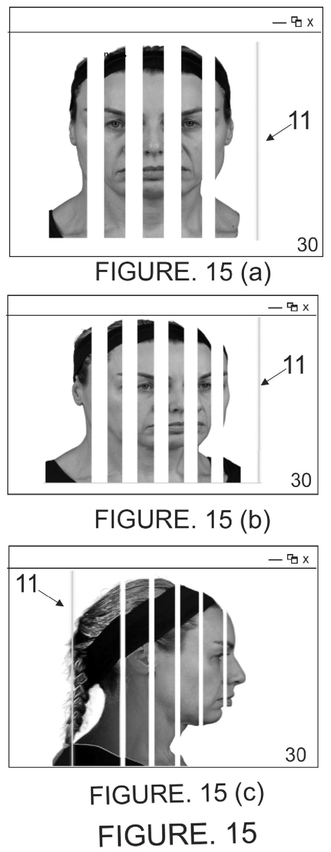

In this way, and now making reference to FIG. 15, the computing platform will segment the user's portion into a plurality of segments and from the scanning of the frontal, oblique, and profile portions. Thus, in FIG. 15 the segments generated from the vertical scanning line 11 for scanning the frontal, oblique, and lateral portions, as shown in Figures 15 (a), (b), and (c), respectively.

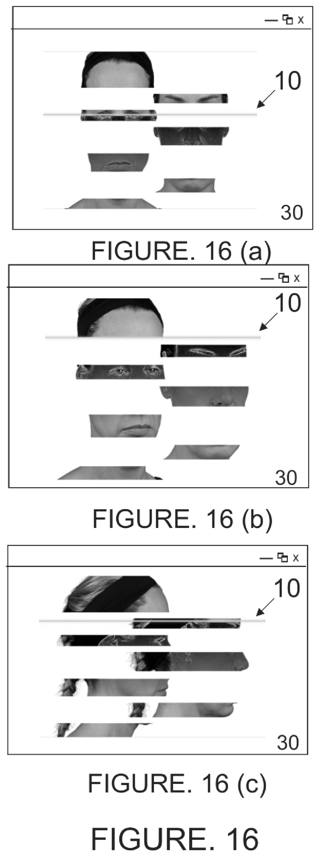

Similarly, the computing platform will also generate the plurality of segments from the scanning through the horizontal scanning line 10, as illustrated in FIG. 16. Thus, the segments for scanning the frontal, oblique, and lateral portions are provided, as shown in Figures 16 (a), 16 (b), and 16 (c).

The methodology described in the present invention further comprises the step of correlating the scanning of the user's portion with at least one among a plurality of virtual positive social data, a plurality of virtual negative social data, and a set of virtual emotional data linked to the user.

Virtual data should be understood as data generated by the computing platform itself, and not provided by the user.

For example, in the description referring to FIG. 3, it was described that the emotional data indicated in the figure was provided by the user, that is, these are desires expressed by the user themself.

Virtual emotional data, on the other hand, should be understood as emotional data evaluated (generated) by the computing platform itself, that is, the computing platform will, based on scanning the user's face, indicate which emotional data should be considered for that user. In other words, the computing platform will, through scanning the user's face, assess what that user's needs are, such as, for example, having a less tired, less sad, less angry appearance, or having less sagging, etc.

Thus, virtual data should be understood as data (indications) generated by the computing platform itself from the scanning of the user's face.

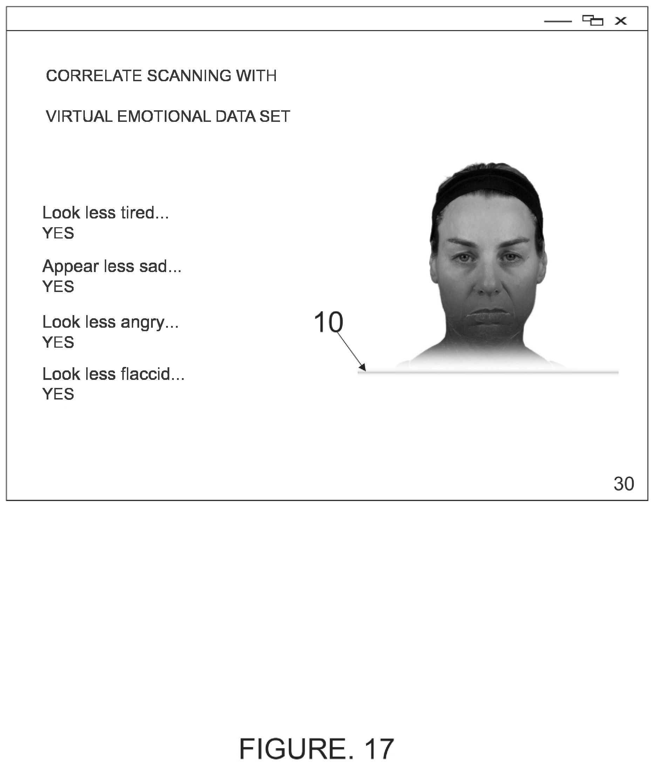

Taking as reference the illustration of FIG. 17, it can be understood that the step of correlating the scanning of the user's portion with a set of the user's virtual emotional data further comprises the step of scanning a portion of the user and determining the set of virtual emotional data linked to the user.

Thus, in FIG. 17, the user's face is scanned, such as scanning through the horizontal scanning line 10 and the subsequent determination of the set of virtual emotional data. More specifically, the determination of the virtual emotional data set indicates which emotional data should be considered in the treatment plan to be generated for that user.

Thus, for each virtual emotional/social data point, an alert indication will be generated for the prioritized correction of both emotional and social unfavorable messages to be considered in the treatment plan.

In this way, it is understood that the computing platform will comprise a set of virtual emotional/social data previously stored in its memory, so that, for each of these data, and based on the scanning of the user's face, a correction indication shall be generated.

In one embodiment of the present invention, at least the following sets of virtual emotional data should be stored on the computing platform: "appear less tired," "appear less sad," "appear less angry," and "appear less sagging," among others. Thus, for each of these data, an indication must be generated, thereby alerting the user to the need for treatment.

Thus, FIG. 17 shows the execution of the user's face scanning step as well as the indication, by the computing platform, from a suggestion (alert indication) that the user in question should have a less tired, less sad, less angry appearance and with less sagging.

Thus, the system suggests that the patient should have a less tired, less sad, less angry appearance, and less sagging, so that, as shown in FIG. 17, in this exemplary embodiment, the computing platform generated an alert indication for all considered virtual emotional data. It is noteworthy that the form of illustration and representation of the warning indication as shown in FIG. 17 should only be considered as an exemplary form of representation, that is, it should not entail any limitation to the scope of protection of the present invention.

Having generated the alert indications from the set of virtual emotional data, it is proposed, by way of example, that a priority list be generated by the computing platform for the considered virtual emotional data. In this embodiment, said priority list could comprise the following virtual emotional data: appearing less tired, appearing less sad, appearing less angry, and appearing less flaccid. As described above, for each of the virtual emotional data considered, an alert indication must be issued by the methodology and system described in the present invention.

Continuing with the description of the methodology proposed in the present invention, the computer method for obtaining a treatment plan related to a user further comprises the step of performing, through the computing platform, a dynamic scan of the user, wherein the step of performing a dynamic scan of the user further comprises the step of comparing portions of the user in a static state and in a dynamic state.

In this way, the computing platform, through its processing unit, will scan a portion of the user in two different states, a first state being understood as a static state and a second state being understood as a dynamic state.

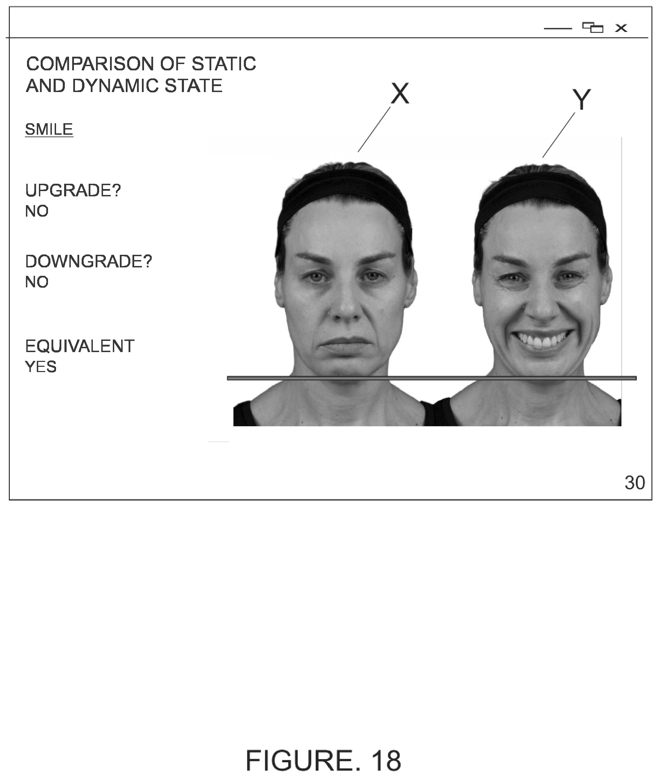

Thus, taking FIG. 18 as a reference, the user's face is scanned in a static state X and in a dynamic state Y, and both scans are compared in order to generate an aesthetic indicator that can assume a treatment state and a non-treatment state. In FIG. 18, the aesthetic signal is generated, motivating the user to smile. Thus, the methodology and system described in the present invention determine whether, during various expressions (dynamic state), the user shows improvement (*upgrade*) or worsening (*downgrade*) of overall appearance, or if equivalence is maintained on a pre-established scale. For example, said pre-established scale may be a scale represented by an index ranging from 0 to 10, with, for instance, index 0 being the worst possible aesthetic condition and index 10 being the ideal aesthetic condition for the age, gender, ethnicity, among other factors. Said index could be assigned, for example, by segmenting the face into a plurality of quadrants.

More specifically, the computing platform compares the user's portion in the static state X and in the dynamic state Y and determines whether there was an aesthetic improvement (non-priority treatment state), an aesthetic worsening (treatment state), or if equivalence was maintained (state to be considered in the treatment, but not a priority state).

If the aesthetic indicator assumes a treatment state (or a state to be considered in the treatment), it is further proposed to carry out the step of generating at least one treatment code to be considered in the user's treatment plan.

For the generation of the aesthetic indicator, it is proposed that, when scanning the user's portion in the static state X, a static index will be generated by the computing platform. Similarly, when scanning the user's portion in the dynamic state Y, a dynamic index will be generated by the computing platform. In summary, the static index and the dynamic index can be understood as values, numbers, that represent the state of the dynamic and static index.

Thus, once the aforementioned static and dynamic indices are generated, a comparison between them is performed and it is then assessed whether there is an aesthetically positive variation (*upgrade*), an aesthetically negative variation (*downgrade*), or if an equivalence has been maintained. This comparison can be performed globally, that is, for the entire face of the user, or it can be performed by segments (by quadrants) of the user's face.

Thus, if an aesthetically negative variation (*downgrade*) is detected, the aesthetic indicator should be assigned the treatment state, and, consequently, if an aesthetically positive variation (*upgrade*) is detected, the aesthetic indicator should be assigned the non-treatment priority state.

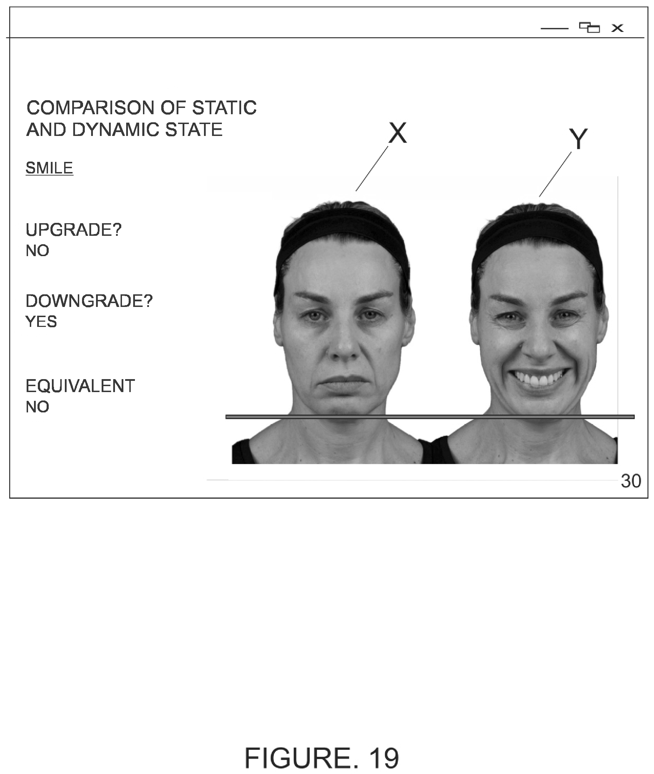

In one example, and based on the illustration of FIG. 19, consider that the static scan of user X generated a static index with a value of 5, while the dynamic scan of user Y generated a dynamic index with a value of 4, thus, the comparison performed indicates that a *downgrade* occurred, that is, an aesthetically negative variation, such that, in this case, the aesthetic indicator is assigned a treatment state. If a loss of comparative numerical value is observed, the treatment indication is referred to as 'correction'; if there is maintenance or improvement of the numerical value, the designation will be prevention or enhancement of the area/region/message in question.

In one embodiment, and as will be described in more detail below, the comparison between the static state X and the dynamic state Y could be performed by segmenting the user's face into a plurality of quadrants. Thus, the user's face must be segmented into a plurality of quadrants, both for the static state X and for the dynamic state Y.

Thus, it is possible to assign indices (values) to each of these quadrants in the static state X (or to a set of quadrants) and then compare it with its respective quadrant (or with the respective set of quadrants) in the dynamic state. In this way, by comparing the indices, it is possible to determine whether there was a positive, negative, or equivalent aesthetic variation.

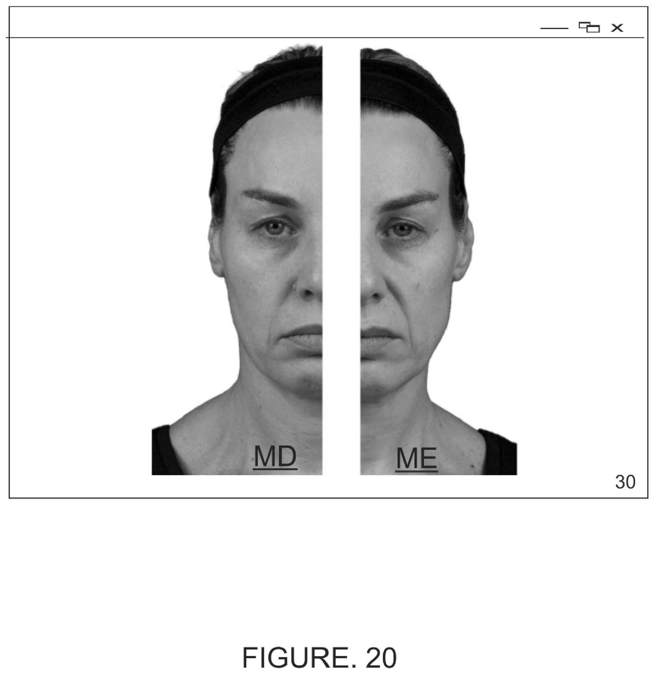

With further reference to the illustration of FIG. 20, the methodology proposed in the present invention further comprises the step of mirroring at least a portion of the user and comparing a first mirroring of the user with a second mirroring of the user.

More specifically, it is proposed that the user's portion, in this case, their face, be segmented vertically into two halves, that is, a left half LH and a right half RH, as illustrated in FIG. 20. Subsequently, the step of forming a first representation of the user from the left half LH and forming a second representation of the user from the right half RH must be carried out.

In other words, the user's face should be formed from two left halves LH and the user's face should be formed from two right halves RH. To do this, the left and right halves must be mirrored so that the correct formation of the face is possible.

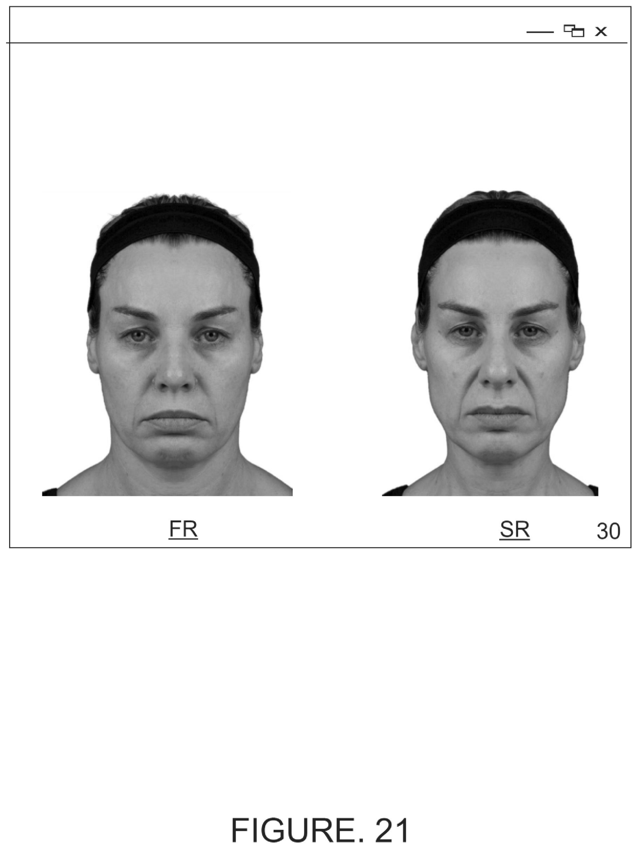

Thus, FIG. 21 illustrates the first and second representation of the user, in which the first FR representation of the face is formed by two right halves RH (also referred to as 2 x RH) as well as the second SR representation of the face formed by two left halves LH (also referred to as 2 x LH).

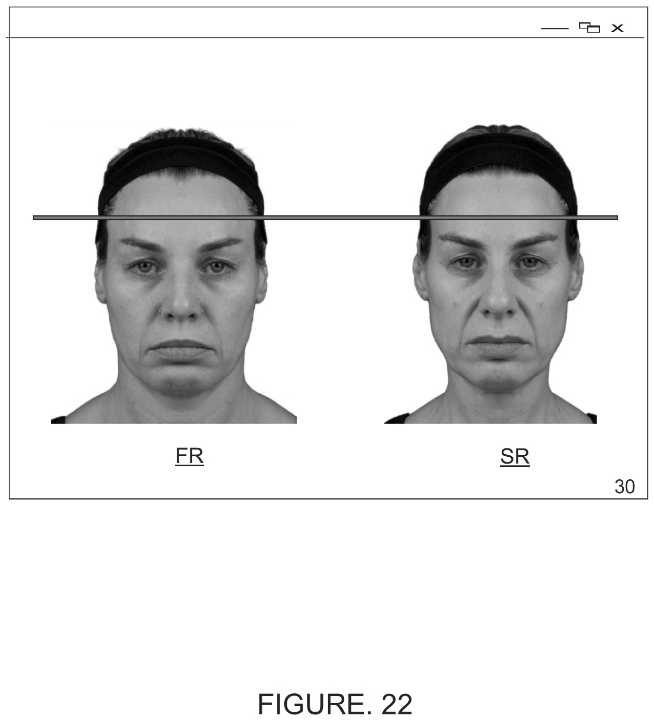

Subsequently, and now with reference to FIG. 22, the step of scanning the first representation FR as well as scanning the second representation SR is performed and identifying which representation is aesthetically more favorable (if any), thus determining a final representation.

In other words, from the scanning of the first representation FR and the second representation SR, one of these representations can be chosen, such that the chosen representation will be the one that is aesthetically more favorable. If both the first representation FR and the second representation SR are discarded, it is possible to proceed with the user's current representation, that is, the representation formed by their left half and their right half. In this case, the user's face can be segmented into a plurality of quadrants, as previously described.

If either the first representation FR or the second representation SR is chosen, said representation can be used as a *benchmark*, that is, a standard, such as a portion of the user considered ideal or suitable for the user in question.

The purpose of the scanning step of the user's first representation FR and second representation SR is to assess whether there is facial symmetry of the user based on such representations. Thus, the first representation FR is initially compared with the second representation SR, and then it is determined which representation illustrates the user's face in a more symmetrical manner. To this end, the performance of a horizontal scan of each of these representations is proposed, as illustrated in FIG. 22.

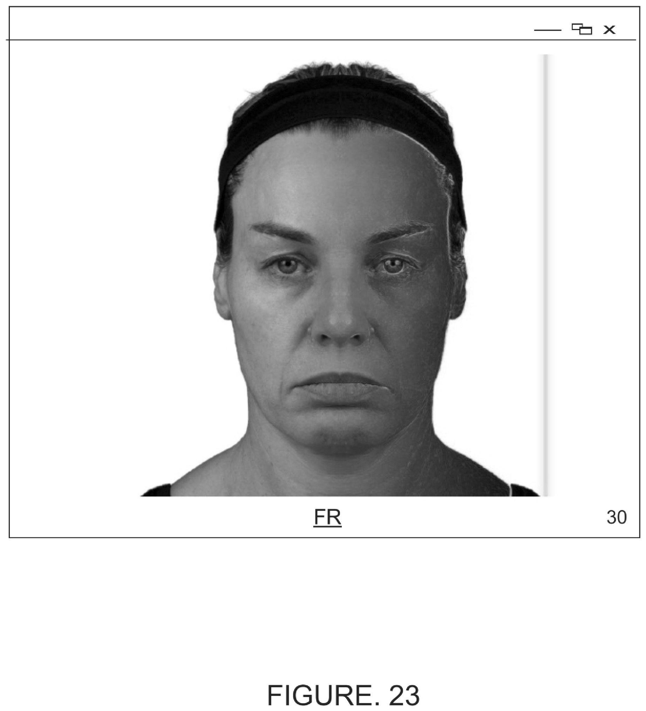

In the example illustrated in FIG. 22, the second SR representation (formed by the two left halves) was excluded, so the evaluation proposed is of the first FR representation (formed by two right halves) based on a vertical scan, as illustrated in FIG. 23. The objective in performing the vertical scanning of the second representation SR is to assess whether there is a favorable or unfavorable indication in relation to the user's actual image.

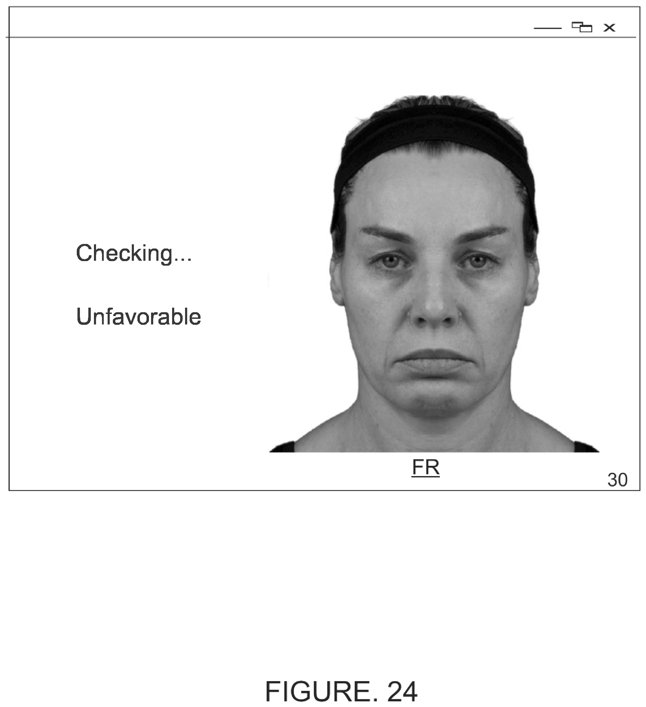

Thus, after the vertical scanning, as shown in FIG. 23, the user will be provided, through the computing platform, with information on whether any side of the face, when analyzed in mirroring, shows a significant improvement compared to the real face. In the example illustrated in FIG. 23, and also making reference to FIG. 24, there is an unfavorable indication, that is, no improvement was observed in the analyzed representation. Thus, since both the representation formed by the two left halves LH (2 x LH) and the one formed by the two right halves RH (2 x RH) were disregarded, the user's actual representation must be evaluated, that is, the one formed by their left face and their right face.

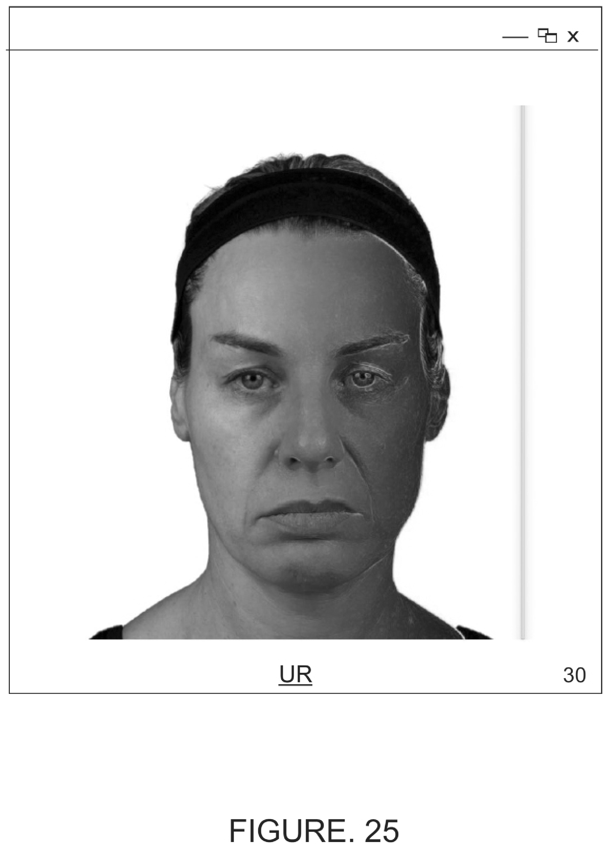

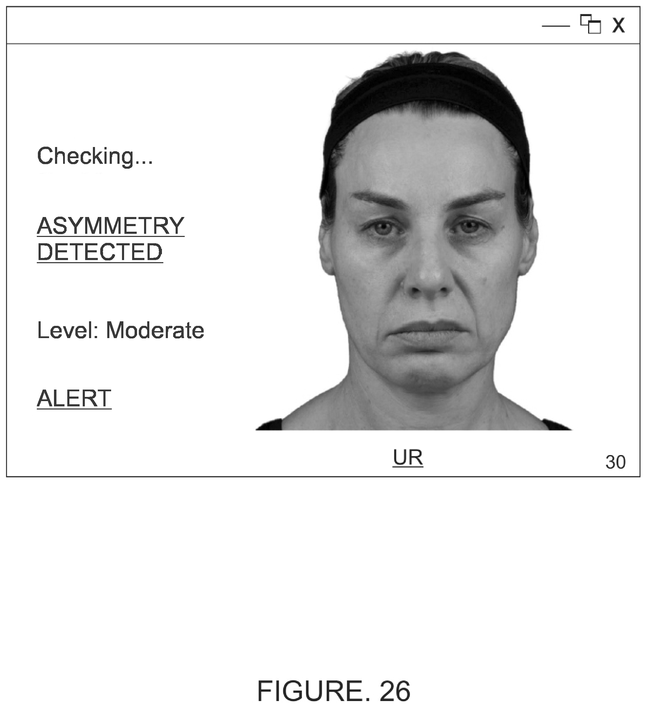

Thus, FIG. 25 represents the vertical scanning of the usual user representation UR, with a view to assessing whether there is a favorable indication (there is symmetry) or an unfavorable indication (there is no symmetry) in the considered representation. Based on the representation of FIG. 26, an unfavorable indication is observed through the computing platform, that is, there is no symmetry in the user's face. In such an evaluation, and as with any step related to scanning the user's face, the face could be segmented into a plurality of quadrants and indices (values) could be assigned to each of these quadrants.

In the present example, since both the 2 x LH and 2 x RH representations had already been considered unfavorable to serve as a model, this fact indicates a tendency for increased technical difficulty of the procedure, thus affecting the difficulty index. If one of the user's sides (either 2 x LH or 2 x RH) had been considered as a superior side compared to the actual one, then the side with the best aesthetic appearance could serve as a guide (*benchmark*), that is, a model considered ideal.

In this case, the unfavorable indication related to the user's facial symmetry provides an alert to the user (and to the medical or healthcare professional) regarding the treatment plan under consideration.

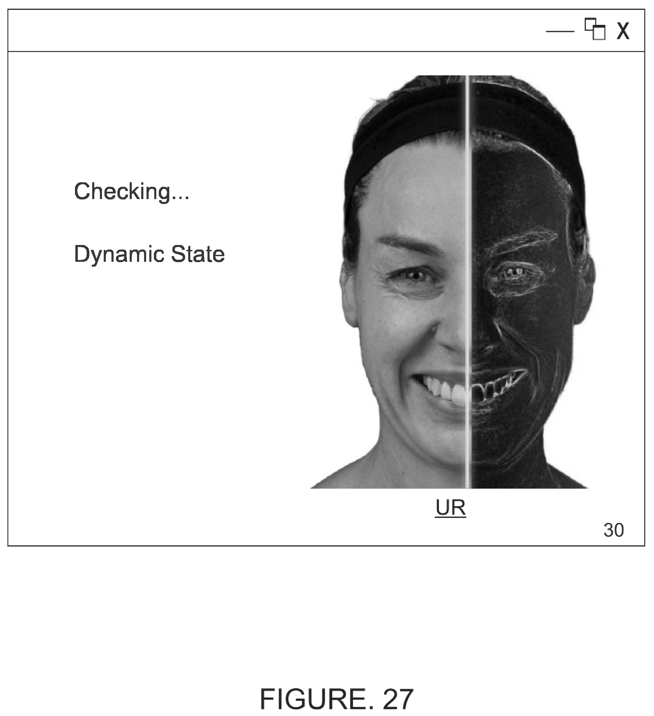

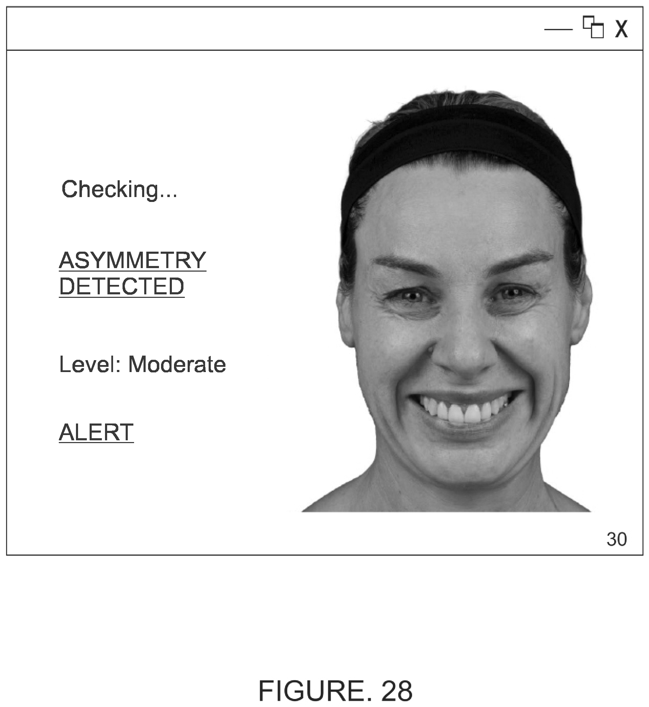

It should be noted that the evaluation of asymmetry must be carried out, for the representation considered (in this case, the usual representation), both in a static manner and in a dynamic manner, that is, considering that the user is both at rest and, for example, smiling, as shown in the FIGS. 27 and 28. Obviously, other expressions, besides a smile, could be considered. It is noted that in the examples illustrated in FIGS. 26 to 28, asymmetry was detected both at rest and in a dynamic state, which tends to increase the level of difficulty associated with the treatment.

Subsequently, user scanning can be initiated based on the next hierarchies, for example, such as the H2, H3, H4, and H5 hierarchies discussed in patent US 11,602,303.

More specifically, scanning from the H2 hierarchy will consider the following evaluation parameters: ideal, favorable, acceptable, unfavorable.

Accordingly, the user's portion will be scanned based on the H2 hierarchy and, for each scan performed, a specific evaluation parameter will be reported through the computing platform.

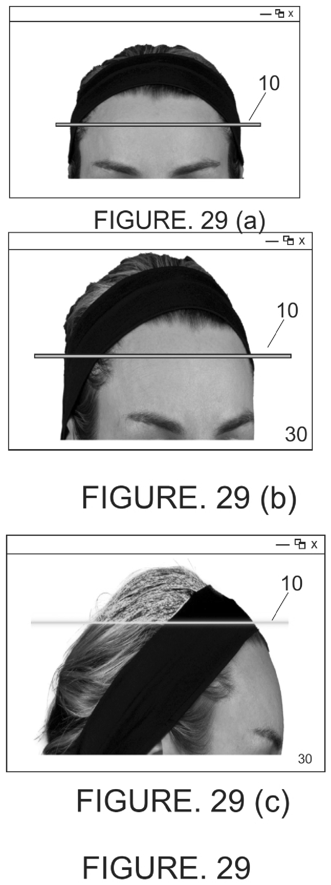

In this way, and taking FIG. 29 as a reference, it is proposed that, for the upper third of H2, the user's portion should be scanned in the frontal (Fig. 29 (a)), oblique (Fig. 29 (b)), and lateral (Fig. 29 (c)) views, and thus return an evaluation parameter. In one example, the evaluation parameter returned after scanning the upper third of H2 is the parameter "favorable". When a favorable parameter is being treated, if the user so desires, we are promoting 'beautification'.

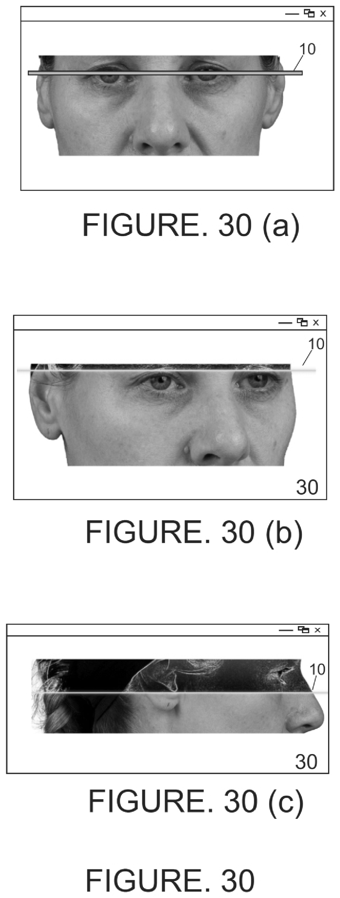

Similarly, the same steps are performed for the middle third of H2, thus scanning the user's portion in the frontal (Fig. 30 (a)), oblique (Fig. 30 (b)), and lateral (Fig. 30 (c)) views, thereby returning an evaluation parameter. In one example, the evaluation parameter returned after scanning the median third of H2 is the "unfavorable" parameter. When treating an unfavorable parameter, we are promoting a 'correction' of that facial third.

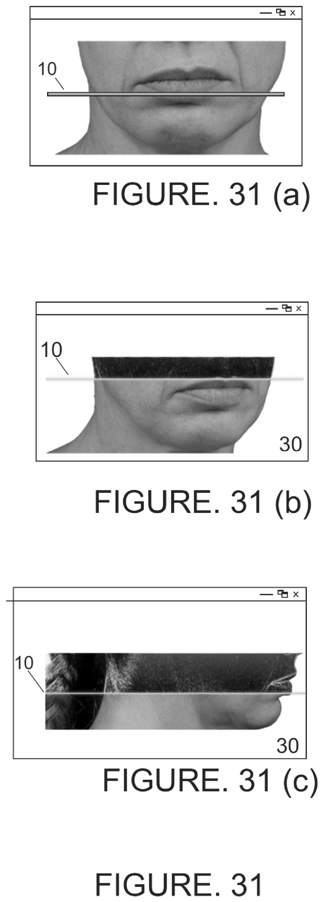

Similarly, the same steps are performed for the lower third of H2, thus scanning the user's portion in the frontal (Fig. 31 (a)), oblique (Fig. 31 (b)), and lateral (Fig. 31 (c)) views, thereby returning an evaluation parameter. In one example, the evaluation parameter returned after scanning the lower third of H2 is the "unfavorable" parameter.

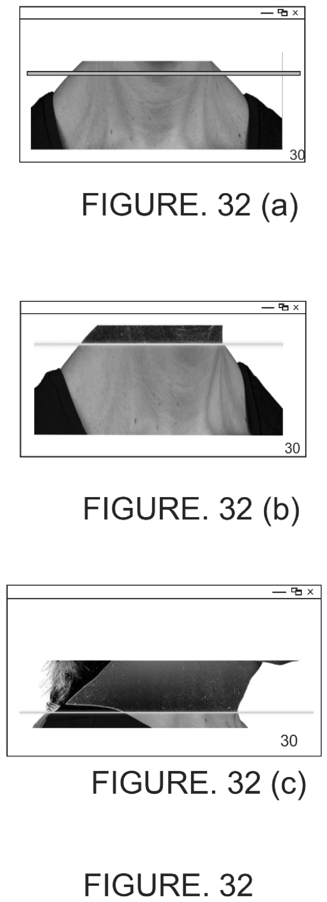

Similarly, the same steps are performed for the user's neck, considering the H2 hierarchy, thus scanning the user's neck in the frontal (Fig. 32 (a)), oblique (Fig. 32 (b)), and lateral (Fig. 32 (c)) positions, thereby returning an evaluation parameter. In one example, the evaluation parameter returned after scanning the user's neck is the parameter "acceptable". When dealing with an 'acceptable' parameter, we are promoting the 'prevention' of the considered region.

To obtain the evaluation parameters, the user's face can be segmented into a plurality of quadrants, and thus indices can be assigned to each of the quadrants, which will correspond to the evaluation parameters. By way of example only, the ideal evaluation parameter may be assigned a maximum index (value), the unfavorable evaluation parameter may be assigned a minimum index (value), and the other parameters (favorable and acceptable) may be assigned intermediate values, between the maximum and the minimum.

Subsequently, it is proposed to scan the user's face based on the H3 hierarchy, so that said scanning should consider the following evaluation parameters: aestheti-cally positive variation (*upgrade*), aesthetically negative variation (*downgrade*), and aesthetically equivalent variation (equivalent).

Thus, in this step, portions of the user are compared in a static state and in a dynamic state, such that static state is meant to be the user's face at rest and, dynamic state is meant to be the movement of the user's face.

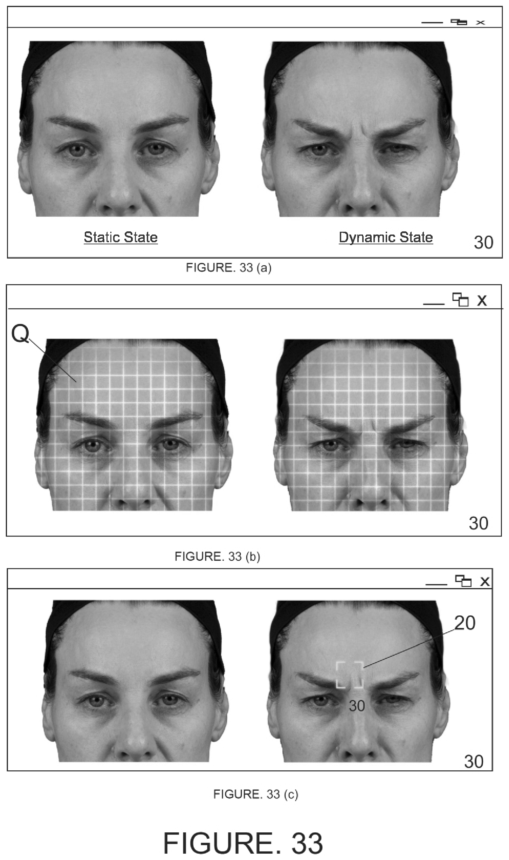

In this way, and taking the illustration of FIG. 33 as a reference, the user's portion is compared in the static state and in the dynamic state, in which, in this case, the dynamic state is understood as the act of the user frowning.

More specifically, it is proposed that through the computing platform, the user's face is segmented into a plurality of quadrants Q, as illustrated in Fig. 33 (b), and, for each of these quadrants, a static index (for the resting state) and a dynamic index (for the animated state) are assigned. Thus, the computing platform must compare each static index and each dynamic index for each of the quadrants and thereby assess whether there was an aesthetically positive variation (*upgrade*), an aesthetically negative variation (*downgrade*), or an aesthetically equivalent variation.

It is understood that the occurrence of an aesthetically negative variation indicates the generation of an aesthetic indicator that will receive a treatment state. On the other hand, the occurrence of an aesthetically positive variation indicates the generation of an aesthetic indicator that receives a non-priority treatment status, but if the treatment is performed, the objective becomes preventive or beautifying. Similarly, the occurrence of an aesthetically equivalent variation indicates the generation of an aesthetic indicator that receives a non-priority treatment status, but treatment is also considered for prevention or beautification purposes, if desired by the user. If the user wishes to treat a region, area (quadrants or set of quadrants) for which an aesthetic non-treatment flag was initially assigned, said aesthetic flag must be updated, thus assigning it an aesthetic treatment flag.

Thus, when it is detected that an aesthetically negative variation has occurred, an aesthetic marker 20 can be displayed on the computing platform in the respective quadrant that originated said aesthetically negative variation, thereby providing an indication to the user that this point on the face will be targeted for treatment. It should be noted that an example of the aesthetic marker 20 is illustrated in Fig. 33 (c), in which it is, by way of example, represented as a square.

Such a form of representation of the aesthetic marker should not be considered as a limiting form of representation, so that any other form of representation would be fully acceptable. Otherwise, despite FIG. 33(c) To illustrate only one aesthetic marker, it is noted that a plurality of aesthetic markers could be illustrated on the computing platform.

Thus, the steps discussed above and illustrated in FIG. 33 should be carried out for certain dynamic states of the user and, for example, for each of the referenced segments of hierarchy H3. Such that, having FIG. 33 illustrated the dynamic state understood as the act of frowning, it is proposed that the same methodology now be applied to the dynamic state of raising the eyebrow and smiling, among others.

Thus, for each of these dynamic states, the user's face must be segmented into the aforementioned quadrants and, from these, the static indices and dynamic indices must be assigned and then the appropriate comparison performed, thus resulting in the evaluation of whether there is an aesthetically positive, negative, or equivalent variation.

It should be noted that the comparison between each of the quadrants of the static index and the dynamic index can be performed individually, that is, each quadrant of the static index will be compared with its respective quadrant of the dynamic index, or it can also be performed in groups, grouping a certain number of quadrants of the static index and comparing them with their respective equivalent quadrants of the dynamic index.

Thus, at the end of the comparison between the static state and the dynamic state, data will be obtained indicating the existence of an aesthetically positive, negative, or equivalent variation. For example, the dynamic state related to the act of frowning resulted in an aesthetically negative variation, while the dynamic state related to the act of raising the eyebrow also resulted in an aesthetically negative variation, whereas the dynamic state of smiling resulted in an aesthetically equivalent variation.

The acquisition of information regarding the variation as aesthetically positive, negative, or equivalent may consider the number of quadrants relative to the user's face. For example, if a greater number of quadrants are observed to have resulted in a positive variation (the quadrant's index (value) increased from the static state to the dynamic state), the data associated with the aesthetically positive variation will then be reported, that is, indicating that there is no treatment priority.

On the other hand, if a greater number of quadrants are observed to have resulted in a negative variation (quadrant index (value) decreased from the static state to the dynamic state), the data associated with the aesthetically negative variation will then be reported, thus indicating that there is a priority need for aesthetic treatment.

Similarly, if it has been observed that the number of quadrants whose index increased and decreased was the same, an aesthetically equivalent variation will be reported, thus indicating that there is no treatment priority.

It is further proposed that the same procedure performed above should also be carried out for the perioral region of hierarchy H3, that is, the region near the user's lips.

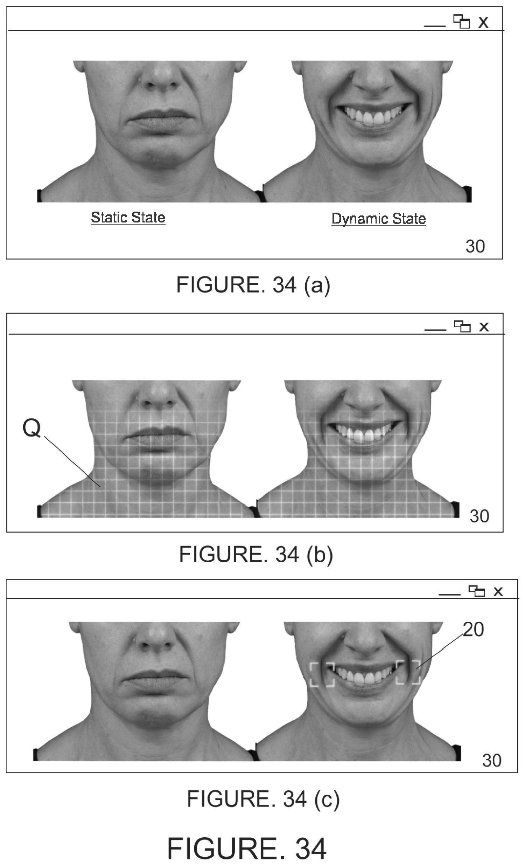

Thus, with reference to FIG. 34, the comparison of the user's portion for the static state and for the dynamic state of the perioral region is presented, in which it is proposed that the dynamic state be understood as, for example, the act of the user smiling (or any other expression), as illustrated in Fig. 34 (a). Therefore, and with reference to Figs. 34 (b) and (c), the segmentation of the user's face into the plurality of quadrants Q is shown, as well as the indication of the aesthetic marker, as previously mentioned.

In one example, the comparison between the static state and the dynamic state considering the act of the user smiling could result in an aesthetically equivalent variation.

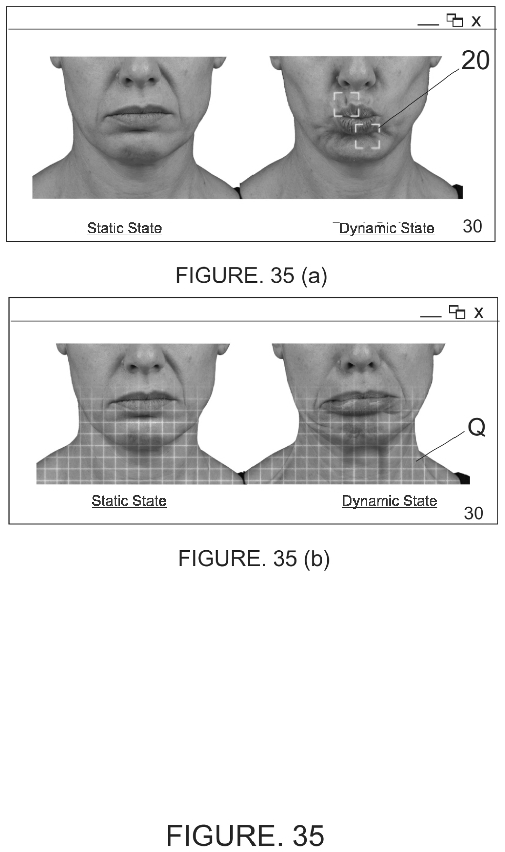

Similarly, it is proposed that other dynamic user variations be considered for the perioral region, such as the act of the user moving the mouth and lips to simulate a kiss (Fig. 35 (a)) as well as moving the mouth and lips to display a pout (*pouting*), as illustrated in Fig. 35 (b).

Thus, by way of example, the dynamic state of simulating a kiss could result in an aesthetically equivalent variation, whereas the dynamic state of displaying the lower lip could result in an aesthetically negative variation, thus indicating the need to consider that region (for example, indicated by a specific quadrant or groups of quadrants) in the treatment plan.

It is proposed that the comparison between the static state and the dynamic state be carried out using photos of the user, which may, for example, be uploaded by the user to the computing platform or directly captured by the computing platform, for example, using a camera. The analysis of expressions through video would also be possible, with this analysis being considered more sophisticated and complex; therefore, the analysis of graphical data by photo becomes faster and more feasible.

So, the computing platform may prompt the user to capture a specific photo, for example, by displaying a message to the user to take a photo while moving their mouth to simulate a kiss, a pout, or even by moving their forehead and eyebrow.

Furthermore, the previously mentioned static index could, for example, be initially assigned by the computing platform and related to a specific ethnicity, age group, or gender of the user.

Subsequently, after the evaluation for hierarchy H3 has been performed, the next hierarchy of the face can now be evaluated, this one referred to as H4.

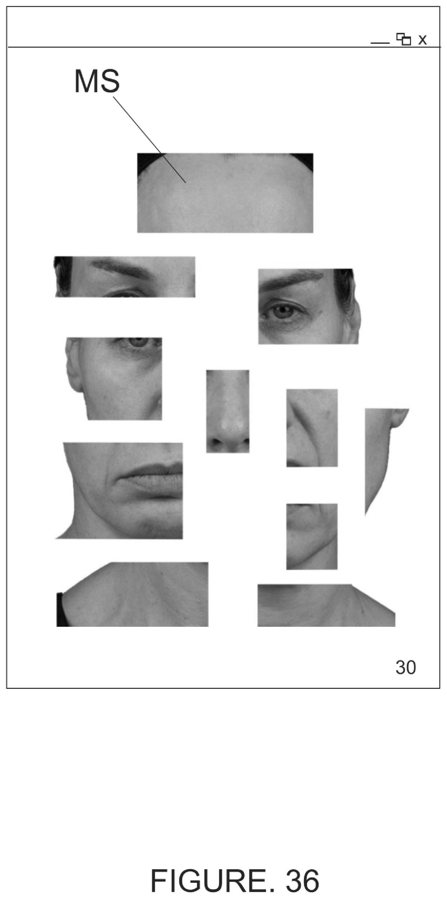

The H4 hierarchy has already been presented, for example, in the corresponding patent US 11,602,303 and refers to the segmentation of the face into a plurality of *subunits*. It is thus understood that the step of scanning at least a portion of the user further comprises the step of segmenting the portion of the user into a plurality of micro segments MS, as illustrated in FIG. 36.

Thus, at this stage, at least a portion of the user (such as one of the segments of the plurality of micro segments MS) must be compared with a portion considered 'ideal'.

The portion considered ideal can be understood as a *benchmark* for comparison, so that said portion considered ideal can be suggested by the computing platform itself, taking into account the user's ethnicity, age group, and gender identity, or it may also be provided by the user of the computing platform.

Thus, if provided by the computing platform, said platform should, for example, suggest 10 models considered ideal, such that in this way the user can select the model that pleases them the most. In one embodiment, each of the models to be suggested to the user may also contain a respective level of difficulty, that is, the existing difficulty for the user, starting from the current aesthetic state, to reach the aesthetic state of the model suggested by the computing platform.

In one embodiment, such ideal models (portions considered ideal) may be understood as people, such as celebrities, to be suggested to the user. Thus, the user has the possibility to choose the ideal model linked to a specific person, thereby indicating that the user wishes, after the treatment plan is carried out, to become aesthetically similar to this chosen person.

In equally valid embodiments, the ideal models to be suggested to the user may be suggested based on age models, that is, the user may choose a model that refers to an "ideal 35-year-old model," said model then comprising aesthetic data considered ideal for a 35-year-old person. Furthermore, the ideal models may also be suggested based on a given ethnicity, for example, thus suggesting an "ideal model for a Caucasian person," or even an "ideal model for a 45-year-old Caucasian person."

In an equally valid embodiment, the user may be prompted, through the computing platform, to freely indicate which ideal model the user wishes to select.

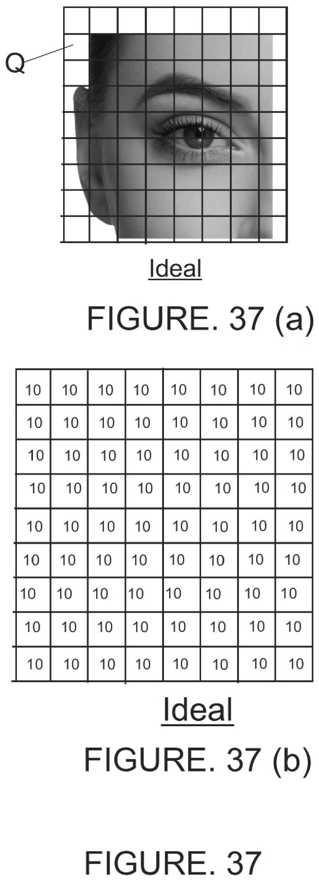

Thus, it is understood that a given model considered ideal will comprise a plurality of ideal aesthetic data, such as a plurality of ideal aesthetic data for each of the quadrants (or set of quadrants) of the user's face, as indicated in FIG. 37. Thus, each of the aesthetic data considered ideal for each of the quadrants may comprise a certain ideal index, such as an ideal value (for example, a scale from 0 to 10), so that said scale will be used as a basis for comparison with the user's actual data, thereby indicating which and how many aesthetic interventions that user needs to undergo in order to get as close as possible to the model considered ideal, or even indicate the impossibility of achieving that goal desired by the user.

It is thus understood that the user portion (such as a specific micro segment MS of the user's face obtained from a plurality of segments) comprises a plurality of actual aesthetic data, for example, each of the segments (quadrants) of the user's face may comprise a specific actual aesthetic data. By way of example, the actual aesthetic data may be understood as indices (such as a numbering from 0 to 10) for each of the user's facial quadrants, so that such indices (actual aesthetic data) will then be compared with the respective ideal aesthetic data, thus indicating whether there is a need for aesthetic treatment in that region.

In one embodiment, the aesthetic data considered ideal (and consequently the ideal model) may be previously stored in a memory of the computing platform; alternatively, or additionally, the aesthetic data considered ideal may be obtained from clinical trials, more specifically, from data provided in clinical trials as well as from data found in literature. The index values for each of the ideal aesthetic data can then be generated and subsequently entered into the computing platform.

Additionally, or alternatively, the ideal model may be generated by the computing platform itself, for example, through artificial intelligence, in which the computing platform may be prompted, for example, to design the face of a model considered ideal and referring to a person 50 years of age. Furthermore, artificial intelligence can be prompted to design an ideal model of the user themself, for example, based on a younger photo of the user.

So when evaluating the user's photo, the artificial intelligence will be able to redesign the photo considering an aesthetically appropriate expression of the user, for example, without the effects of aging, stress, and poor nutrition.

Moreover, as previously mentioned, the model considered ideal (and consequently the ideal aesthetic data) may be previously stored in the computing platform and obtained from a plurality of models considered ideal.

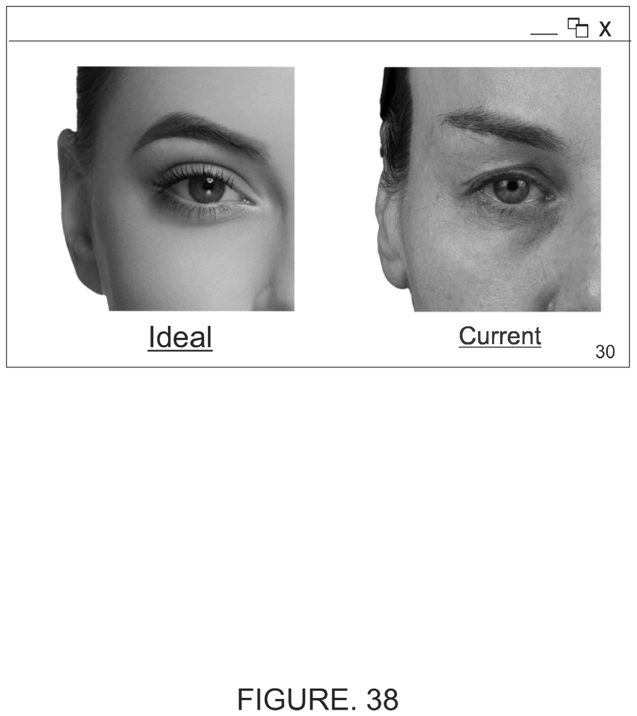

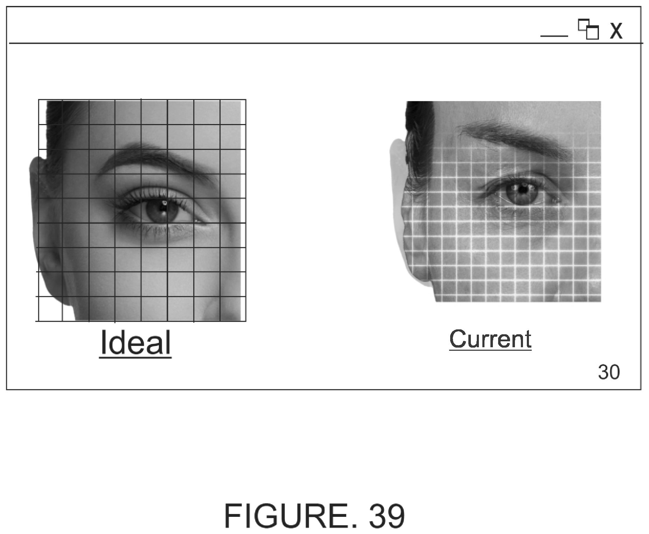

In this way, and with reference to FIGS. 38 and 39, each of the micro segments MS must be compared with its respective micro segment MS considered ideal. To perform such a comparison, and as already described, the micro segment MS can be divided into a plurality of quadrants Q, thus assigning to each of the quadrants a respective index (such as a numbering from 0 to 10). Therefore, as previously described and with reference to hierarchy H3, the indices considered ideal are compared to their respective actual indices.

Subsequently, it is proposed that the computing platform returns which aesthetic interventions that particular user should undergo in order to become aesthetically close (or similar) to the model considered ideal for that specific segment.

Thus, and with reference at least to FIGS. 38 and 39, each of the values (indices) of the quadrants (or set of quadrants) considered ideal is compared with the respective actual quadrants. Thus, by way of example, if there is a difference between the ideal index and the actual index, this fact will indicate that the specific region comprising the referred quadrant should be targeted for an aesthetic treatment.

Accordingly, by performing the commented evaluation, the computing platform may, for example, return unaesthetic parameters that should be treated so that the actual user becomes aesthetically similar (or close) to the model considered as ideal.

In one embodiment, such unaesthetic parameters may, for example, be identified as: excessively high eyebrow, excess skin on the upper eyelid, small eye shape, low lateral canthus, prominent tear trough, loss of cheek volume.

It is thus proposed that the methodology discussed above be carried out for each of the micro segments MS of the plurality of segments illustrated in FIG. 38. Furthermore, the comparison with a model considered ideal can be performed either in a static state or in a dynamic state.

Based on the comparison between the model considered ideal and the user's current model, as well as the scanning of the user's face, the computing platform can thus return monitoring portions of the user, consideration portions, and treatment portions.

Thus, measures to be taken for that user are defined, based on the comparison with their model considered ideal.

Thus, monitoring portions can be understood as portions (areas, regions, quadrants) of the user that deserve to be monitored over time, that is, they may indicate portions that, at present, do not require an aesthetic treatment, but that eventually, after a period of time (monitoring period), may require an aesthetic intervention. Thus, such portions can be stored in the database of the computing platform linked to that specific user, so that monitoring is automatically performed by the computing platform.

In one embodiment, it is proposed that the user will receive a notification, such as an audible, textual, or graphical signal, as well as a combination thereof, when the time period, referred to as the final monitoring period, is approaching or has already expired.

Portions for consideration can be understood as portions that are slightly distant from those considered ideal, that is, they are portions that are currently in an aesthetic state deemed acceptable. Thus, the computing platform may suggest that the user consider such portions in the treatment plan to be generated, for beautification or preventive purposes.

Finally, treatment portions should be understood as those portions (regions, areas, segments, quadrants, groups of quadrants) that are excessively far from the respective portion considered ideal, thus indicating that there is a need for said portion to be treated so that the user can achieve an appearance close to (or similar to) the appearance of the model considered ideal according to age group, ethnicity, and gender identity.

In one embodiment, the evaluation that a portion will be defined as a monitoring, consideration, or treatment portion may be made based on an aesthetic variation index Δₑₛₜ, such that said aesthetic variation index can be understood as the difference between the ideal aesthetic index and the actual aesthetic index, that is:$Δ est = \left|{lest}_{ideal}-\left.{lest}_{real}\right|\right.$

Thus, the aesthetic variation index Δₑₛₜ indicates how far the actual aesthetic index (Iestᵣₑₐₗ) is from the ideal aesthetic index (Iest_{ideal}). Thus, it is possible to determine magnitude ranges, thereby indicating, for example, three magnitude ranges that correspond to the monitoring, consideration, and treatment portions, respectively.

In one embodiment, the ideal aesthetic index may be assigned a total value of 10; thus, for each of the quadrants of the segment under analysis, a value of 10 may be assigned. Reference is made, for example, to Fig. 37 (b).

Thus, values for the aesthetic variation index Δₑₛₜ that are in the range from 0 to 1 should return, for example, that the given portion falls within a monitoring portion, thus indicating that the actual aesthetic index has assumed the value equal to 10 or equal to 9.

Values for the aesthetic variation index Δₑₛₜ that fall within the range of 2 to 4 should, for example, indicate that the given portion fits into a consideration portion, thus indicating that the actual aesthetic index assumed values of 8, 7, or 6.

Values for the aesthetic variation index Δₑₛₜ that fall within the range of 5 to 10 indicate that the portion under consideration (current/actual portion) is excessively far from the portion considered ideal, so that the referred portion should then be classified as a treatment portion, thus indicating that the actual index assumes values of 5, 4, 3, 2, 1, or 0.

The value ranges described herein should be considered for illustrative purposes only. Additionally, the ideal aesthetic index does not necessarily have to assume the total value of 10, so any value can be assigned to the index in question (for this reason, it is suggested that the expression I be considered in modulus). In any case, it is suggested that the values for the ideal and actual aesthetic index should be positive values.

In summary, the further the actual aesthetic index is from the ideal aesthetic index, the greater the indication of the need for aesthetic treatment in the respective portion of the user.

In one embodiment, signaling to the user which portions should be understood as monitoring portions, consideration portions, and treatment portions can be performed by indicating the quadrants of the user's face that originated the respective signaling.

Thus, for example, the quadrants in question can be superimposed on the user's face, delimiting, for example, in green, the quadrants that indicate the monitoring portions, in yellow, the quadrants that indicate portions for consideration, and in red, the quadrants that indicate the treatment portions.

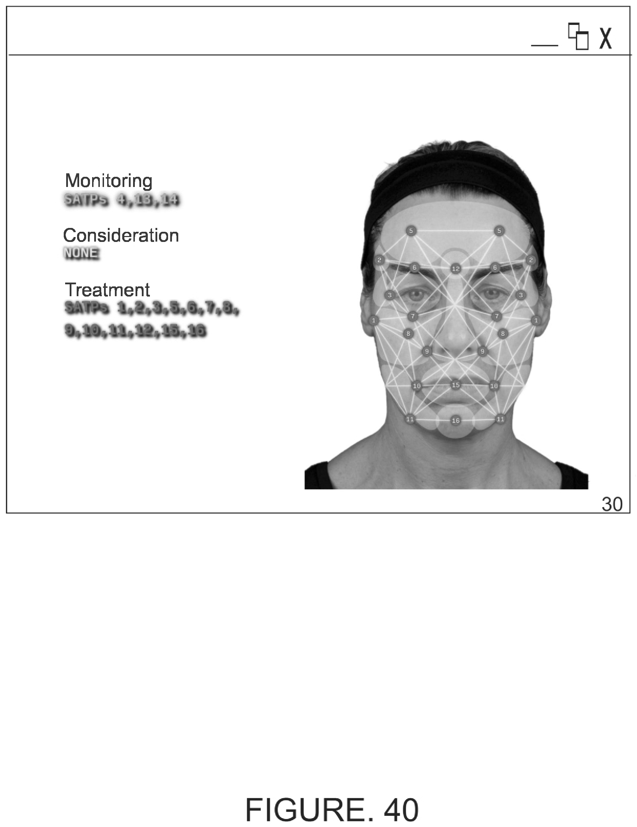

Additionally, or alternatively, the indication to the user regarding the monitoring, consideration, and treatment portions may be carried out as illustrated in FIG. 40, thus being carried out from the so-called ATP *Aging Trigger Points*, these points already discussed, for example, in patent US 11,602,303.

Thus, in an embodiment as illustrated in FIG. 40, the ATPs related to the treatment portions could be highlighted for the user in red. Otherwise, it may optionally be chosen not to display to the user the ATPs related to the monitoring and consideration portions, or, alternatively, the monitoring portions could be indicated in green and the consideration portions could be indicated in yellow.

Thus, once the portions that will be targeted for an aesthetic treatment are defined, the aesthetic indicator is generated, more specifically, the indication of the aesthetic indicator in a treatment state is provided.

In this sense, the aesthetic indicator can be understood as data (information) generated by the computing platform, indicating that an aesthetic treatment should be performed for that specific portion of the user. The aesthetic indicator can be generated from any user face scanning embodiment described in the present invention.

Thus, having defined the portions that will be subject to an aesthetic treatment, that is, having defined the treatment portions, the methodology described in the present invention further proposes the step of obtaining and generating at least one treatment code from the aesthetic indicator, so that, by aesthetic indicator, it should be understood as any data that refers to the computing platform the need to perform an aesthetic treatment.

Treatment code should be understood as a code that indicates to the physician or qualified healthcare professional at least the location that should receive the aesthetic treatment. Furthermore, said treatment code may also indicate to the healthcare professional not only the treatment site, but also provide instructions linked to the application of a specific substance, so that said instructions may be related to the application dose, the application instrument, the method of distributing the product, the application depth, the application angle, among others.

In a valid embodiment, such application codes may be understood as the MD Codes and/or the MDDyna codes addressed in patent US 11,602,303. In any case, it should be noted that, considering the teachings of the present invention, such treatment codes should be understood as any treatment code (that is, a textual, numerical, graphical representation, as well as a combination thereof) that indicates to the healthcare professional at least the site of application of a substance.

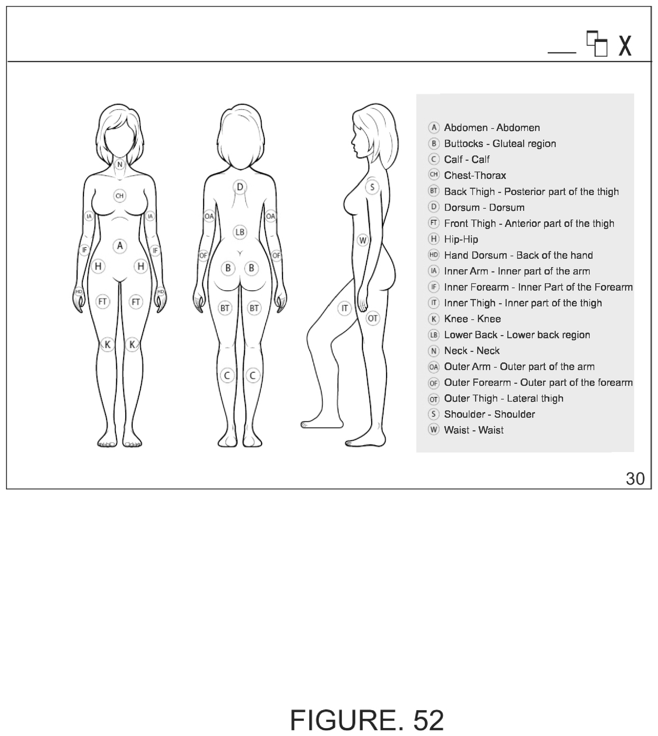

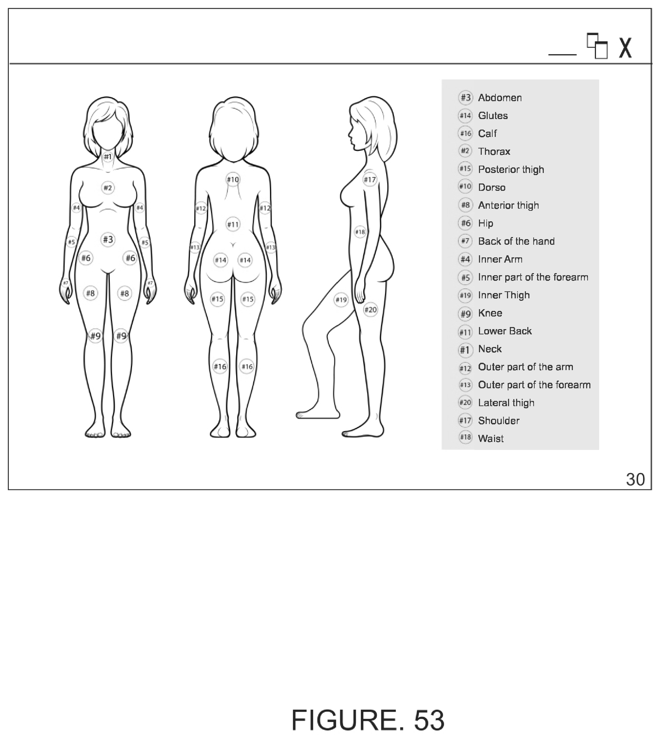

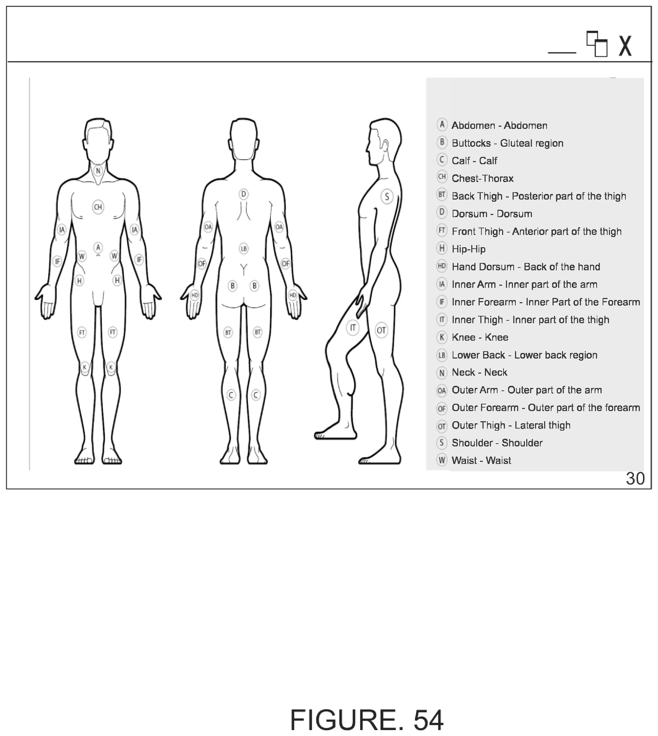

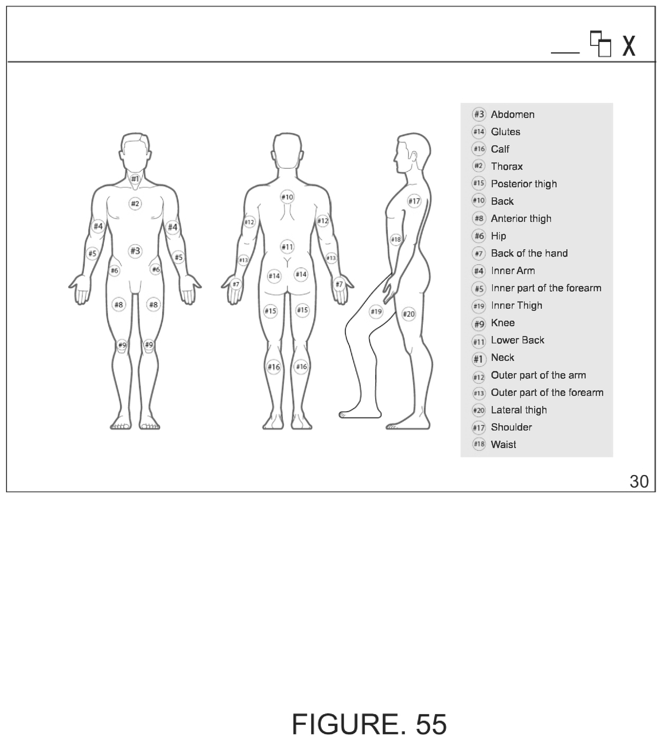

In an exemplary embodiment, such treatment codes TC may refer to treatment codes that indicate the application site on a user's face. In an equally valid embodiment, such treatment codes TC may refer to body treatment codes, such as the indication of FIGS. 52 to 55.

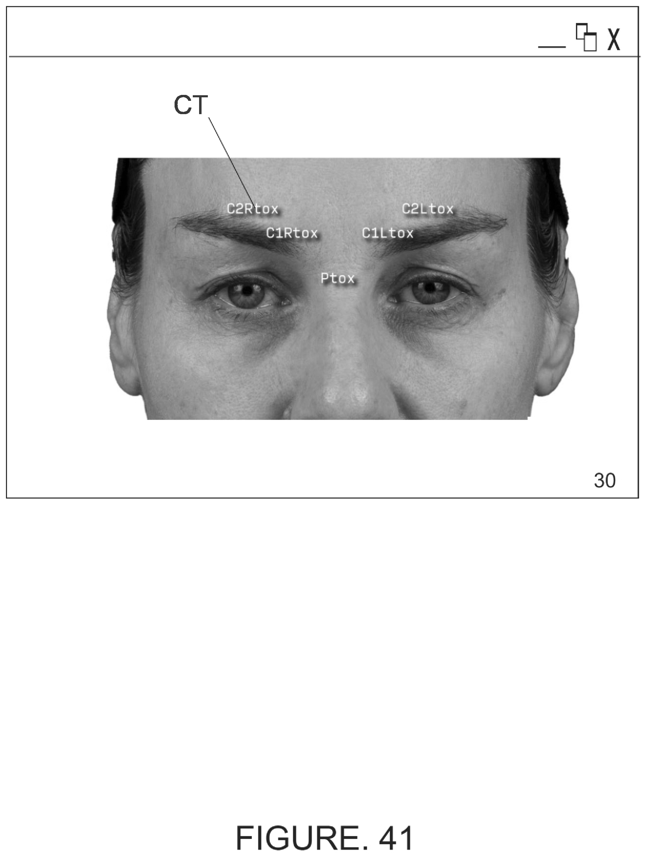

Thus, the present invention further comprises the step of displaying the treatment codes TC on a portion of the user, as exemplarily illustrated in FIG. 41. It should be noted that such treatment codes TC should also be displayed on the screen of the computing platform.

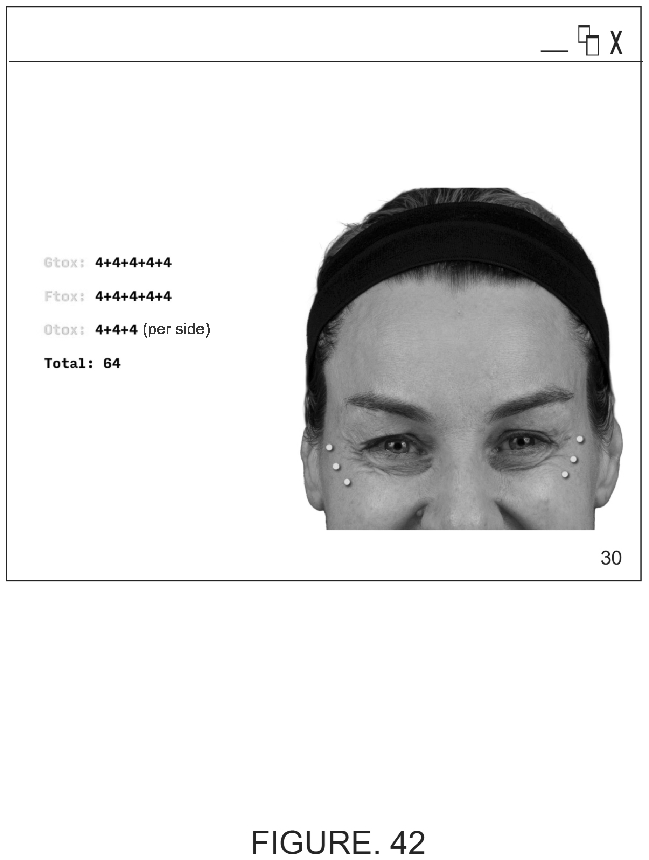

Additionally, the display step of at least one application dose related to the treatment codes TC is proposed. Accordingly, for each treatment code obtained, a respective treatment dose must be displayed on the computing platform, as exemplified in FIG. 42. Therefore, a total number of doses to be administered and related to the user's treatment plan must also be displayed.

In a valid embodiment of the present invention, and based on the generated treatment codes TC, the step of correlating the generated treatment codes TC with the final set of previously obtained and already discussed aesthetic, emotional, and social data is also proposed.

Thus, the computing platform evaluates and informs the user whether the obtained treatment codes TC correspond to the regions and areas previously indicated by the user as desirable treatment regions and areas.

It should be noted that for the generation of treatment codes TC, it is suggested that priority be given to the action of scanning the user's face and/or body and thus detecting areas, regions, and quadrants that require priority aesthetic intervention.

Having identified and generated the treatment codes TC with their respective application doses, as well as having displayed them through the computing platform, the present invention further proposes the step of generating at least one application seal linked to the user.

The application seal S1, S2, S3, S4...SN can be understood as data (information) suitable to be read by a computer and which comprises a plurality of information related to the generated treatment plan stored therein. Thus, the application seal S1, S2, S3, S4...SN is understood as, for example, a file, which can be read, processed, stored, transmitted, and received through computer means. Furthermore, it is understood that the application seal S1, S2, S3, S4...SN is capable of storing a plurality of information that can later be read and processed by computer means. In a valid embodiment, the application seals S1, S2, S3, S4...SN could be read and processed through an augmented reality and/or virtual reality device, such as glasses equipped with functionalities linked to augmented reality and/or virtual reality.

Thus, the application seal S1, S2, S3, S4...SN is configured as a structure that directs (guides) application parameters of a given substance and considering a specific treatment code. In this way, the application seal could, for example, be sent from the computing platform to the personal computer of a physician or qualified healthcare professional, so that, upon receiving the aforementioned application seal S1, S2, S3, S4...SN, the physician will have all the necessary information to thus carry out the application of the substance.

Additionally, the application seal S1, S2, S3, S4...SN could, for example, be sent from the computing platform to an automated applicator of a certain substance, such as an applicator robot (mechanical arm). Thus, upon receiving the application seal S1, S2, S3, S4...SN, said automated applicator could read and process it, thereby automatically applying the substance to the user. It is thus understood that, upon receiving the application seal S1, S2, S3, S4...SN, the mechanical arm of the automated applicator will move, thus transitioning from a resting state (stationary) to a moving state.

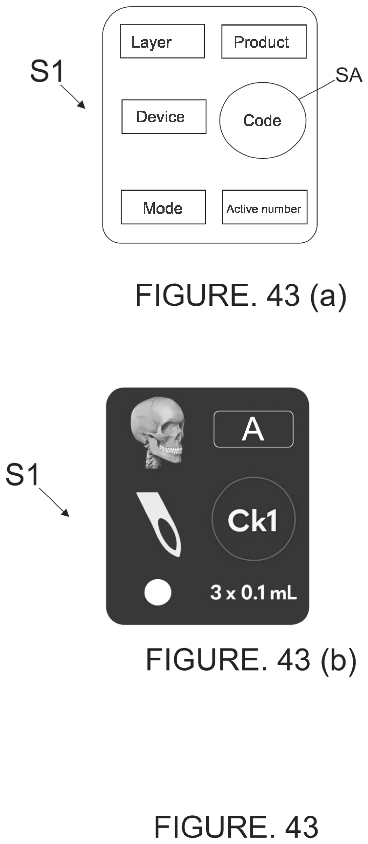

Considering the teachings of the present invention, and further referring to the illustration of FIG. 43, it is proposed that the application seal stores at least the following information (data): a treatment code TC data, an application product data, an application layer data, an application device data, an application mode data, an active number data (dose).

Moreover, and even if the application seal S1, S2, S3, S4...SN is to be understood as a file suitable for computer reading, it is proposed that said application seal also comprise a graphical arrangement of its information that enables correct reading and identification by the professional who will receive the aforementioned application seal.

Thus, the application seal is also understood as a way to graphically provide information (instructions) related to the application of the substance to a specific user.

Thus, and based on the description previously provided, it is understood that the application seal is configured as at least one of: a machine-readable application seal and a user-readable application seal.

FIGURE 43 illustrates a preferred graphical representation of the proposed structure for the application seal S1. Preferably, the application seal S1 comprises a structure in the form of a square, in which a set of three pieces of information are arranged in a first half of the application seal and an additional set of three pieces of information are arranged in the second half of the application seal. It is thus understood that the application seal is segmented into at least a first portion and a second portion, in which each portion comprises the same amount of data.

Therefore, and with reference to Figs. 43 (a) and (b), it is established that the application seal comprises a treatment code TC data, in this case, Ck1, thus indicating the application site to the medical professional or to the professional making use of the teachings of the present invention. Furthermore, it is noted that the application seal S1 indicates the application product, in this case, referenced as a product A.

The application seal S1 also indicates the application layer, such that said indication can be made through a graphic element, such as a figure. In this case, there is an image of a skull, thus indicating to the professional that the application should be performed at the bone plane (periosteum/supraperiosteal) of the patient.

Similarly, the application seal also indicates the application device through a graphic element, so that, with reference to the illustration in Fig. 43(b), the professional is informed that the application should be performed with a specific instrument, such as, for example, a needle.

Furthermore, the indication of the mode of application also occurs through a graphic element; in this case, there is the representation of a circle, thus allowing the professional to understand that the application should occur in aliquots.

Finally, the data regarding the active number (volume/dose/units) indicates to the professional the application dose, in this case consisting of three applications of 0.1 ml each.

It is noteworthy that the graphic elements and treatment codes that should make up the application seals may be those already known in the state of the art, for example, found in the literature and related to MD Codes and MD Dyna Codes.

Having generated the application seals S1, S2, S3, S4...SN, the present invention then proposes the step of generating the treatment plan from said application seals S1, S2, S3, S4...SN.

In one embodiment, the treatment plan could be generated directly by the computing platform and thus comprise at least data regarding the number of sessions, cost data of the generated treatment plan, and data regarding the person responsible for carrying out the treatment plan (whether a medical professional, a healthcare professional, or an automated applicator). In this sense, such data tends to be the most valuable information for the user who will receive the treatment plan, data that can be accepted or rejected by the user.

Furthermore, it should be noted that any information contained in the treatment plan should be understood as treatment data.

For example, if the treatment plan generated directly by the computing platform includes a cost data point informing the user that the said treatment will cost 10,000 monetary units, such cost may be accepted or rejected by the user. Likewise, if the treatment plan generated directly by the computing platform comprises a given number of sessions indicating that the treatment will be carried out in seven sessions, such fact may be accepted or rejected by the user.

Thus, if the cost data and the number of session data are accepted by the user, the treatment plan will be duly generated, based on the application seals S1, S2, S3, S4...SN. It is thus understood that, if there is no objection from the user regarding the treatment data, the previously generated treatment plan will be implemented.

Alternatively, if at least one of the session number data or the cost data is rejected by the user, a step of generating an updated treatment plan based on the user's requests is proposed. Thus, if there is a user objection regarding at least one of the treatment data, it is proposed to carry out the step of receiving an update data from the user and generating an updated treatment plan, as will be described below.

By way of example, if the treatment plan directly generated by the computing platform has indicated a treatment data, for example, a number of sessions equal to 5 sessions, and the user has, through the computing platform, rejected said data, it is then proposed to carry out the step of requesting the user to provide an update (update data) regarding the rejected data.

In this case, following the requested update, the user must indicate whether they wish to carry out the treatment plan in a greater or lesser number of sessions compared to the number provided; alternatively, the user may, through the computing platform, specify in how many sessions they would like to carry out the treatment plan.

Once the user update data has been received, the treatment plan update step is then proposed, such that the treatment plan will now include the session number data as provided by the user.

It should be noted that if for any reason the update data provided by the user is not acceptable, the computing platform may issue a warning message, thus indicating that the number of sessions provided by the user is not allowed, based on the information contained in the application seals S1, S2, S3, S4...SN.

By way of example, if the user expresses the desire to undergo the treatment in only one session, a warning message may be issued, thus informing the user of the minimum number of sessions required for that specific treatment.

It is thus understood that the present invention comprises the step of, after receiving the update data from the user, performing the step of verifying the feasibility of the received update data, and then generating an updated treatment plan (if the update data are considered feasible).

Similarly to the description provided above, the cost data may be accepted or rejected by the user.

For example, if the treatment data, such as the cost data directly generated by the computing platform and referring to the cost of 10,000 monetary units, is rejected by the user, the computing platform will prompt the user to provide updated data. Thus, the user may indicate that the reported cost is high and, then, the computing platform may generate an updated treatment plan with a reduced cost, for example, the cost reported by the user.

As previously described, it is also proposed to include a step for verifying the feasibility of the received update data. For example, if the update data (new cost) provided by the user is not feasible, the computing platform will issue a warning message, thus indicating to the user the impossibility of performing the treatment at the informed cost or further informing the minimum possible cost for carrying out that treatment.

By way of example only, the costs of a treatment can be reduced by replacing the type of product considered in the treatment and/or by replacing the applicator (whether a physician or a robot) who will perform the treatment and/or by changing the number of sessions.

In fully valid embodiments, upon receiving the cost data linked to the treatment plan, the user may indicate, through the update data, the possibility of bearing a higher cost than the one informed for carrying out the treatment.

For example, the cost data of 10,000 monetary units was generated considering the use of product A, which can be considered a medium-quality product in the market. Thus, upon receiving the treatment plan with the aforementioned product, the user may indicate the possibility of bearing the cost of 15,000 monetary units and then undergo treatment with product B, which is considered a premium product on the market.

Similarly, the cost data can be updated when the professional responsible for carrying out the treatment plan is changed. For example, the cost data of 10,000 units was generated for professional A, who is considered a professional with average experience. Thus, if the user wishes, they may request the update of the treatment plan so that it is generated/carried out by professional B, who is considered a highly experienced professional. Thus, as the treatment plan will be carried out by a more experienced professional, in theory the cost tends to be higher.

In an equally valid embodiment of the present invention, and before the treatment plan is generated, the step of motivating the user to provide, by way of example, a cost data and a data of the desired number of sessions is proposed.

Thus, it is up to the user to freely specify, for example, how many sessions the user wishes to undergo the treatment and, in this way, the computing platform will assess the feasibility of the number of sessions specified by the user. If the number of sessions provided is feasible, the treatment plan will be generated; otherwise (if the number of sessions is unfeasible), a warning message will be sent to the user, thus requesting the user to provide the updated data, such as a previously provided description.

Additionally, or alternatively, and before generating the treatment plan, the computing platform may prompt the user to provide a cost amount that the user is willing to bear for the treatment in question. Thus, upon receiving the aforementioned cost data, the computing platform will automatically generate a treatment plan that fits the provided cost data, comprising a certain number of sessions and the type of product to be applied.

When the treatment plan is generated, it is proposed that it include information on total costs, that is, for the entire treatment, as well as information on the cost per session.

Thus, if the generated treatment plan indicates the performance of four sessions, it is proposed that the cost of each of the four sessions be provided separately, so that the user is aware of the individual costs and can, for example, issue an update for each session or for the entire treatment. It is thus understood that according to the teachings of the present invention, the update data to be sent by the user may refer to the entire treatment, or may refer to each of the treatment sessions.

It is also advantageous for the treatment plan to include an estimated date for each session, so that the user can compare the scheduled date of the treatment plan with the cost of each session and thus assess whether the user will be able to afford the respective cost on that date. If the user wishes to propose any changes to the treatment plan, an update data may be sent by the user.

Thus, with the present invention, the user has the freedom, through interaction with the computing platform, to send update data for any information present in the treatment plan, so that in this way the treatment plan to be generated can meet the user's needs and desires. In this way, and by way of example, the user may, therefore, accommodate the investment amount they are comfortable spending.

Obviously, and as already described above, any update provided by the user must be validated by the computing platform, thus assessing the feasibility of carrying out the treatment plan based on the update provided by the user.

In this way, and as previously described, after presenting the objective diagnostic result (application seals), the system will ask the user in how many sessions the user wishes to achieve the proposed objective, which will impact the amount of material to be used and the financial investment. The user may also present their *budget* for each stage of the treatment (or for the entire treatment), and the system will provide the number of sessions and quantity of products that fit the user's *budget*. The system may also indicate if the *budget* is not sufficient to meet the user's requests or the desired outcome.

This being the case, the system is enabled to generate the treatment plan in an active or responsive manner, for example, by indicating a treatment plan directly to the user (active) or calculating the possible treatment options based, for example, on the *budget* sent by the user (responsive).

Thus, the generated treatment plan may be segmented by session, with each session comprising the respective treatment codes, the products to be used, as well as the corresponding application doses.

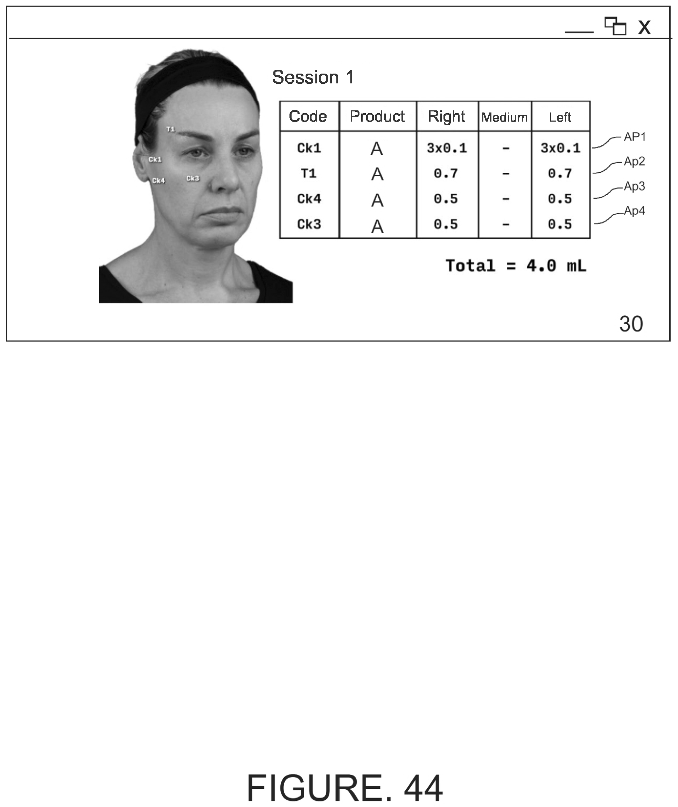

Thus, and with reference to FIG. 44, an example is given of a treatment plan for a first session comprising four application actions AP1 to AP4, in which there is a first application AP1 in the treatment code Ck1, with a product designated as product A, with the application of 3 aliquots of 0.1 ml on the right side and 3 aliquots of 0.1 ml on the left side.

Similarly, there is a second application AP2 in the treatment code T1, with the product A, with an application of 0.7 ml on the right side and 0.7 ml on the left side. Furthermore, and with reference to FIG. 44, there is a third application AP3 in the treatment code Ck4 (0.5 ml on each side) and a fourth application in the treatment code Ck3 (0.5 ml on each side).

It should be noted that the methodology and system proposed in the present invention may also, based on the generated treatment plan, indicate data regarding the number of syringes required to carry out the treatment plan, such that this data can be segmented, for example, by session, or may also be indicated for the entirety of the treatment.

Subsequently, the step of correlating the treatment plan with the application seals is proposed. More specifically, the step of generating and displaying the application seals linked to the treatment plan is proposed, thus enabling the correct application of the product, promoting reproducibility, and reducing adverse events due to the lack of specific guidance on best practices in the healthcare field. In one embodiment, the step of correlating the treatment plan to the application seals could be performed for each treatment session, or for each application action of the respective session, or even for the entirety of the treatment.

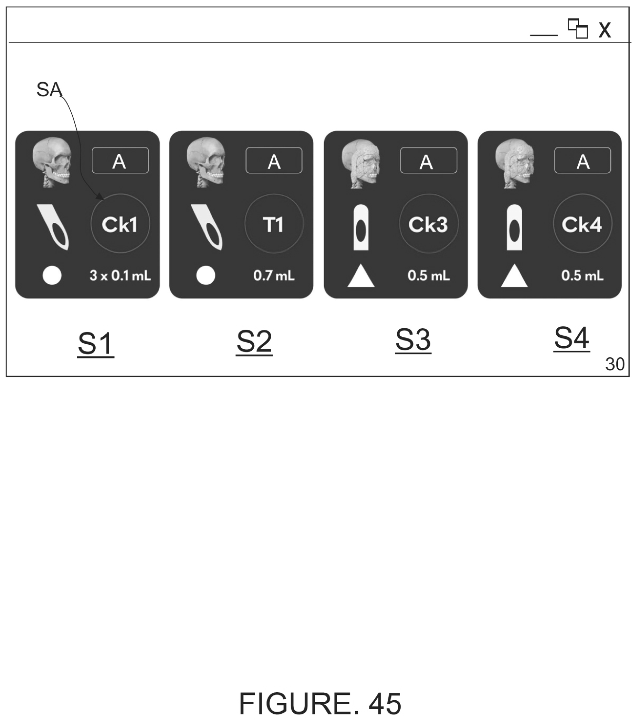

Thus, with the application seals, information is provided on how to inject (apply) the substance (product) so that the treatment plan is properly carried out. Thus, and with reference to FIG. 45, there is the step of obtaining the application seals linked to the treatment plan, more specifically, there is the step of obtaining the application seals for the first session of the treatment plan, that is, the application seals S1, S2, S3, and S4, which correspond to each of the four application actions AP1, AP2, AP3, and AP4.

With further reference to FIG. 45, it is noted that the first treatment session comprises four applications, thus, and as illustrated in FIG. 45, four treatment seals are generated and obtained, in which each treatment stamp refers to the application in a specific treatment code.

Thus, there is a first treatment seal S1 for code Ck1, a second treatment seal S2 for code T1, a third treatment seal S3 for code Ck3, and a fourth treatment seal S4 for code Ck4.

The application seals (such as seals S1 to S4) thus provide an indication of how the application should be performed, for example, taking as a reference the application stamp S1 of FIG. 45, there is an indication that the medical professional (or the automated applicator) should proceed to code Ck1, so that, as the aforementioned code is outlined by a blue circle, the professional (or automated applicator) will be aware that the referred code is a safety code. Subsequently, product A should be applied to the bone plane, and to reach the bone plane, a needle must be used. At the time of product application, it should be distributed in aliquots, with each aliquot comprising an application of 0.1 ml.

Subsequently, once the application related to the application seal S1 has been completed, the applicator may proceed to the next seal, that is, the application seal S2, thus performing the application on the treatment code T1 (second application action AP2).

In one embodiment of the present invention, it is proposed that the treatment code data arranged on an application seal be outlined by an warning light WL, as illustrated in the FIGS. 43 (a) and 45. The warning light WL can be understood as a circumference of a certain color, such as blue or red, which provides the applicator with an indication regarding the treatment code to be considered.

Thus, a warning light WL in blue indicates that the treatment code is a safety treatment code, while a warning light WL in red indicates that the treatment code is a warning code. The red halo (circle) codes denote proximity to critical structures such as arteries that require greater caution, for example, prior aspiration before product injection as well as specific training in areas considered advanced.

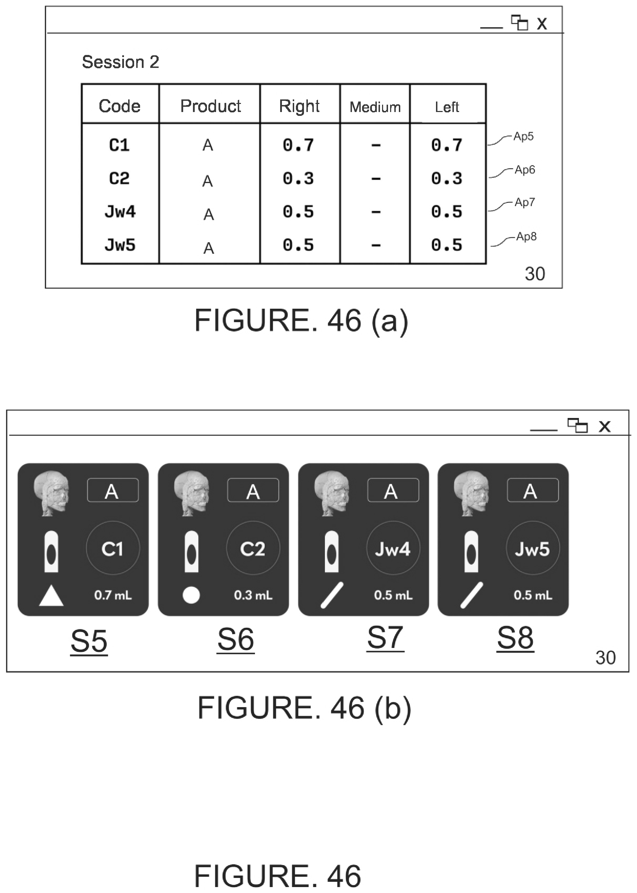

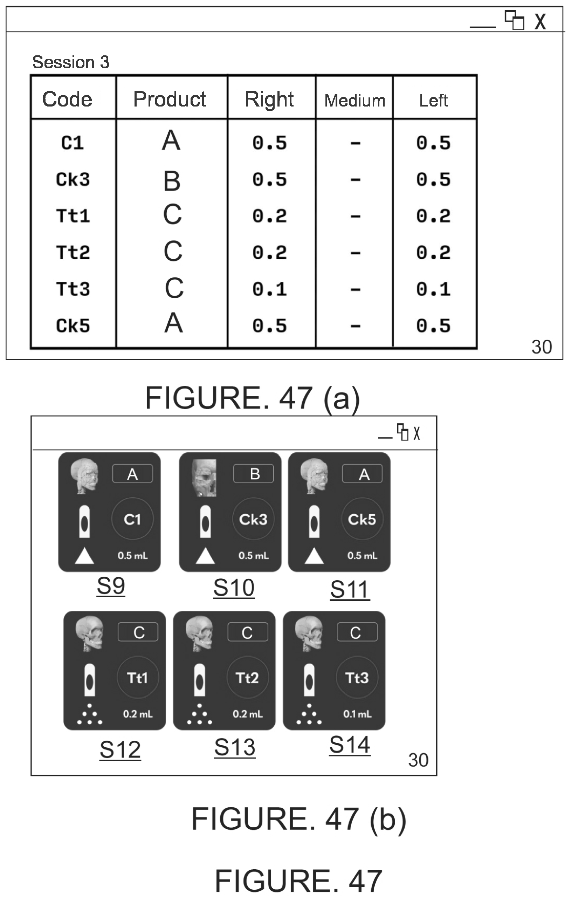

In this way, application seals are generated for each of the sessions in the treatment plan. Thus, FIGS. 46 to 48 illustrate the treatment plans and application seals for the second, third, and fourth sessions, respectively, in which each of these sessions comprises a plurality of application actions. It is thus understood that, for each application action, there will be a corresponding application seal.

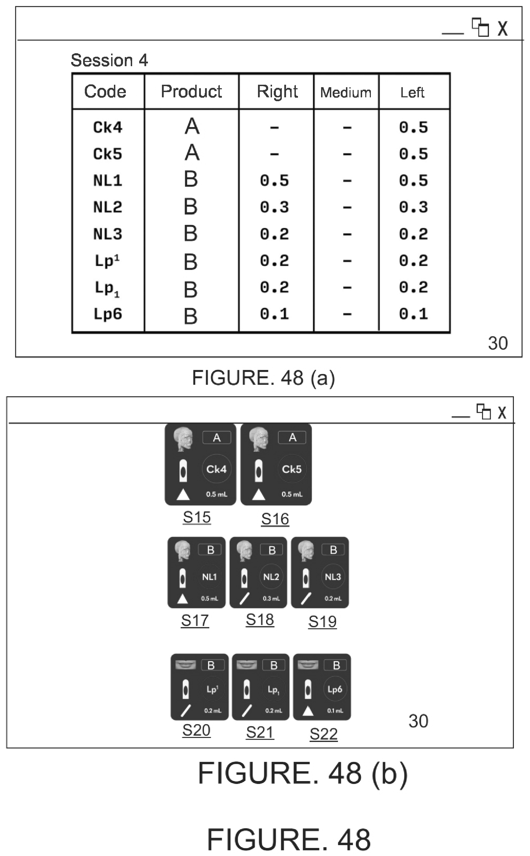

With specific reference to Fig. 48 (a), it is observed that for the treatment codes Ck4 and Ck5, no application was suggested on the right side of the user's face, precisely so that any asymmetry previously detected for the user during the face scan can be corrected. It is noteworthy that any information (received data, scanning) previously detected for a given user may be considered in the generated treatment plan.

Once the application seals have been generated for each of the sessions, it is then possible to generate an application chain AC corresponding to the generated treatment plan. The application chain AC can be understood as the grouping of a set of application seals linked to a specific user, in which the application chain further comprises the identifier code of the treatment CT as well as a date on which the treatment plan was generated.

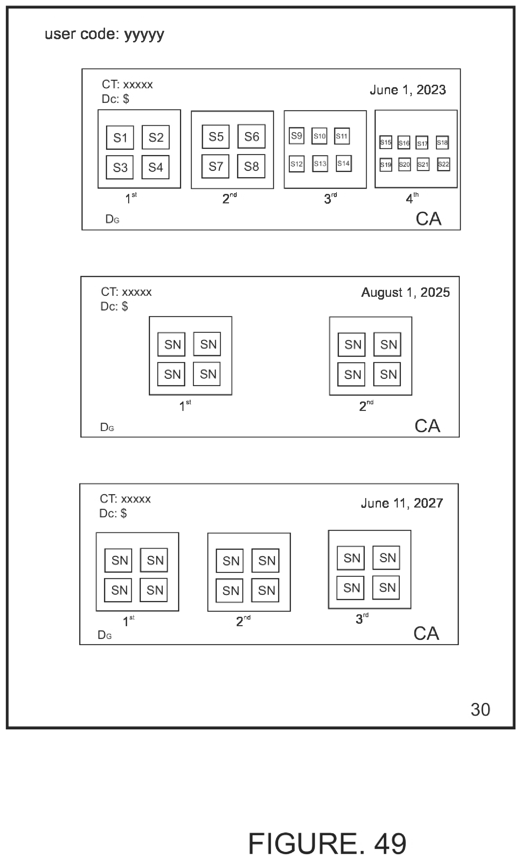

Thus, and with reference to FIG. 49, the application chain AC can be segmented by sessions (portions), in which the first session will comprise the set of application seals used in the first session of the treatment plan, that is, seals S1 to S4.

Thus, each portion of the application chain AC will comprise the set of application seals used in the treatment plan session; in this way, the application chain AC acts as a consolidated block referring to the treatment plan of that respective user. So, the application chain (as well as the application seals) can be sent to an applicator, such as a professional, medical professional, an automated applicator, or a professional in the field of aesthetic health.

Furthermore, once the treatment has been performed, the application chain AC can be stored on the computing platform; additionally, it can be stored in a digital wallet linked to the user. Thus, the application chain AC allows tracking the application history of that particular user, enabling that, when interacting (for example, clicking, tapping, processing) with a specific application seal stored in an application chain (or in a specific portion of the application chain), the user will have access to the information contained in that application seal, that is, the user will have knowledge of all information related to the treatment performed. The advantages resulting from the generation of the application chain are enhanced as a given user may have undergone dozens of treatments throughout their life.

Thus, having generated the application seals S1, S2...SN as well as the application chain AC, these can be sent to an automated applicator and/or to a professional applicator. Thus, it is understood that the present invention comprises at least one of the following steps: sending at least one of the application seal and the application chain to an automated applicator, and sending at least one of the application seal and the application chain to a professional applicator.

In other words, it is understood that the application seals as well as the application chain can be sent to a medical professional and/or to an aesthetic professional and/or to an automated applicator (robot), so that the treatment plan can be put into practice.

Consequently, a medical professional (healthcare professional and/or aesthetic professional) who receives a given application chain may interpret it, thus reading each of the application session seals and thereby carrying out the application of the treatment plan on the user.

Similarly, an automated applicator that receives a given application sequence can process it, thus interpreting the information contained therein and transmitting it to an automated mechanical arm responsible for carrying out the treatment plan application.

As an additional feature of the teachings of the present invention, it is proposed that the computing platform 30 itself is capable of suggesting to the user a plurality of applicators (whether professional or automated) that would be able to carry out the treatment plan provided to the user.

Thus, based on a certain criterion (pre-determined criterion), such as, for example, the user's location (or their native language), the computing platform 30 itself may suggest a list of applicators qualified to carry out the treatment plan and who are located near the user. It is further proposed that the user should have the freedom to stipulate the maximum desired distance of the applicators to be suggested.

Additionally, or alternatively, the computing platform is also able to suggest to the user only applicators who are qualified (certified) in a given procedure. For example, the computing platform 30 may be configured to indicate to the user clinics that have certified professionals, for example, in the well-known MD Codes. Thus, this functionality of the computing platform acts as a *"store locator"* for the user, facilitating the indication to the user of an applicator qualified to carry out the generated treatment plan.

Additionally, or alternatively, the teachings of the present invention are also capable of suggesting to the user a plurality of applicators, so that said suggestion is made based on the difficulty index linked to the generated treatment plan.

Furthermore, it is understood that the suggestion provided to the user encompasses at least one of the following: a suggestion of professional applicators, a suggestion of automated applicators, and a suggestion of clinics that would be qualified to carry out the generated treatment plan.

In one embodiment, and regardless of the mode of application (human or automated applicator), the application of the application chain (that is, the performance of the treatment) can be recorded, for example, through a camera.

Thus, once the treatment has been performed, it is proposed that the application chain AC be updated with at least one recording data _{R}D (video data), this recording data _{R}D comprising the recording of the generated treatment plan. In one embodiment, the application chain AC could comprise a plurality of recording data _{R}D, in which each recording data _{R}D corresponds to a specific application seal; additionally, it is fully acceptable that the recording data _{R}D be grouped, for example, for each session of the treatment plan or even for each application action AP1, AP2...APN of a given session. Thus, and with reference to FIG. 44, each of the four application actions of session 1 could comprise a recording data _{R}D. Furthermore, it is noteworthy that the collection, storage, and sharing of recording data may be carried out with the user's prior authorization.

It should be noted that application action is understood as each application existing in a treatment plan, such as each application existing in a given session of the treatment plan. Thus, and with reference to FIG. 44, the indicated session comprises four application actions, these referenced as: first application action AP1, second application action AP2, third application action AP3, and fourth application action AP4.

Similarly, and now referring to Fig. 46 (a), there is a fifth AP5, sixth AP6, seventh AP7, and eighth AP8 application actions.

Thus, by grouping the recording data _{R}D for each application action of a given session, it is possible, for example, to group the recording data _{R}D according to a specific product that was considered in a treatment plan. For example, the manufacturer of product B considered in the treatment plan shown in Fig. 48 (a) may view, through the application action-segmented recording data _{R}D, whether their product was properly applied to the user.

Furthermore, the application chain AC must also be updated with a code of the professional responsible for performing the treatment, whether this is a human or a machine.

Thus, the application chain AC allows the manager of the computing platform to generate a plurality of reports, segmented, for example, by the code of the professional who performed a given treatment, by the code of the user who received the treatment, by the type of product applied, by the code of the treatment considered, among others.

It should be noted that any information present on an application seal and/or present in an application chain AC and/or present on the computing platform and/or present in a treatment plan could be a parameter for generating a specific report.

Furthermore, it is proposed that the generation of the application chain AC is also accompanied by a cost data CT; thus, the present invention also comprises the step of generating a cost data CT from at least the application chain.

As previously described, the CT cost data can be understood as the cost related to the treatment plan performed; thus, upon receiving the treatment plan, the user will also receive a cost for carrying out the treatment in question, considering the chosen applicator. As previously described, the CT cost data can be provided for the entire treatment, or it can also be segmented by session, by application stamp, or by application action.

As an additional feature of the present invention, it is further proposed that each application chain AC also comprises at least one satisfaction index related to the treatment performed. By way of example, said satisfaction index is understood as an input from the user themself, thus indicating their satisfaction with the treatment performed as well as their satisfaction throughout the entire interactive process with the computing platform.

Moreover, the teachings of the present invention are also beneficial since the computing platform can be used as a platform for training new applicators. For example, based on the recording data _{R}D, a manager of the computing platform (such as a company manufacturing aesthetic substances or even an aesthetic clinic) can view whether a particular applicator (either human or robot) performed the treatment plan as suggested in the application seals and in the application chain.

Thus, by way of example, a manager of the computing platform (or any user/entity responsible for conducting training) may monitor the recording data _{R}D and provide, in real time, at least one compliance parameter, thereby indicating whether or not the performed application is compliant.

In one embodiment, said compliance parameter may be linked to each of the application seals S1, S2, S3, S4...SN that make up a given application chain AC. Thus, for each application seal, there will be associated with it a compliance parameter, that is, a parameter that indicates whether the application of the substance according to the instructions of the application seal was carried out properly or not. It is understood that the compliance parameter may be displayed on a screen of the computing platform and/or on a screen of an augmented reality device 1200.

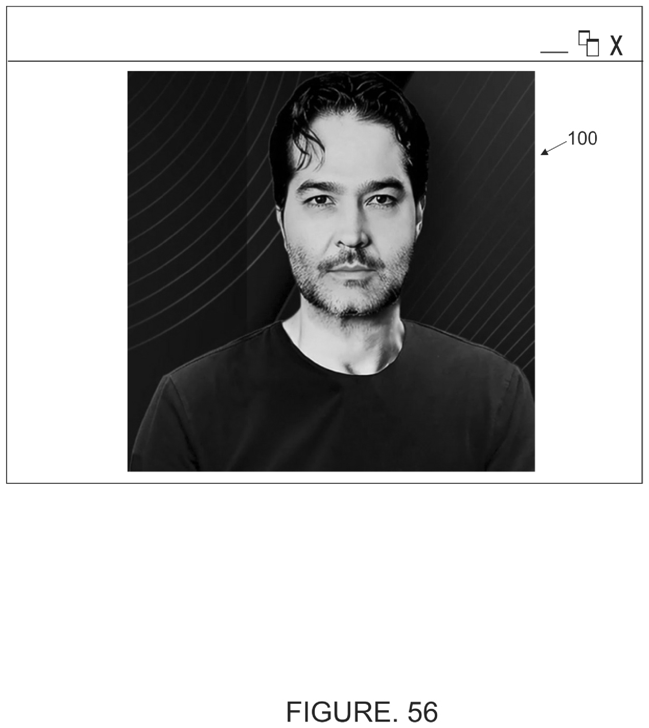

Thus, the teachings of the present invention can be incorporated into a computing platform aimed at training new healthcare professionals, thereby functioning as an academy or aesthetic university, in which, through the interaction of the user with a given professional, such as the interaction of the user with a specific graphical representation 100, 200, 300, 400, 500, 600, 700, 800, 900, 1000, and 1100 generated by artificial intelligence (as illustrated in FIGS. 56 and 57), a specific treatment plan can be generated and consequently the user can receive appropriate training. In addition, there will be support from multidisciplinary professionals represented by "avatars" to clarify, educate, and plan in various areas of activity relevant to a clinic's operations, including but not limited to the areas of healthcare, administration, management, marketing (digital media and influencers, among others), legal, commercial, and accounting, as illustrated in FIG. 57. These avatars are designated with names that contain the letters AI or IA in reference to artificial intelligence. By way of example, said academy, or aesthetic university, could be commercially referred to as AKADEMIAE. In a valid embodiment, said avatars can be configured to provide the platform user (such as the user undergoing substance application training) with the compliance parameters linked to each of the application seals.

It is further understood that the compliance parameters can be configured at least as a text data, a numerical data, an audio data, a video data, a data represented in an augmented reality environment, as well as a combination thereof.

By way of example, the compliance parameter can be set as a text data, thus indicating to the user, through a text, that the application of the application seal was carried out properly. More specifically, said parameter can be represented in the form of the following message: "incorrect application" or "correct application".

More specifically, it is proposed that, for each of the data that make up a given application seal, a corresponding compliance parameter be generated. Thus, it is proposed to generate a compliance parameter linked to the treatment code, the application product, the application layer, the application device, the application mode, and the active number (dose). In this way, it is possible to specifically determine the point of compliance (or non-compliance) during application of the substance. For example, if there is a nonconformity related to the application product, it becomes possible to identify that the substance was applied using a product not specified on the application seal. It is thus understood that the present invention proposes the step of generating a compliance parameter for each application seal, and/or generating a compliance parameter for each of the data that make up the application seal.

In a valid embodiment, a user's interaction with the computing platform (such as a user receiving training) could be carried out in the form of a survey, this survey to be displayed on the screen of the computing platform. In an equally valid manner, said interaction could be conducted by a graphical representation generated by artificial intelligence, as already described above.

The aforementioned survey presents the trainee user with a series of questions related to the application of a specific substance, so that the user is tasked with answering them through interaction with the computing platform. In one embodiment, after the user has provided the answer, the graphical representation generated by artificial intelligence may indicate, through an audio cue (voice), whether or not the answer is correct, thus generating a compliance parameter.

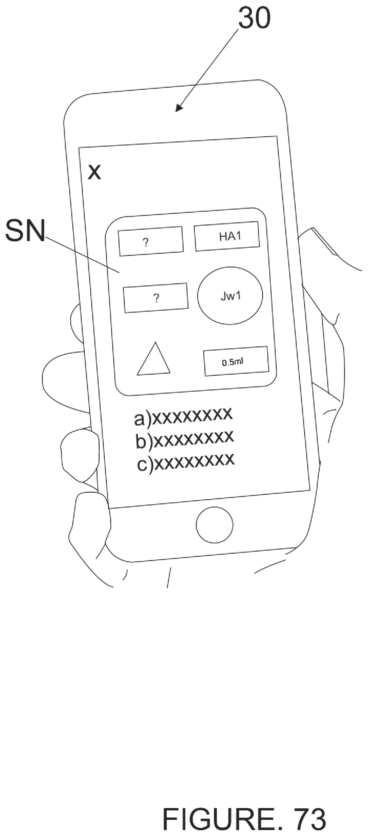

It is further proposed that the survey be correlated with the application seals. In this sense, and as illustrated in figure 73, the interaction with the user could occur through the display of a specific SN application seal on the screen of the computing platform as well as through questioning the user so that they indicate "how the application seal referring to treatment code Jw1 can be injected." In one embodiment, said inquiry could occur by voice, through the graphical representation generated by artificial intelligence.

Furthermore, the arrangement of the application seal on the screen of the computing platform 30 could, at first, omit some data from the application seal, such as the data related to the application layer and the application device, as illustrated in figure 73 (since this data is related to the question asked). At a later stage, after the user's response has been submitted, the application seal could be displayed to the user in its entirety.

In an equally valid embodiment, the aforementioned graphical representations generated by artificial intelligence could be used not only in the interaction with a user who wishes to receive a treatment plan, but also in the interaction with a user of the computing platform who is receiving specific training.

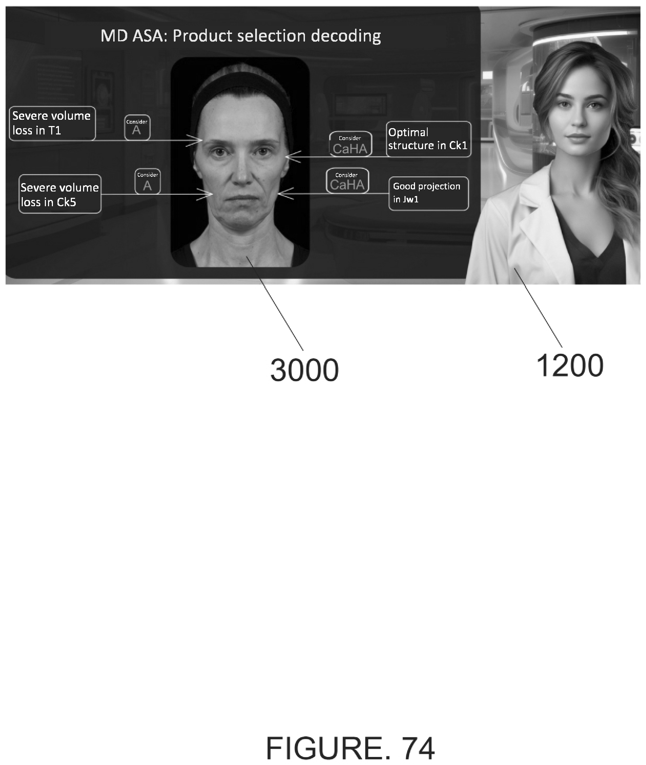

In this way, the graphical representation generated by artificial intelligence 1200 may, for example, present to the user in training a specific "case study" 3000 as well as provide the user with an explanation (whether by voice, text, video, and/or a combination thereof) regarding the selection of products to be considered for the case study in question, as exemplarily illustrated in figure 74.

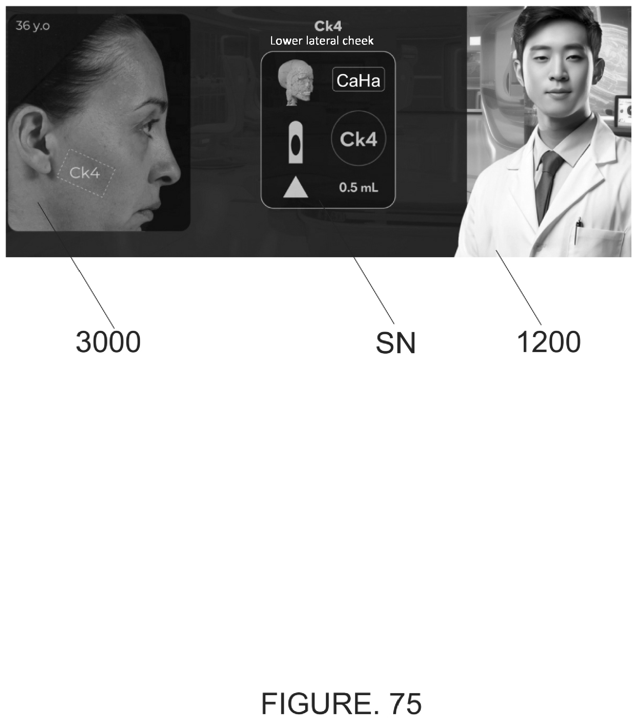

Additionally, the graphical representation generated by artificial intelligence 1200 may also associate a specific application seal SN with the case study 3000 in question, thus providing an explanation to the user (whether by voice, text, video, and/or a combination thereof) regarding how a particular product should be applied in that case study, as illustrated in figure 75.

The teachings proposed in the present invention are also capable of providing a set of instructions on the screen of the computing platform, said set of instructions thus providing guidance on how the application of a given substance should be carried out. The aforementioned set of instructions can be understood as graphical, video, audio, or text instructions (or a combination thereof), which can be viewed by the platform user, such as a user who is receiving training.

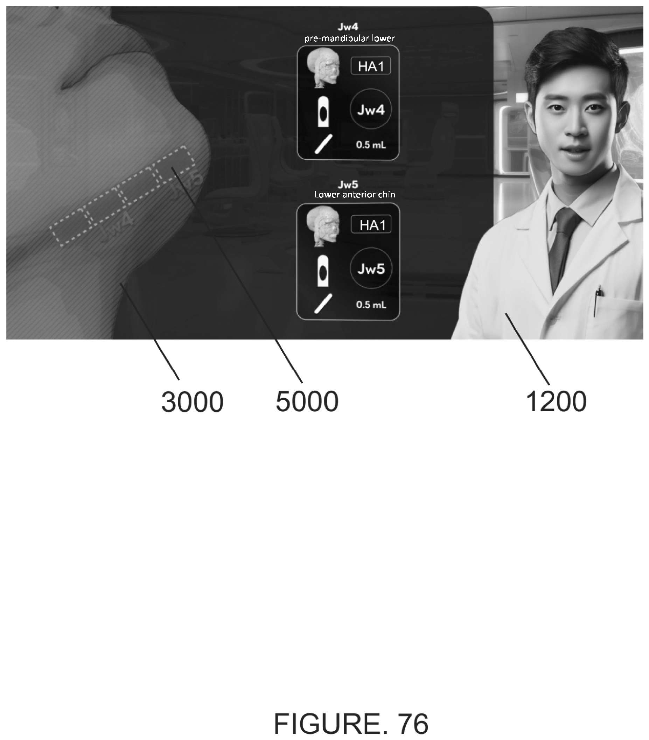

Thus, with reference to figure 76, there is an application instruction 5000 that indicates to the user how a given application seal should be read and interpreted. The application instruction 5000 is understood as a graphical instruction, generated by the computing platform and displayed over a graphical representation (photo, video, and/or representation generated by artificial intelligence) of the case study 3000 in question. It is thus understood that the application instruction 5000 is arranged in an overlapping manner with the graphical representation of the case study 3000.

It is proposed that the provision of the graphic instruction 5000 be carried out together with an audio instruction (such as the voice of avatar 1200), said audio instruction thus indicating to the user how to apply the substance in question. For example, in the case of the illustration in figure 76, said audio instruction could indicate to the user in training: *"place the patient with their chin up and mark Jw4 and Jw5 below the edge of the mandible, be aware that Jw4 is three times the length of Jw5"*. Additionally, both the graphic instruction 5000 and the audio instruction could indicate to the user in training how a specific application seal SN should be read and interpreted.

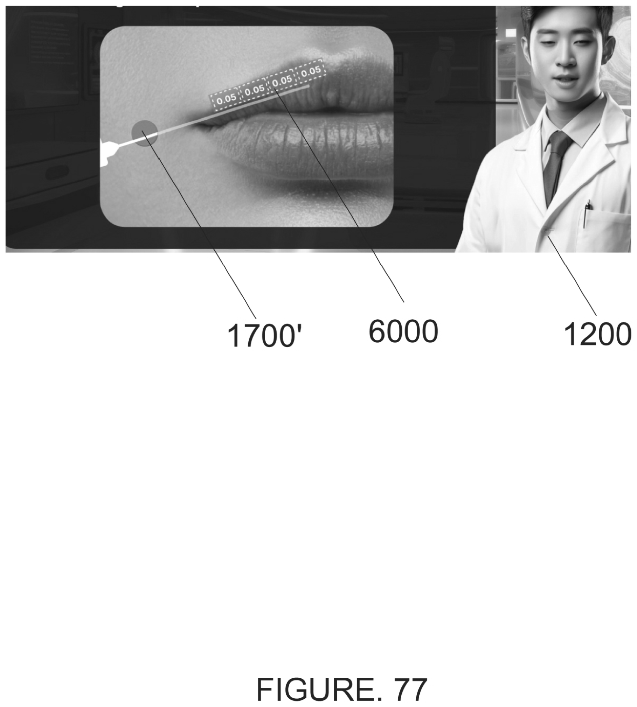

In an equally valid embodiment, and as illustrated in figure 77, the application instruction 1700' could be configured in the form of a graphical representation (such as a circle) superimposed on the surface of the case study 3000 and indicating to the user in training the positioning point of the cannula. Additional application instructions 6000 could be provided, these graphically indicating, over the case study representation 3000, the application points and their respective volumes.

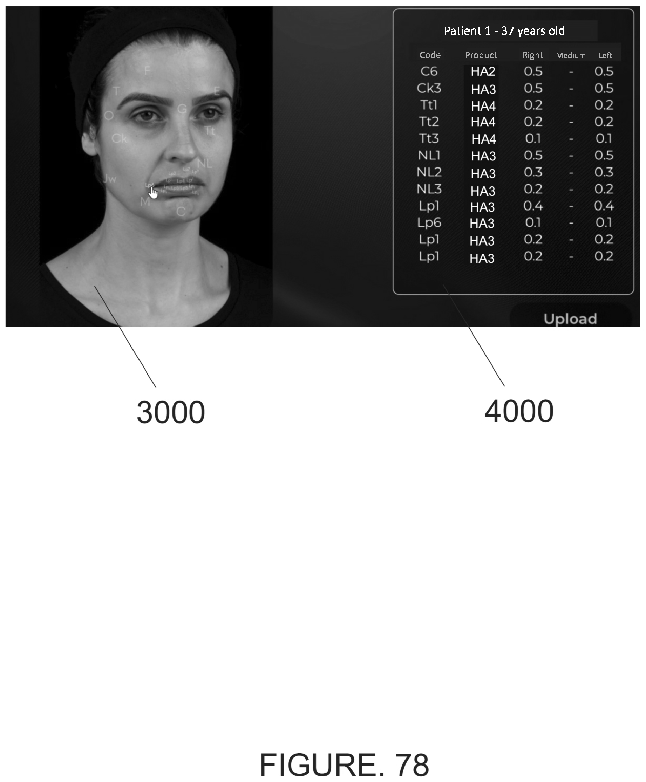

Additionally, and with reference to figure 78, the user in training may be prompted, through the computing platform, to indicate the treatment codes that the user considers appropriate for a given case study 3000. Thus, by interacting with the computing platform, the user can suggest the treatment codes (for example, by clicking on the appropriate codes) and these will be inserted into a test seal 4000 that can later be sent to the computing platform database. In one embodiment, the test seal could also be sent to an automated applicator.

Subsequently, it is proposed that a comparison be made between the test seal 4000 sent by the user and a template seal (not shown) previously stored on the computing platform. Furthermore, the computing platform is capable of generating a compliance score, thus indicating to the user the accuracy of their chosen treatment codes. The template seal is understood as previously stored data that comprises the correct treatment codes for the case study 3000 in question.

It is thus understood that the teachings of the present invention can be applied on an educational platform, both for healthcare professionals and other related professionals, as well as for patients.

Furthermore, the medical professional himself can evaluate the recording data, thus viewing the video of the procedure performed by himself and thereby determining possible areas for improvement.

The generation and storage of the recording data _{R}D recording data are equally beneficial to the user who received the treatment plan, as this way they will have confirmation that the treatment plan was properly followed as suggested. Thus, from the application chain AC, it becomes possible to track the history of applications performed, for example, for a specific user.

In this way, and according to the description previously provided, it is understood that one of the advantages of the application chain lies in the possibility of tracking the data linked to the treatment plan, thus acting, for example, as legal support (for example, for regulatory agencies) regarding the generated treatment plans.

Thus, the teachings of the present invention are beneficial not only to the user who receives the treatment plan, but also, for example, to a company that manufactures aesthetic substances, which makes use of the methodology, system, and computing platform proposed in the present invention and which can be used, for example, for the generation of thousands of treatment plans simultaneously. It is thus understood that the present invention comprises the step of generating a plurality of treatment plans simultaneously.

In one embodiment, the teachings of the present invention could be applied, for example, in a medical office (temporary clinic) and/or at a specialized trade show in the aesthetic and/or medical sector, which could comprise a totem and/or a *stand* equipped with a computing platform that makes use of the methodology described herein. The application of the teachings of the present invention in an aesthetic clinic would also be fully acceptable.

Once the treatment has been performed, it is possible to display, on the computing platform, a representation of the user's face as it appeared before the treatment and as it appears after the treatment, thus allowing the user (and/or the medical professional) to assess the effectiveness of the treatment performed. Such display may occur with the user's prior authorization.

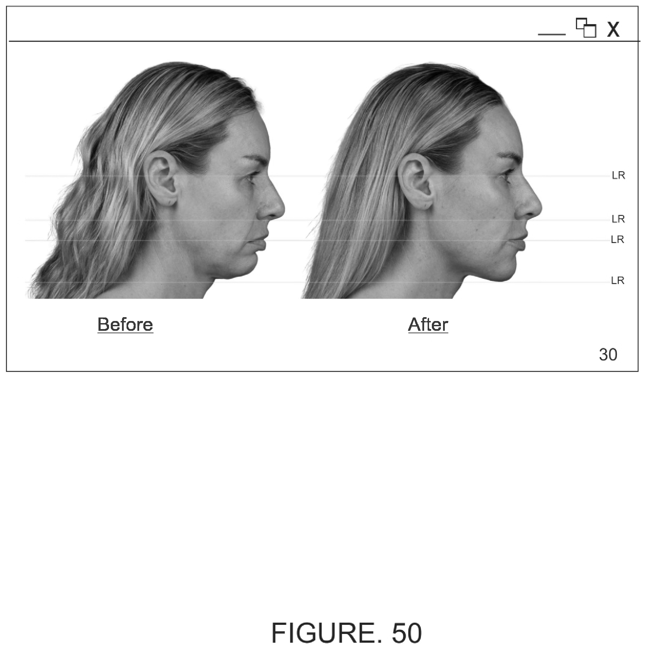

In one embodiment, the evaluation of treatment efficiency can also be obtained from the arrangement of LR reference lines on the user's face, these LR reference lines serving as a reference to assess whether a given portion of the user is aesthetically and scientifically adequate. For example, the reference lines can be useful for assessing whether or not a portion of the user is sagging (drooping). Such reference lines RL are exemplarily illustrated in FIG. 50.

Additionally, or alternatively, the evaluation of treatment efficiency may also be obtained from the segmentation of the user's portion into the plurality of quadrants, and thus assigning indices to each of the quadrants of the plurality of quadrants, as previously described.

In a valid embodiment of the present invention, the application seals as well as the application chain could be read and processed by an augmented reality device. It is thus understood that the present invention proposes the step of associating the treatment plan with an augmented reality environment. In valid embodiments, the step of associating the treatment plan with an augmented reality environment can be understood as the step of applying the treatment plan in an augmented reality environment.

Augmented reality environment is understood to mean an environment that mixes the real context with the virtual context; thus, through the use of an electronic device (such as augmented reality glasses), the user is able to perceive virtual objects placed in the real context.

Thus, for example, the augmented reality device can be used as a support and/or training tool related to the implementation of the training plan.

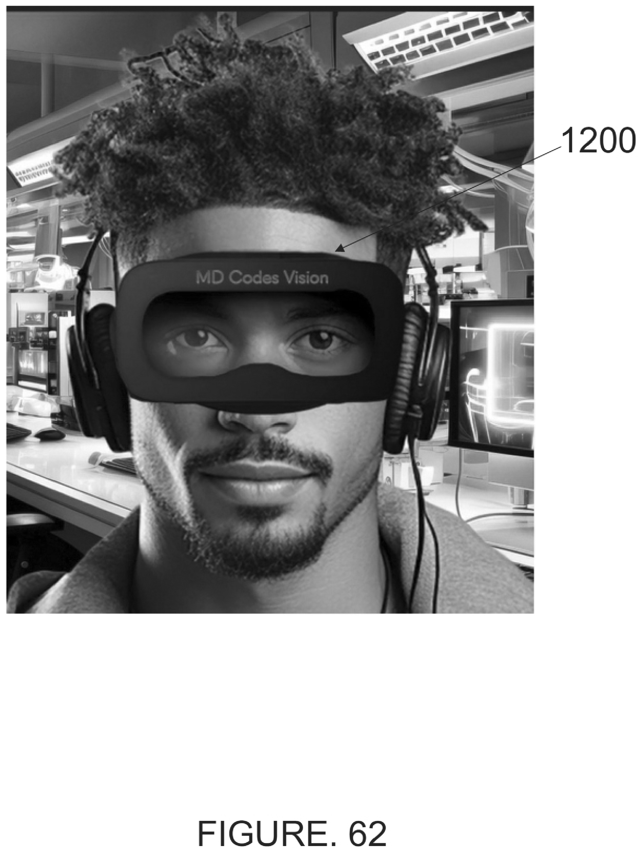

Thus, and with reference to FIG. 62, a user of the present methodology, through the use of an augmented reality device 1200, could receive and view a set of instructions 1300, 1310, 1500, 1700, 1800, 1710, 1810, 1900, 2000, 2010, 2020, 2100, 5000, 6000, and 1700' linked to the application of the substance to the patient, so that said set of instructions 1300, 1310, 1500, 1700, 1800, 1710, 1810, 1900, 2000, 2010, 2020, 2100, 5000, 6000, and 1700' are received and viewed in real time by the user, as the user looks at the patient who is to receive the application.

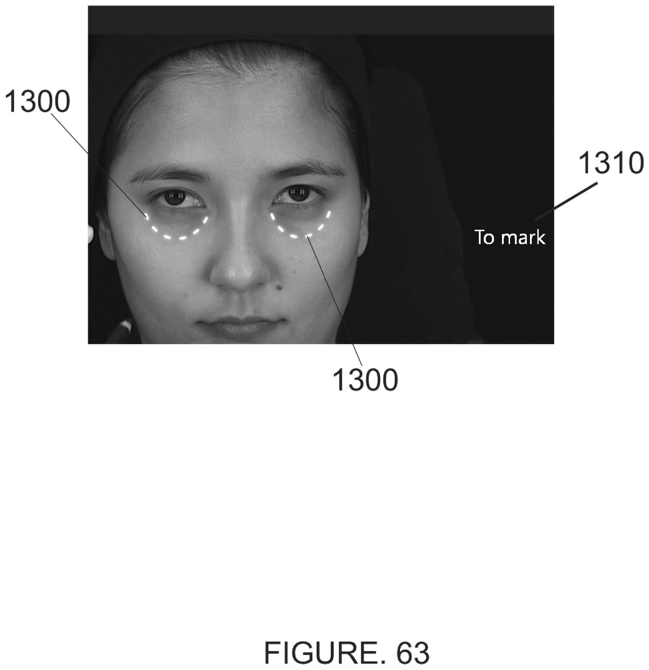

Thus, and also with reference to FIG. 63, using the augmented reality device 1200, the user, when viewing the patient who will receive the application, may receive and view a set of instructions that assist the professional during the application. Said set of instructions can be understood as graphical, video, audio, or text instructions (or a combination thereof), which can be viewed by the professional using the augmented reality device 1200.

Thus, a first instruction 1300 may be generated indicating the location on the patient's face where the user should make a specific marking.

It is understood that said first instruction 1300 is configured as a computer-generated instruction (more specifically, by the augmented reality device 1200) and that it can be viewed by the user through the use of the augmented reality device 1200. It is thus understood that the first instruction 1300 is an instruction generated and visible in an augmented reality environment.

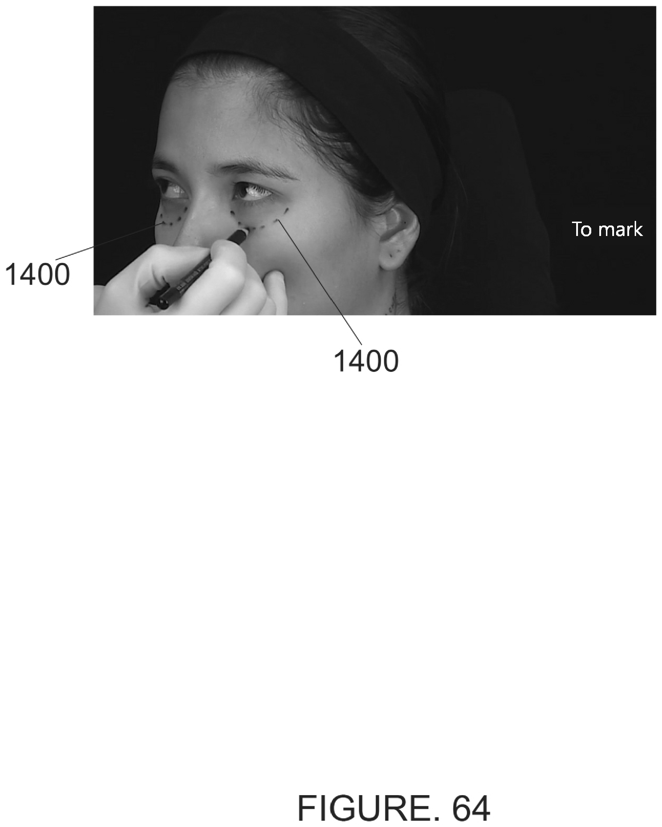

More specifically, it is proposed that the first instruction 1300 be displayed to the user superimposed on a representation of the patient, such as a representation of a photo of the patient and/or an actual representation of the patient. Real representation of the patient is understood to mean that by using the augmented reality device 1200 and looking at the patient's face, the user will view the first instruction 1300 superimposed on the patient's face, thus allowing the user to make the necessary markings 1400 directly on the patient's face, as illustrated in FIG. 64.

It is thus understood that the first instruction 1300 is a virtual instruction, while marking 1400 should be understood as a real marking, that is, actually performed on the patient.

Moreover, and as can be seen from FIG. 63, the first instruction 1300 can be understood as a graphic instruction, configured as the arrangement of lines indicating the marking location on the patient's face.

Together with the display of the first instruction 1300, an additional instruction 1310 could be arranged in the augmented reality environment and thus be perceptible to the user, said additional instruction 1310 (first additional instruction 1310) configured as a text instruction and indicating to the user the need for marking on the patient's face.

It is thus understood that the augmented reality environment allows the visualization of combined instructions, whether these instructions are graphical, textual, video, audio, sensory, or others. In a valid manner, the augmented reality environment also allows the visualization of compliance parameters, as previously described. Thus, the user who uses the augmented reality device 1200 can be understood as a trainee user, such that the compliance parameters are sent to the device 1200 from an entity that provides said training. It is thus understood that the augmented reality device allows the sending, receiving, and processing of data. In harmony with the description of the present invention, it is further understood that the computing platform is also capable of sending, receiving, and processing data.

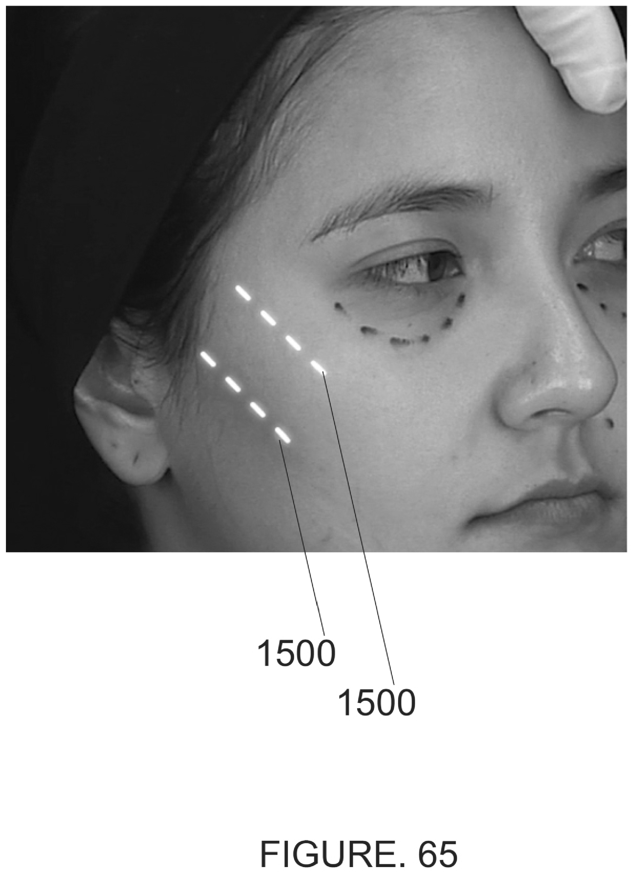

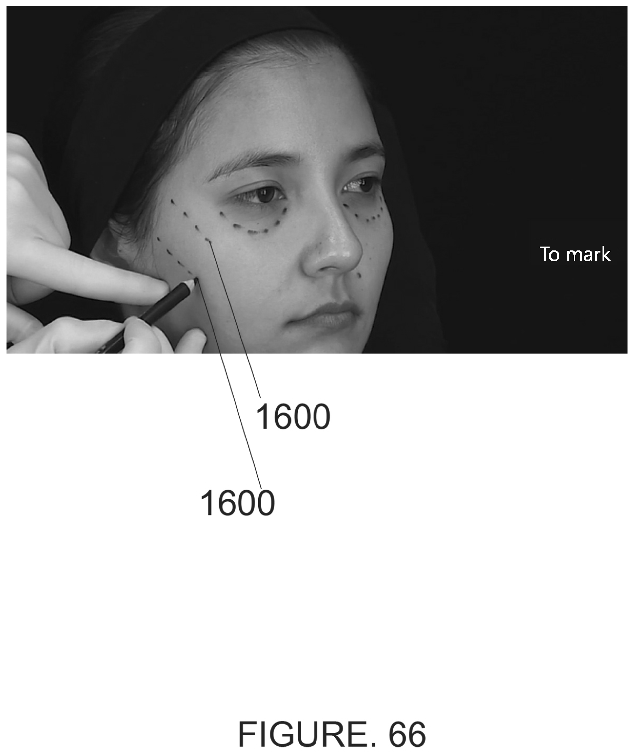

With reference now to FIG. 65, a second instruction 1500 may be displayed to the user and viewed through the augmented reality device 1200, said second instruction 1500 configured to indicate to the user the upper and lower marking locations of the zygomatic margins. It is understood that the second instruction 1500 acts as a reference, created in real time and superimposed over the patient's face, and that it facilitates the execution of markings by the patient, said marking 1600 being effectively illustrated in FIG. 66.

Additional instructions may be generated and displayed to the user utilizing the augmented reality device, such as instructions indicating the need to clean a portion of the user's face as well as the need for a certain portion of the user's face to be pressed/pinched (*pinch*).

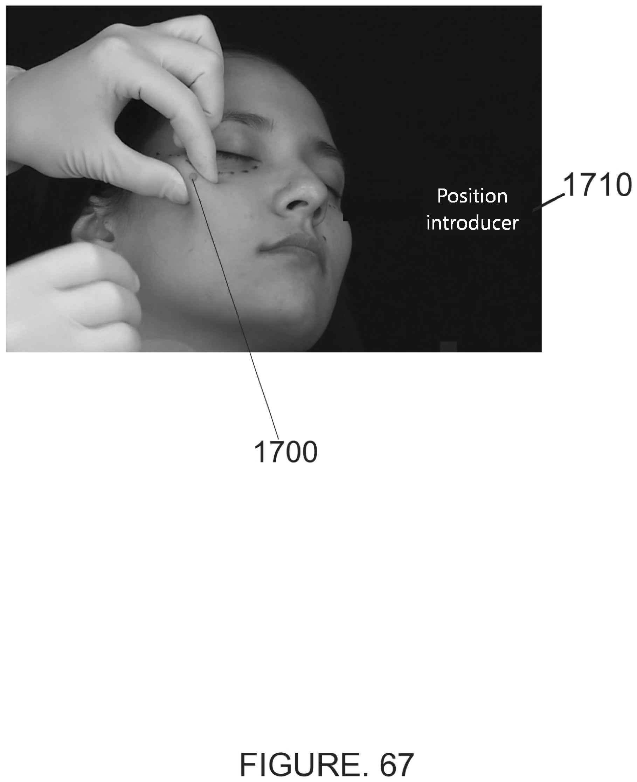

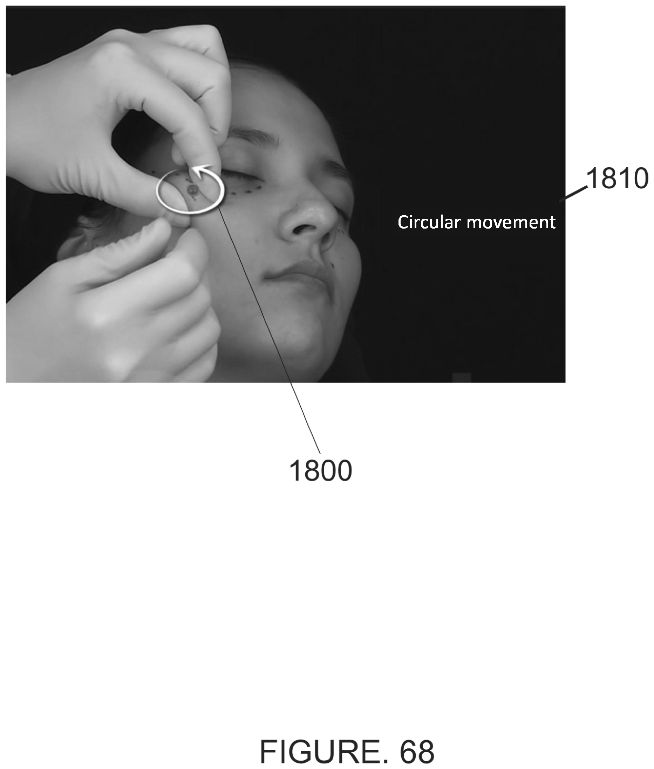

With reference to FIG. 67, an additional instruction, here referred to as third instruction 1700, may be viewed by the user and indicates, through a red circle overlaid on the patient's face, the exact location where the "introducer" should be placed, so that a fourth instruction 1800 may also be virtually generated to graphically indicate the need to perform circular movements to enlarge the orifice, as illustrated in FIG. 68. Obviously, the reference to a red circle should not be considered as a limiting feature of the present invention.

It is understood that additional instructions 1710 and 1810 (referred to as the second and third additional instructions) can respectively be combined with the third instruction 1700 and fourth instruction 1800, said additional instructions 1710 and 1810 indicating, in textual form, an action that must be performed by the user of the methodology.

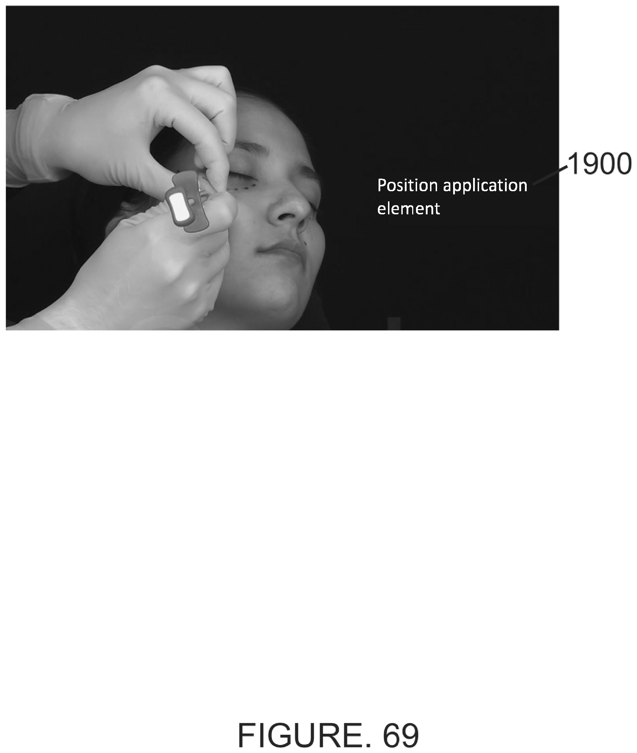

With reference now to FIG. 69, a fifth instruction 1900 could be generated indicating the positioning step of the application element, such as the cannula.

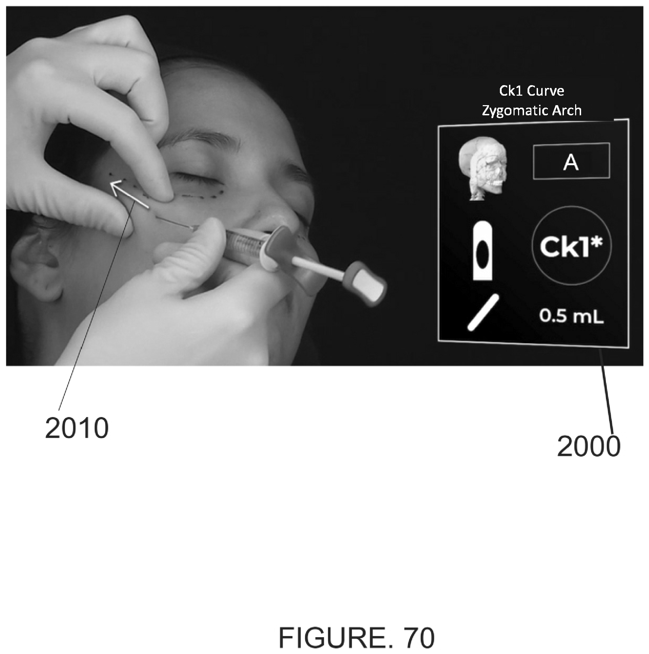

Subsequently, a sixth instruction 2000 could be generated, as illustrated in FIG. 70, thus indicating the need for application of the substance. In this case, it is proposed that said sixth instruction 2000 be understood as the virtual illustration of one of the application seals that is part of that patient's treatment plan. Thus, the user who uses the augmented reality device 1200 will virtually view the application seal containing all the instructions related to the application.

During the application, additional instructions 2010 and 2020 may be generated with the aim of assisting the user in the application of the substance. Such instructions may refer, for example, to a direction for applying the substance (instruction 2010) as well as to a virtual dispenser 2020 of the amount of substance already injected. The additional instructions 2010 and 2020 can be respectively understood as a fourth additional instruction and a fifth additional instruction.

Additionally, an audio instruction may be played to the professional using the augmented reality device, for example, and with reference to FIG. 70, said audio instruction may comment on the information contained in the application seal, thus said audio instruction could reproduce the following statements: *"Inject into Ck1 with hyaluronic acid (VM), in the sub-SMAS layer, using a linear retrograde technique with a total of 0.5 ml"*.

It should be noted that any instruction generated and displayed through the augmented reality device 1200 could be configured as an audio instruction. Furthermore, it should be noted that a given instruction could be generated either graphically (such as in text, video, or through arrows, circles, etc.) or audibly. Thus, the professional could see the aforementioned instruction and also hear it.

Once the application is completed, additional instructions may be generated for user visualization, such as instructions to remove the cannula, stop any bleeding, confirm the volume of substance actually applied, and clean the application area.

It should be noted that any of the instructions mentioned above can be understood as an instruction that is part of the set of instructions generated by the augmented reality device 1200, that is, any of the instructions mentioned should be understood as a virtual instruction, that is, an instruction perceptible in an augmented reality environment.

Additionally, any of the instructions mentioned above can be understood as an instruction that is part of the set of instructions, which may be displayed directly on the screen of the computing platform.

An additional feature of the present invention, linked to the use of the augmented reality device 1200, consists of the possibility of using an anatomy tool.

In summary, the anatomy tool enables the professional (user of the methodology) to review the patient's anatomy related to the application in question at any time, such as before performing a procedure.

Thus, through the use of the augmented reality device 1200, a graphical indication 2100 (also referred to as seventh instruction) can be arranged over the patient's face and consequently be visualized by the user of the methodology, so that said graphical indication 2100 can represent the patient's arteries.

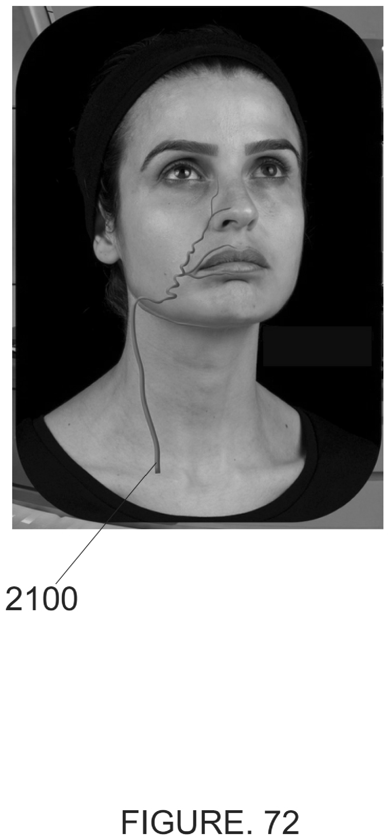

FIGURE 72 is an illustration of the graphic indication 2100 placed on the patient's face; in this, the illustration of the external carotid artery and the facial artery with the submental branch *(submental branch)* can be seen. It is also possible to visualize the inferior and superior labial arteries, followed by the lateral and angular nasal arteries.

It is proposed that, during the application of the substance, the professional be able to review, at any time, the relevant anatomical structure of the patient considering the generated treatment plan.

In one embodiment, the representation of the graphic indication 2100 referring to the patient's anatomical structure could be viewed by the professional through the augmented reality device by means of displaying a video; in this way, said video could sequentially display the arteries mentioned previously.

In one embodiment, the graphic indication 2100 could be configured as a solid line, displayed in red, representing the patient's arteries, so that said graphic indication should be superimposed on the patient's face, thus facilitating the visualization of the graphic indication 2100 by the medical professional. In one embodiment, said graphical indication could be virtually placed over the actual face of the patient and, through the use of the augmented reality device, that is, when looking at the patient, the user will virtually view the graphical indication 2100. In equally valid embodiments, the graphic indication 2100 could be placed over a photo and/or video of the patient.

Furthermore, it is understood that the graphic indication 2100 is a virtual graphic indication, that is, visualized in an augmented reality environment and through the use of the augmented reality device.

In harmony with the previously provided description, the present invention also describes a computing system for consultation, diagnostic evaluation, and financial planning linked to a treatment plan. It is understood that said system is capable of performing all the steps of the method described by using a computing platform.

Thus, the user can interact with the computing platform and thereby carry out the steps of the described method.

It is thus understood that the computing platform comprises an input block 31 configured to receive user information, a memory, a processor, as well as a comparator module to perform the described comparisons and thereby assess whether there is a need for an aesthetic treatment.

Thus, according to the teachings described, it is understood that the invention can be implemented in a mobile application of an electronic device, such as a cell phone.

Furthermore, it is understood that the teachings of the present invention can be applied with the use of an augmented reality device.

Furthermore, and as described, the invention is also based on the use of servers that communicate with the computing platform, such as servers arranged in the cloud.

It is thus understood that the invention also covers a non-transitory computer-readable medium comprising a set of instructions capable of executing the steps of the previously described method as well as performing the functionalities of the described system.

Finally, and in accordance with the teachings of the present invention, the step of scanning, on the computing platform, at least a portion of the user results in obtaining an aesthetic indicator, which may assume a treatment state or a non-treatment state.

As described, it is understood that an aesthetic indicator that assumes a treatment state results in obtaining a treatment code. Furthermore, said aesthetic indicator can be obtained in various ways, such as by segmenting the user's face into a plurality of quadrants and assigning an index (value) to each of the quadrants, or to a set of quadrants, as previously described.

Finally, the step of scanning, on the computing platform, at least a portion of the user can be performed based on the face hierarchies H1, H2, H3, H4, and H5 previously addressed in patent US 11,602,303. It should be noted that scanning the face based on hierarchies should not be considered a limiting feature of the present invention, such that performing a scan not linked to hierarchies would be fully acceptable.

It is noteworthy that the teachings of the present invention can be absorbed by any person, such as an individual who wishes to receive a treatment plan comprising a specific treatment code. Thus, the teachings of the present invention are beneficial, for example, for users who wish to receive a treatment, such as an aesthetic treatment. In any case, it should be noted that the teachings of the present invention are not limited to aesthetic treatment plans.

Furthermore, the teachings of the present invention may also be adopted by healthcare professionals, such as professionals in the field of aesthetic healthcare. Obviously, medical professionals can also absorb the teachings of the present invention; however, it is emphasized that the teachings described herein can be absorbed by anyone, and this person does not necessarily have to be a physician.

Likewise, managers of healthcare, aesthetics, and medical companies can also benefit from the teachings of the present invention.

The professional who will administer the treatment must be a qualified professional authorized to perform the treatment in accordance with the regulatory standards of each country. The system may include information regarding the regulations of each country.

Furthermore, it is understood that the present invention also covers a system capable of performing the described methodology.

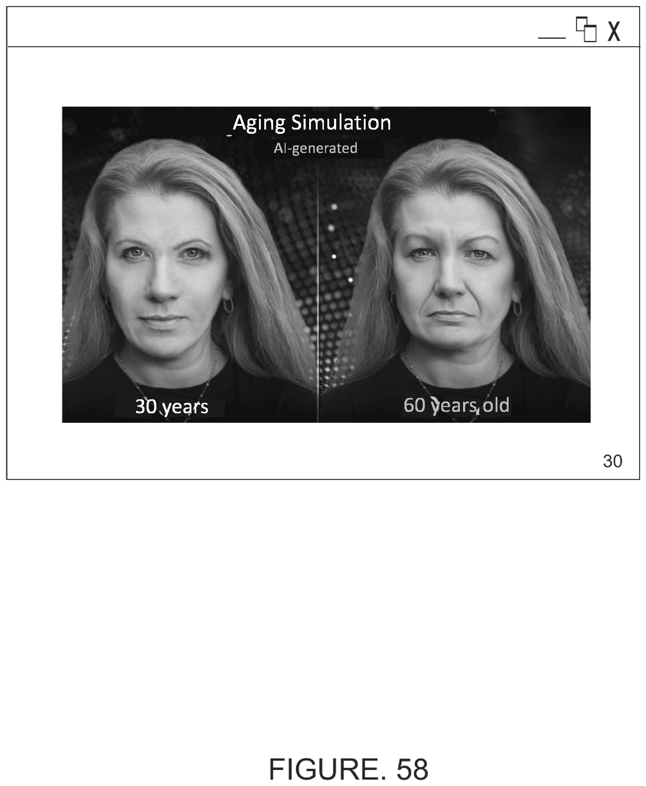

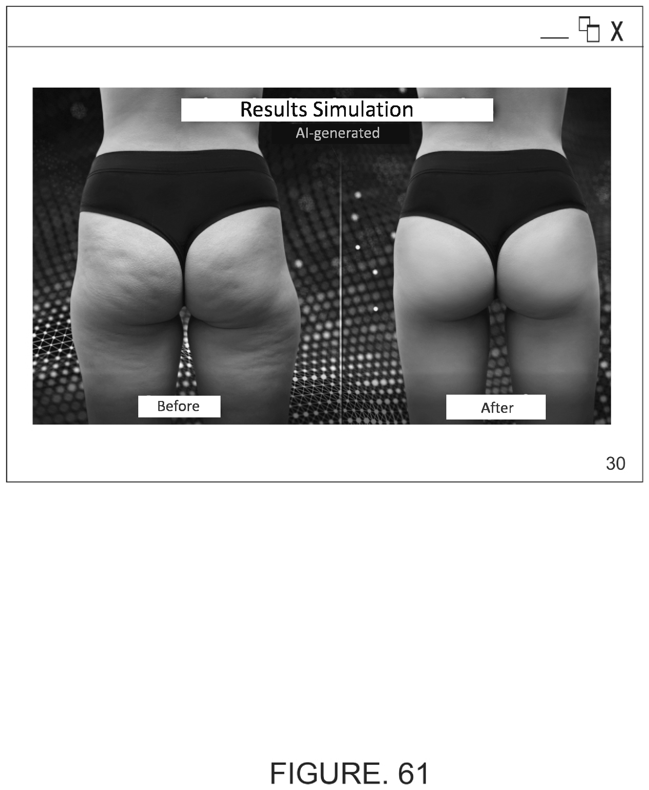

Furthermore, for the final acquisition of the treatment plan (that is, for the generation of the treatment plan), it is proposed that the described methodology will provide options to the user, including simulations of the aging process and simulations of possible outcomes following the treatment, as shown in FIGS. 58 and 61 (these may be generated, for example, through artificial intelligence) thus identifying inconsistencies and consistencies between the subjective (impulsive) and graphical (objective) evaluation performed by the user and the evaluation performed from the face scan. Thus, the methodology described in the present invention proposes the step of asking the user whether they wish to proceed with the subjective and graphical evaluation or with the evaluation performed from the scanning of the face and/or body, depending on the area focused on by the patient.

Thus, the methodology described in the present invention further comprises the step of prioritizing, in the treatment plan, at least one among the final set of aesthetic data, the final set of emotional data, the final set of social data, and the user's facial scan.

It should be noted that, if desired by the user, the final set of aesthetic data, the final set of emotional data, and the final set of social data may be disregarded for the generation of the treatment plan, thus prioritizing solely the generation of the treatment plan based on the scanning of the user's face and/or body.

Furthermore, the teachings of the present invention allow for before-and-after simulations for a specific portion of the user, such as their face and/or body, as well as simulations of the aging process for both the face and the body.

It is also noted that the teachings of the present invention can be applied to any portion of the user, whether to the user's face, the user's body, or any portion of the user.

Finally, it is understood that, according to the teachings of the present invention, the treatment plan can be generated from the application seals, as well as the application seals can be generated from the treatment plan.

From the description provided, it is understood that the teachings of the present invention can be used by users from any location, thus eliminating geographical and communication barriers, and accommodating different regulatory and legal rules in a single computing platform.

Furthermore, as described, the teachings of the present invention can be used not only to personalize treatment plans, but also to personalize the training of professionals according to their level of experience, specialty, and individual needs.

Additionally, through the already described compliance parameters, the present invention allows for the provision of instant feedback related to the application of the substance, thus enabling the identification of areas for improvement and providing specific guidance to help injectors improve their skills.

Additionally, certifications can also be conducted and granted within the platform described herein to injectors who successfully complete courses or programs on the platform, adding value to their résumé and helping to validate their skills.

In one embodiment, the teachings of the present invention may be used in the form of a computing platform that functions as a virtual laboratory, thus allowing users to practice their skills and procedural techniques in a simulated environment, using, for example, an artificial intelligence-generated avatar as patients (case study) as well as using any other form of test model (such as the use of photographs and videos of people). Thus, the teachings of the present invention provide a practical learning experience, further enabling the application of tests in an augmented reality environment, thereby allowing users to refine their skills and develop confidence in performing aesthetic procedures safely and effectively.

In harmony with the description provided, the teachings of the present invention incorporate financial planning tools to estimate treatment costs, outline payment options, and facilitate transparent communication regarding prices and financial responsibilities. Users can explore different financing alternatives and budgeting strategies to make informed decisions about their aesthetic treatments.

A significant advantage of the teachings proposed herein is the ability to detect errors that may be made by injectors during injections. These errors are promptly captured and transmitted (for example, through the _{R}D recording data, as previously described), thus serving as a valuable database of injector practices. By highlighting areas for improvement, training, or further study, this feedback mechanism allows injectors to refine their skills and gain experience more effectively.

In addition, the teachings of the present invention may also be used as an immersive reality tool (virtual reality and/or augmented reality) that allows users to practice technical skills in a realistic virtual environment. These simulations can replicate treatment rooms, equipment, and interactions with patients, providing a safe and controlled environment for users to refine their techniques and build confidence. Whether learning new codes or refining existing skills, the present invention provides support by offering visual overlays, anatomical landmarks, and step-by-step instructions tailored to the user, as previously described and referring to the set of instructions 1300, 1310, 1500, 1700, 1800, 1710, 1810, 1900, 2000, 2010, 2020, 2100, 5000, 6000, and 1700' and compliance parameters. It is understood that the set of instructions may be displayed directly on the computing platform and/or through the augmented reality device. The same applies to the compliance parameters.

Based on the description provided, it is understood that the teachings of the present invention are beneficial at least for professionals in the health/aesthetic field, such as professionals who administer injectable substances, for the general public who wish to receive a treatment plan, for cosmetic and/or pharmaceutical companies, as well as for students, educational institutions, and users who wish to receive some type of training (or provide training).

In harmony with the previously provided description, the present invention also addresses a device 30 capable of performing the steps of the described method. In one embodiment, said device 30 may be understood as a cell phone, tablet, television, kiosk, or any other equivalent device.

As described, the device 30 comprises at least one processor, a display, a camera, and a memory. As previously described, such components can be understood as the input block 31 of the device 30. It is understood that the device 30 is capable of performing any of the steps of the proposed method.

Thus, in summary, the device 30, through its input block 31, is capable of receiving a plurality of general input data from the user and generating a treatment identifier code from the plurality of general input data. The device 30 is also capable of collecting a plurality of consultation data related to the user and, additionally, scanning (for example, through the device's camera) at least a portion of the user. Thus, the device 30, through its processor, is capable of generating a treatment plan from the scanning of the user's face.

As already mentioned, the device 30 proposed in the present invention is capable of performing any of the steps of the proposed method. Thus, it is understood that the device 30 is capable of segmenting a portion of the user (such as the user's face) into a plurality of quadrants and assigning at least one index to at least one of the quadrants of the plurality of quadrants.

Thus, it is understood that the device 30 is also capable of defining monitoring portions, consideration portions, and treatment portions of the user, so that said portions are defined through the previously mentioned aesthetic variation index.

It is understood that the device 30 is also capable of obtaining at least one treatment code from scanning the user's face and displaying the treatment codes on a screen of the device.

Furthermore, the device 30 is also capable of generating, sending, receiving, processing, and displaying at least one application seal S1, S2, S3, S4...SN linked to the user. The characteristics of the application seal S1, S2, S3, S4...SN have already been previously described.

As already described, in one embodiment, the application seal S1, S2, S3, S4...SN may be illustrated on the screen of device 30. Additionally, the application seal S1, S2, S3, S4...SN can be sent from the device 30 to an automated applicator.

In harmony with the previously provided description, the proposed device 30 in the present invention, through its processor, is also capable of generating an application chain AC from the grouping of a set of application seals S1, S2, S3, S4...SN linked to the user. As previously described, the application chain AC could also be sent, from device 30, to an automated applicator.

It is further understood that the proposed device 30 is capable of storing data in the application seals S1, S2, S3, S4...SN and/or in the application chain AC, such as storing at least one recording data RD in the application seal and/or in the application chain.

The device 30 proposed in the present invention is capable of performing any step of the method already described. Thus, the device 30 described in the present invention is capable of generating a graphical representation of a professional, in which the graphical representation is generated by means of artificial intelligence, such that the device 30 is also capable of conducting an interaction with the user, said interaction being conducted, for example, through the graphical representation.

As already described, the device 30 proposed in the present invention is also capable of associating the treatment plan with an augmented reality environment.

It is thus understood that the present invention also describes a device 30 capable of generating a treatment plan.

The present invention also describes an automated applicator capable of communicating with the device 30 proposed in the present invention. So, the automated applicator is able to communicate, for example, with a mobile device 30 that performs the steps of the method proposed in the present invention.

Said automated applicator can be understood as an applicator of aesthetic substances, such as injectable substances. Thus, in accordance with the description previously provided, the automated applicator is capable of receiving an application seal S1, S2, S3, S4...SN and thereby performing the reading (processing) of said application seal S1, S2, S3, S4...SN.

In one embodiment, the sending of the application seal S1, S2, S3, S4...SN from the device 30 (cell phone, computer, or any other equivalent means) to the automated applicator could occur through an internet connection; in any case, any sending embodiment that uses a communication protocol is acceptable. By reading the application seal S1, S2, S3, S4...SN by the automated applicator, it should be understood as the processing of the application seal S1, S2, S3, S4...SN (computer processing) by the automated applicator. It is thus understood that the automated applicator comprises at least one processor and a memory.

Thus, upon receiving the application seal S1, S2, S3, S4...SN, a mechanical arm of the automated applicator will move, thus transitioning from a resting state (stationary) to a moving state. It is understood that the mechanical arm of the automated applicator is associated with a substance injection means (such as a syringe).

In one embodiment, the automated applicator may be located in a different geographic location from the geographic location where the device 30 is located.

In one embodiment, the automated applicator is capable of reading and processing each of the data elements that make up an application seal and performing the application of the substance according to the data provided on the application seal.

It is also understood that the automated applicator is capable of reading and processing the information contained in an application chain AC.

It is thus understood that the present invention also addresses an automated applicator associated with device 30, said device 30 being capable of performing the steps of the proposed method.

Having described a preferred embodiment, it should be understood that the scope of the present invention encompasses other possible variations, being limited only by the content of the accompanying claims, possible equivalents included therein.

## Claims

1. A computer method for consultation, diagnostic evaluation, and financial planning for generating a treatment plan related to a user, **characterized by** comprising the steps of:
receiving, on a computing platform, a plurality of general input data from a given user,
generating a treatment identifier code from the plurality of general input data,
collecting a plurality of consultation data related to the user,
scanning, on the computing platform, at least a portion of the user, and
generating the treatment plan at least from the scanning of at least a portion of the user.

2. The method according to claim 1, **characterized by** further comprising the step of:
obtaining at least one first set of aesthetic user data from an impulsive representation of the user,
obtaining at least a second set of aesthetic user data from a graphical representation of the user,
evaluating, in a comparator module, whether there is consistency between each of the aesthetic data of the first set of aesthetic data and the second set of aesthetic data,
if a consistency is not detected, issue a perceptible representation to the user and prompt the user, through the computing platform, to enter a final set of aesthetic data, alternatively,
if consistency is detected, issue a perceptible representation to the user and transform the first set of aesthetic data and the second set of aesthetic data into the final set of aesthetic data.

3. The method according to any of the preceding claims, **characterized by** further comprising the step of:
obtaining a first set of emotional user data from the first set of aesthetic data,
obtaining a second set of emotional user data from the second set of aesthetic data,
evaluating, in the comparator module, whether there is consistency between each of the emotional data from the first set of emotional data and each of the emotional data from the second set of emotional data,
if a consistency is not detected, issue a perceptible representation to the user, alternatively,
if a consistency is detected, issue a perceptible representation to the user and transform the first set of emotional data and the second set of emotional data into a final set of emotional data.

4. The method according to any of the preceding claims, **characterized by** further comprising the steps of:
obtaining, from a socializing block, a plurality of favorable social data linked to the user and a plurality of unfavorable social data linked to the user,
consolidating the plurality of favorable social data and the plurality of unfavorable social data into a final set of social data.

5. The method according to any of the preceding claims, **characterized by** further comprising the steps of:
generating a difficulty index from at least the evaluation of the final set of aesthetic data, the final set of emotional data, and the final set of social data, and
displaying the difficulty index, through the computing platform, to the user,
wherein the method further comprises at least one of the steps of:
suggesting to the user a plurality of applicators linked to the obtained difficulty index, wherein the plurality of applicators is segmented between professional applicators and automated applicators,
suggesting to the user a plurality of applicators based on a predetermined criterion, wherein the predetermined criterion is at least one of: a location of the user, a native language of the user, and
suggesting to the user a plurality of applicators who are qualified in a certain procedure.

6. The method according to any one of the preceding claims, **characterized in that** the step of scanning, through the computing platform, at least a portion of the user, further comprises the step of scanning the portion of the user in a horizontal and vertical manner, and also considering a frontal portion, an oblique portion, and a profile portion of the user,
wherein the scanning step further comprises the step of segmenting the user's portion into a plurality of segments.

7. The method according to any of the preceding claims, **characterized by** further comprising the step of correlating the scanning of the user's portion with at least one among a plurality of positive virtual social data, a plurality of negative virtual social data, and a set of virtual emotional data linked to the user, wherein the correlating step further comprises the step of scanning a portion of the user and generating an alert indication for each virtual emotional data considered.

8. The method according to any one of the preceding claims, **characterized in that** the step of scanning, on the computing platform, at least a portion of the user further comprises the step of:
segmenting the user portion into a plurality of quadrants,
assigning at least one index to at least one of the quadrants of the plurality of quadrants, and
generating an aesthetic indicator from the segmentation of the user's portion in the plurality of quadrants.

9. The method according to any one of the preceding claims, **characterized in that** the step of scanning, on the computing platform, at least a portion of the user further comprises the step of performing, through the computing platform, a dynamic scan of the user, wherein the step of performing a dynamic scan of the user further comprises the step of:
comparing user portions in a static state and in a dynamic state,
mirroring at least a portion of the user, wherein the step of mirroring at least a portion of the user further comprises at least the step of: comparing a first mirroring of the user with a second mirroring of the user and,
identifying a final mirroring based on the comparison between the user's first mirroring and the user's second mirroring.

10. The method according to any one of the preceding claims, **characterized in that** the step of comparing portions of the user in a static state and in a dynamic state further comprises the step of:
generating, for each comparison performed, the aesthetic indicator, wherein the aesthetic indicator assumes at least one treatment state and one non-treatment state, wherein the method further comprises the step of:
obtaining and storing at least one treatment code if the aesthetic flag assumes a treatment state.

11. The method according to any one of the preceding claims, **characterized in that** the step of comparing portions of the user in a static state and in a dynamic state further comprises the step of:
assigning a static index and a dynamic index to each user portion, the static index linked to the static state and the dynamic index linked to the dynamic state,
comparing the static index and the dynamic index for each user portion,
assessing whether there is an aesthetically positive or aesthetically negative variation based on the comparison between the static index and the dynamic index,
if an aesthetically negative variation is detected, perform the step of assigning the treatment state to the aesthetic indicator, and
if an aesthetically positive variation is detected, perform the step of assigning the aesthetic indicator the state of no treatment.

12. The method according to any one of the preceding claims, **characterized in that** the step of scanning, on the computing platform, at least a portion of the user further comprises at least one of the following steps:
comparing at least a portion of the user with at least a portion considered ideal, and
generating, for each comparison performed, the aesthetic indicator, wherein the aesthetic indicator assumes at least the treatment state and the non-treatment state, wherein the method further comprises the step of:
obtaining and storing at least one treatment code if the aesthetic indicator assumes the treatment state.

13. The method according to any one of the preceding claims, **characterized by** the fact that the step of comparing at least one portion of the user with at least one portion considered ideal further comprises the step of:
comparing, for each user portion, a real aesthetic data with an aesthetic data considered ideal, wherein the aesthetic data considered ideal is a data previously stored in the computing platform, wherein the aesthetic data considered ideal is obtained from at least one of the following forms: clinical tests, provided by the computing platform itself, proposed by an artificial intelligence tool associated with the computing platform, wherein:
the aesthetic data considered ideal is related to a specific ethnicity and age group associated with the user, wherein the method further comprises the step of:
updating the aesthetic data considered ideal based on ideal learning aesthetic data.

14. The method according to any one of the preceding claims, **characterized in that** the step of scanning, on the computing platform, at least a portion of the user further comprises at least one of the following steps of:
define user monitoring portions,
defining user portions for consideration,
defining user treatment portions,
in which the user portions for monitoring, consideration, and treatment are obtained from an aesthetic variation index (Δₑₛₜ), in which the aesthetic variation index (Δₑₛₜ) is obtained from the following expression:$Δ est = \left|{lest}_{ideal}-\left.{lest}_{real}\right|\right.$, in which:
the Iest_{ideal} should be understood as an ideal aesthetic index, and
The Iestᵣₑₐₗ should be understood as a real aesthetic index.

15. The method according to any one of the preceding claims, **characterized by** further comprising the step of obtaining at least one treatment code from the step of scanning at least a portion of the user, and
displaying the treatment codes in a user portion, wherein the method further comprises the step of: for each obtained treatment code, also obtaining a related dose, and
displaying the related dose on the computing platform.

16. The method according to any of the preceding claims, **characterized by** further comprising the step of:
from the treatment codes, generate at least one application seal (S1, S2, S3, S4...SN) linked to the user, in which the application seal (S1, S2, S3, S4...SN) comprises at least:
a treatment code data,
a product application data,
one application layer data,
an application device data,
an application mode data,
an active number data,
wherein the application seal is configured as at least one of: a machine-readable application seal and a user-readable application seal.

17. The method according to any of the preceding claims, **characterized by** further comprising the steps of:
generating the treatment plan from the application seals (S1, S2, S3, S4...SN), wherein the treatment plan comprises treatment data, wherein the treatment data represent at least one of:
a data on the number of treatment sessions,
a treatment cost data,
data indicating the person responsible for carrying out the treatment plan,
wherein the method further comprises the step of:
assessing whether there is a user objection regarding at least one of the processing data, in which,
if there is a challenge, perform the step of receiving an update data from the user and generating an updated treatment plan, and
if there is no objection, proceed to the step of implementing the generated treatment plan.

18. The method according to any of the preceding claims, **characterized by** further comprising the steps of:
receiving, on the computing platform, an input data relating to the at least one of a cost data and a treatment session number data,
generating a treatment plan based on the at least one of the cost data and the treatment session number data.

19. The method according to any of the preceding claims, **characterized by** the fact that the step of generating a treatment plan further comprises at least one of the following steps:
generating and displaying the application seals linked to the treatment plan,
generating an application chain (AC) from the grouping of a set of application seals linked to a specific user, wherein the application chain further comprises at least one of the treatment identifier code and the treatment cost data.

20. The method according to any one of the preceding claims, **characterized in that** the application seal is segmented into at least a first portion and a second portion, wherein each portion comprises the same amount of data, wherein the treatment code data is outlined by a warning light.

21. The method according to any of the preceding claims, **characterized by** further comprising at least one of the following steps:
sending at least one of the application seal and the application chain to an automated applicator, and
sending at least one of the application seal and the application chain to a professional applicator.

22. The method according to any of the preceding claims, **characterized by** further comprising at least one of the following steps:
recording the application of the application chain, whether said application is performed by the automated applicator or by the professional applicator, and
generating at least one recording data (RD), and
storing the recording data (RD) in the application chain (AC).

23. The method according to claim 22, **characterized in that** the treatment plan comprises at least one application action (AP1, AP2, AP3...APN), wherein the method further comprises at least one of the following steps:
generating the recording data (RD) for each application action (AP1, AP2, AP3...APN) present in the treatment plan,
generating the cost data (CD) for each application action (AP1, AP2, AP3...APN) existing in the treatment plan.

24. The method according to any of the preceding claims, **characterized by** further comprising the steps of:
generating a graphical representation of a professional, wherein the graphical representation is generated by means of artificial intelligence, and
conducting an interaction between the user and the computing platform, in which the interaction is conducted by the graphical representation generated by means of artificial intelligence.

25. The method according to any one of the preceding claims, **characterized in that** the graphical representation generated by means of artificial intelligence is a graphical representation of a medical professional (100) and/or a multidisciplinary graphical representation (200, 300, 400, 500, 600, 700, 800, 900, 1000, 1100).

26. The method according to any one of the preceding claims, **characterized in that** the multidisciplinary graphical representation (200, 300, 400, 500, 600, 700, 800, 900, 1000, 1100) is configured as at least one among:
a first multidisciplinary graphical representation (200) of a biomedical professional,
a second multidisciplinary graphical representation (300) of a teacher,
a third multidisciplinary graphical representation (400) of a nurse,
a fourth multidisciplinary graphical representation (500) of a pharmacist,
a fifth multidisciplinary graphical representation (600) of an accounting professional,
a sixth multidisciplinary graphical representation (700) of a marketing professional,
a seventh multidisciplinary graphical representation (800) of a product consultant,
an eighth multidisciplinary graphical representation (900) of a business manager,
a ninth multidisciplinary graphical representation (1000) of a digital influencer, and
a tenth multidisciplinary graphical representation (1100) of an attorney.

27. The method according to any of the preceding claims, **characterized by** further comprising the step of:
associating the treatment plan with an augmented reality environment, wherein said step further comprises:
generating a set of instructions (1300, 1310, 1500, 1700, 1800, 1710, 1810, 1900, 2000, 2010, 2020, and 2100) linked to the application of the substance to the patient, wherein the set of instructions (1300, 1310, 1500, 1700, 1800, 1710, 1810, 1900, 2000, 2010, 2020, and 2100) is perceptible in at least one augmented reality environment and perceptible through the computing platform.

28. The method according to claim 27, **characterized in that** the set of instructions (1300, 1310, 1500, 1700, 1800, 1710, 1810, 1900, 2000, 2010, 2020, and 2100) comprises at least one graphical instruction (1300, 1500, 1700, 1800, 2000, 2010, 2020), such that said graphical instruction (1300, 1500, 1700, 1800, 2000, 2010, 2020) is arranged so as to be superimposed on a representation of the patient.

29. The method according to claim 28, **characterized in that** the set of instructions (1300, 1310, 1500, 1700, 1800, 1710, 1810, 1900, 2000, 2010, 2020, and 2100) comprises at least one instruction (2000) configured as the virtual illustration of one of the application seals (S1, S2...SN).

30. A computing system for obtaining a treatment plan related to a user, **characterized by** comprising a computing platform (30) equipped with a processor, the processor being capable of receiving a set of instructions that, when executed, perform the steps of the method as defined in claim 1.

31. A computer-readable memory, **characterized by** containing a set of instructions which, when executed, perform the steps of the method as defined in claim 1.

32. A computing platform (30) comprising a processor, **characterized in that** the processor is capable of receiving a set of instructions which, when executed, perform the method as defined in claim 1.

33. A computer method for consultation, diagnostic evaluation, and financial planning for generating a treatment plan related to a user, **characterized by** comprising the steps of:
receiving, on a computing platform, a plurality of general input data from a given user,
generating a treatment identifier code from the plurality of general input data,
collecting a plurality of consultation data related to the user,
scanning, on the computing platform, at least a portion of the user, and
generating the treatment plan at least from the scanning of at least a portion of the user, and
associating the treatment plan with an augmented reality device.
